# EUROPEAN PATENT APPLICATION

(11) **EP 1 640 394 A1**
(43) Date of publication of application: **29.03.2006**
(21) Application number: 04745938.3
(22) Date of filing: 09.06.2004
(51) Int. Cl.: C08G 18/32, C08G 18/10, C08G 18/52, C08G 18/63, C08G 18/66, C08G 18/74, C08L 75/02, C08L 75/04, C08L 75/14, G02B 1/04, G02C 7/02

(54) **POLYOL COMPOUND, TRANSPARENT MOLDED OBJECTS, AND PROCESS FOR PRODUCING TRANSPARENT MOLDED OBJECT**

(30) Priority: 09.06.2003 JP 2003163145; 02.02.2004 JP 2004026061
(71) Applicant: HOYA CORPORATION, Shinjuku-ku, Tokyo 161-8525 (JP)
(72) Inventor: KITAHARA, Yoshitaka, c/o Hoya Corporation, Shinjuku-ku, Tokyo 1618525 (JP)
(74) Representative: Meyers, Hans-Wilhelm
(86) International application number: PCT/JP2004/008386
(87) International publication number: WO 2004/108786

(57) **Abstract**

A polyol compound **characterized by** being denoted by general formula (I). A transparent molded article obtained by polymerizing monomer components comprising the aforementioned polyol compound. A transparent molded article obtained by polymerizing monomer components comprising components (C) and (D). A method of manufacturing a transparent molded article, comprising casting a mixture of component (C) and component (D) into a casting mold and then polymerizing component (C) and component (D) to obtain a molded article.

## Description

### TECHNICAL FIELD

The present invention relates to a polyol compound and a transparent molded article obtained by using the same.

In addition, the present invention relates to a transparent molded article employed as lenses and the like and a method of manufacturing the same. In particular, the present invention relates to a transparent molded article suitable for optical applications and comprised of polyurea comprising intramolecular urethane bond or thiourethane bond, and a method of manufacturing the same.

### TECHNICAL BACKGROUND

Since plastic is lighter, tends to crack less, and dyes more readily than glass, it has been employed in optical applications such as various lenses in recent years. Plastic materials generally employed in optics include polyethylene glycol bisallyl carbonate (CR-39) and polymethyl methacrylate (PMMA). However, since these plastic materials have a refractive index of less than or equal to 1.5, when employed as lens materials, for example, the lens becomes thicker with the level of magnification. Not only does the lightweight advantage of plastic end up being lost, but thick plastic is undesirable from an esthetic point of view. Further, when these plastic materials are employed in concave lenses, there are problems in that the thickness of the lens perimeter (edge) increases, tending to result in birefringence and chromatic aberration.

Accordingly, there is a need for a plastic material of high refractive index to permit reduction in lens thickness while fully exploiting the low specific gravity characteristic of plastics. Examples of materials having such properties that have been proposed are: (1) plastic lenses comprised of (meth)acrylates having aromatic rings substituted with halogens and intramolecular alcohol hydroxyl groups, radical polymerizable compounds capable of copolymerizing with such (meth)acrylates, and polymers of isocyanate compounds (Japanese Unexamined Patent Publication (KOKAI) Heisei No. 6-211960); (2) the cured materials of compositions comprised of diol compounds having curled structures and radical polymerizable isocyanate compounds (Japanese Unexamined Patent Publication (KOKAI) Heisei No. 10-45855); (3) plastic lenses comprised of polyol compounds having sulfur atoms and bifunctional or greater polyisocyanates (Japanese Unexamined Patent Publication (KOKAI) Heisei No. 5-80201); and (4) plastic lenses comprised of polythiol compounds comprising sulfur atoms outside of the thiol groups and bifunctional or greater polyisocyanates (Japanese Unexamined Patent Publication (KOKAI) Heisei No. 9-184901).

However, although the plastic lenses of (1) and the cured material of (2) yield high refractive indexes, there are drawbacks in that the aromatic rings introduced to achieve high refractive indexes compromise the Abbé number and weatherability. In the plastic lenses of (3), although the use of polyol compounds having sulfur atoms yields high refractive indexes, there is a problem in the form of poor strength. And in the plastic lenses of (4), the use of a principal component in the form of a polythiol compound results in problems such as the peculiar odor of thiol compounds during handling, resulting in poor handling properties.

Accordingly, the first object of the present invention is to provide a novel polyol compound, which can be employed as one of monomer components for obtaining a transparent molded article having a high refractive index and high Abbé number as well as being excellent in impact resistance and weatherability.

Furthermore, another object of the present invention is to provide a transparent molded article having a high refractive index and high Abbé number as well as being excellent in impact resistance and weatherability, which can be obtained by polymerizing monomer components comprising the aforementioned novel polyol compound.

In addition, as set forth above, when polyethylene glycol biscarbonates and polymethyl methacrylates are employed as lens materials, for example, the thickness of the lens increases with the degree of magnification. Thus, there is a risk of losing the lightweight advantage of plastic. Further, although these plastic materials are more resistant to cracking than glass, there is a demand for lens materials of even greater resistance to cracking.

Accordingly, to further exploit the lightweight, crack-resistant characteristics of plastics, the polycarbonates obtained by injection molding and the polythiourethanes obtained by casting polymerization have begun to be employed in optical applications. Although these polycarbonates have extremely high impact strength, they share the common drawbacks of injection molding materials of low resistance to solvents and heat. Although the polythiourethanes do not exhibit the common drawbacks of injection molding materials, they are currently no match for polycarbonates in terms of strength.

Accordingly, to avoid the common drawbacks of injection molding materials, there is a need for a material that can be obtained by casting polymerization that affords impact strength comparable to that of polycarbonates. Known examples of materials having such characteristics are the materials obtained through the casting polymerization of aromatic diamines and isocyanate terminal prepolymers having intramolecular urethane bonds (U.S. Patent Nos. 5,962,617 and 6,127,505).

However, the aromatic diamine employed in the material disclosed in U.S. Patent No. 5,962,617 is solid at ordinary temperatures and undergoes rapid polymerization, tending to leave melt residues. As a result, there is a problem in that the molded article obtained has poor transparency. On the other hand, the material disclosed in U.S. Patent No. 6,127,505 achieves high impact strength and adequate transparency for optical materials by designing a starting material aromatic diamine. However, the refractive index, at about 1.53, is inadequate, and there is a need for a material with a higher refractive index.

Accordingly, the second object of the present invention is to provide a transparent molded article suitable for optical applications, having high impact resistance, excellent transparency and high refractive index.

### DISCLOSLTRE OF THE INVENTION

The present inventors conducted extensive research to achieve the above first object, resulting in the discovery of a novel polyol compound of prescribed structure comprising sulfur atoms contributing to a high refractive index and a high Abbé number and ester bonds or carbamate bonds imparting either intramolecular or intermolecular interaction in a main structure. Further, the present inventors discovered that a transparent molded article of high refractive index, high Abbé number and excellent impact resistance and weatherability was obtained by employing this new polyol compound as one of monomer components. The first aspect of the present invention was devised based on these discoveries.

That is, the first aspect of the present invention to achieve the above first object is as follows;
(1) A polyol compound characterized by being denoted by general formula (I): 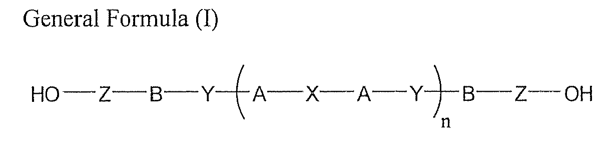 (wherein X and Y each independently denote alkylene groups comprising 3 to 12 carbon atoms, in which at least one of methylene groups (excluding terminals) contained is substituted with a sulfur atom (without direct bonding between sulfur atoms); Z denotes an alkylene group comprising 2 to 6 carbon atoms, in which a methylene group (excluding terminals) contained is optionally substituted with a sulfur atom (without direct bonding between sulfur atoms); A and B each independently denote an ester group, thioester group, carbamate group, or thiocarbamate group; and n denotes an integer ranging from 0 to 12).
(2) The polyol compound according to (1), wherein said polyol compound has an average molecular weight ranging from 300 to 2500.
(3) The polyol compound according to (1) or (2), wherein, in general formula (I), X and/or Y comprise the following structure:

   -S-CH₂-S-.
(4) The polyol compound according to any of (1) to (3), wherein, in general formula (I), B is an ester group or carbamate group.
(5) A transparent molded article obtained by polymerizing monomer components comprising the polyol compound according to (1) to (4).
(6) The transparent molded article according to (5), which is comprised of polyurethane obtained by polymerizing monomer components comprising component (A) comprising the polyol compound according to any of (1) to (4) and component (B) comprising at least one multifunctional isocyanate compound.
(7) The transparent molded article according to (5), which is comprised of a polymerization-cured material obtained by polymerizing a polymerizable composition comprising a radical polymerizable compound having a carbamate bond, which has been obtained by prereacting the polyol compound according to any of (1) to (4) and a compound having at least one isocyanate group and at least one (meth)acryloyl group in a molecule.
(8) The transparent molded article according to any of (5) to (7), wherein the transparent molded article is a lens.
(9) The transparent molded article according to (8), wherein the lens is an eyeglass lens.
   Furthermore, as a result of further extensive research, the present inventors discovered that the above second object was achieved by a transparent molded article obtained by polymerizing a prescribed isocyanate terminal prepolymer comprising intramolecular sulfur atoms and a prescribed aromatic diamine. The second aspect of the present invention was devised on this basis.
   That is, the second aspect of the present invention to achieve the above second object is as follows;
(10) A transparent molded article obtained by polymerizing monomer components comprising the following components (C) and (D).
   Component (C): isocyanate terminal prepolymer in the form of a reaction product of an aliphatic diisocyanate having an intramolecular cyclic structure and a diol or dithiol comprising an intramolecular sulfur atom and having an average molecular weight of 300-2,500.
   Component (D): one or more aromatic diamines denoted by general formula (II). (In general formula (II), R₁, R₂ and R₃ are each independently any of a methyl, ethyl or thiomethyl group.) 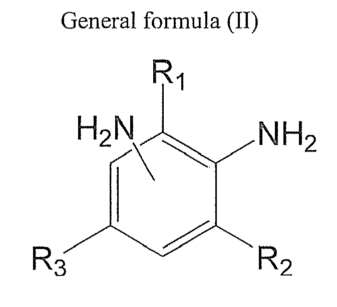
(11) The transparent molded article according to (10), wherein the aliphatic diisocyanate having an intramolecular cyclic structure, that is a starting material of component (C), is an alicyclic diisocyanate.
(12) The transparent molded article according to (11), wherein the alicyclic diisocyanate comprises a sulfur atom in its structure.
(13) The transparent molded article according to any of (10) to (12), wherein the diol or dithiol comprising an intramolecular sulfur atom and having an average molecular weight of 300-2,500, that is a starting material of component (C), comprises an intramolecular sulfur atom in the form of at least one bond from among a sulfide bond, disulfide bond, thioester bond, dithioester bond, thiocarbonate bond and dithiocarbonate bond.
(14) The transparent molded article according to any of (10) to (12), wherein the diol comprising an intramolecular sulfur atom and having an average molecular weight of 300-2,500, that is a starting material of component (C), is the polyol compound according to any of (2) to (4).
(15) The transparent molded article according to any of (10) to (14), wherein the isocyanate group content of component (A) ranges from 10 to 20 weight percent.
(16) The transparent molded article according to any of (10) to (15), wherein, in general formula (II), R₁ is a methyl group, and R₂ and R₃ are independently any of a ethyl group or thiomethyl group.
(17) The transparent molded article according to any of (10) to (16), wherein, in said polymerization, the molar ratio of the isocyanate group of component (C) to an amino group of component (D) ranges from 1.00 to 1.15.
(18) The transparent molded article according to any of claims (10) to (17), wherein the transparent molded article is a lens.
(19) The transparent molded article according to (18), wherein the lens is an eyeglass lens.
(20) A method of manufacturing a transparent molded article, comprising casting a mixture of component (C) and component (D) defined in any of (10) to (19) into a casting mold and then polymerizing component (C) and component (D) to obtain a molded article.

### BRIEF DESCPJPTIONS OF THE DRAWINGS

Fig. 1 is a 'H-NMR spectrum of the polyol compound of the present invention obtained in Example 1.
Fig. 2 is a ¹H-NMR. spectrum of the polyol compound of the present invention obtained in Example 2.

### BEST MODE FOR CARRYING OUT THE INVENTION

The first aspect of the present invention is a polyol compound denoted by general formula (I) and a transparent molded article obtained by polymerizing monomer components comprising the aforementioned polyol compound.

The second aspect of the present invention is a transparent molded article obtained by polymerizing monomer components comprising component (C) and component (D), and a method of manufacturing a transparent molded article, comprising casting a mixture of component (C) and component (D) into a casting mold and then polymerizing component (C) and (D) to obtain a molded article.

In the present invention, any molded article having transparency that does not impede its use as an optical material is covered under the term "transparent molded article." There are many indexes of transparency employed for the various optical materials used in the transparent molded article of the present invention. Examples are light transmittance, the haze value, and visual inspection.

In some cases below, the "transparent molded article" will be referred to simply as a "molded article" below.

### First aspect

### (Polyol compound)

The polyol compound of the present invention is denoted by general formula (I) below: 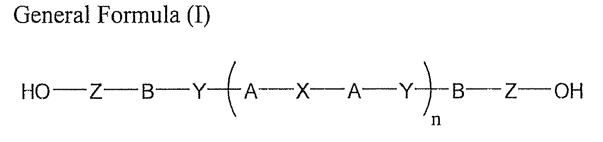 (wherein X and Y each independently denote alkylene groups comprising 3 to 12 carbon atoms, in which at least one of methylene groups (excluding terminals) contained is substituted with a sulfur atom (without direct bonding between sulfur atoms); Z denotes an alkylene group comprising 2 to 6 carbon atoms, in which a methylene group (excluding terminals) contained is optionally substituted with a sulfur atom (without direct bonding between sulfur atoms); A and B each independently denote an ester group, thioester group, carbamate group, or thiocarbamate group; and n denotes an integer ranging from 0 to 12).

The above polyol compound comprises sulfur atoms at prescribed positions within the molecule. The refractive index of the transparent molded article obtained by polymerizing monomer components comprising a polyol compound can be increased by incorporating sulfur atoms. Further, the position at which the sulfur atoms are introduced greatly affects the increase in the Abbé number of the transparent molded article. A transparent molded article in which sulfur atoms are incorporated at positions within the molecule capable of increasing the Abbé number, and which possesses both a high refractive index and a high Abbé number, can be obtained based on the polyol compound of the present invention, which comprises sulfur atoms at prescribed positions within the molecule.

However, when a bond between sulfur atoms is contained in any one from among X, Y, and Z in general formula (I) above, despite effectively increasing the refractive index of the transparent molded article obtained using the material, this bond lowers the Abbé number and compromises coloration and weatherability. Accordingly, in the present invention, neither X, Y, nor Z in general formula (I) contains a bond between sulfur atoms.

In the present invention, X and/or Y in general formula (I) desirably comprises the following structure:

-S-CH₂-S-.

When a methylene group is present between sulfur atoms, while a decrease in the Abbé number and deterioration in coloration and weatherability are inhibited in the transparent molded article obtained by polymerization of monomer components comprising the polyol compound of the present invention, the rate of introduction of sulfur atoms can be increased to improve the refractive index.

X and Y in general formula (I) above denote alkylene groups comprising 3 to 12 carbon atoms, in which at least one of the methylene groups (excluding terminals) contained is substituted with a sulfur atom. The number of carbon atoms must be three or more because at least one of the methylene groups, excluding terminals, is substituted with a sulfur atom. When a terminal is substituted with a sulfur atom, the ester group, thioester group, carbamate group, or thiocarbamate group in general formula (I) above bonds with a sulfur atom. As a result, there is a risk of the polyol compound itself becoming unstable. Further, even when the polyol compound itself is stable, there are problems in that the Abbé number of the molded article obtained by using the polyol compound decreases and weatherability deteriorates. For these reasons, in X and Y in general formula (I), the methylene group substituted with a sulfur atom is not a terminal group. So long as the carbon number is less than or equal to 12, there is good compatibility with other monomers when obtaining a molded article by polymerizing monomer components including the polyol compound of the present invention. As a result, optical transparency can be imparted to the molded article obtained. The number of carbon atoms desirably ranges from 5 to 9.

Z in general formula (1) denotes an alkylene group having 2 to 6 carbon atoms, in which a methylene group (excluding terminal groups) contained may be substituted with a sulfur atom. For the same reasons as above, in Z of general formula (I), the methylene groups, that may be substituted with a sulfur atom, are not terminal groups. So long as the number of carbon atoms falls within a range of 2 to 6, a good balance between heat resistance and impact resistance is achieved in the molded article obtained by polymerizing monomer components comprising the polyol compound of the present invention as well as good compatibility is maintained with other monomers. As a result, the molded article obtained is imparted with optical transparency.

Among X, Y, and Z of general formula (I), so long as sulfur atoms are not bonded together and none of the terminal methylene groups are substituted, the greater the number of sulfur atoms substituted for methylene groups the better, as this imparts a higher refractive index and Abbé number to the molded article obtained by polymerizing the polyol compound of the present invention.

Further, the polyol compound of the present invention comprises intramolecular ester bonds, thioester bonds, carbamate bonds, or thiocarbamate bonds. Thus, the transparent molded article obtained using the polyol compound is imparted with intramolecular interactions and intermolecular interactions derived from these bonds. As a result, the transparent molded article can be imparted with good mechanical characteristics such as high impact resistance. Further, it is preferable that the polyol compound of the present invention contains intramolecular ester groups or carbamate groups from the perspective of achieving stronger intramolecular and intermolecular interactions.

In general formula (1), n denotes an integer ranging from 0 to 12. When *n* is less than or equal to 12, the molded article obtained by polymerizing the polyol compound of the present invention maintains good mechanical characteristics while being able to hold its shape. From the perspective of ease of synthesis, *n* is desirably 0.

The average molecular weight of the polyol compound of the present invention desirably ranges from 300 to 2,500. In the present invention, the term "average molecular weight" refers to the number average molecular weight. When the average molecular weight of the polyol compound of the present invention is greater than or equal to 300, when polymerizing monomer components containing the polyol compound of the present invention with a crosslinking agent, a suitable distance between crosslinked points can be achieved and the mechanical characteristics of the molded article obtained can be improved. On the other hand, an average molecular weight of the polyol compound of the present invention of less than or equal to 2,500 is desirable in that the molded article obtained by polymerizing monomer components including the polyol compound can be imparted with good heat resistance.

Examples of the novel polyol compound of the present invention are as follows;

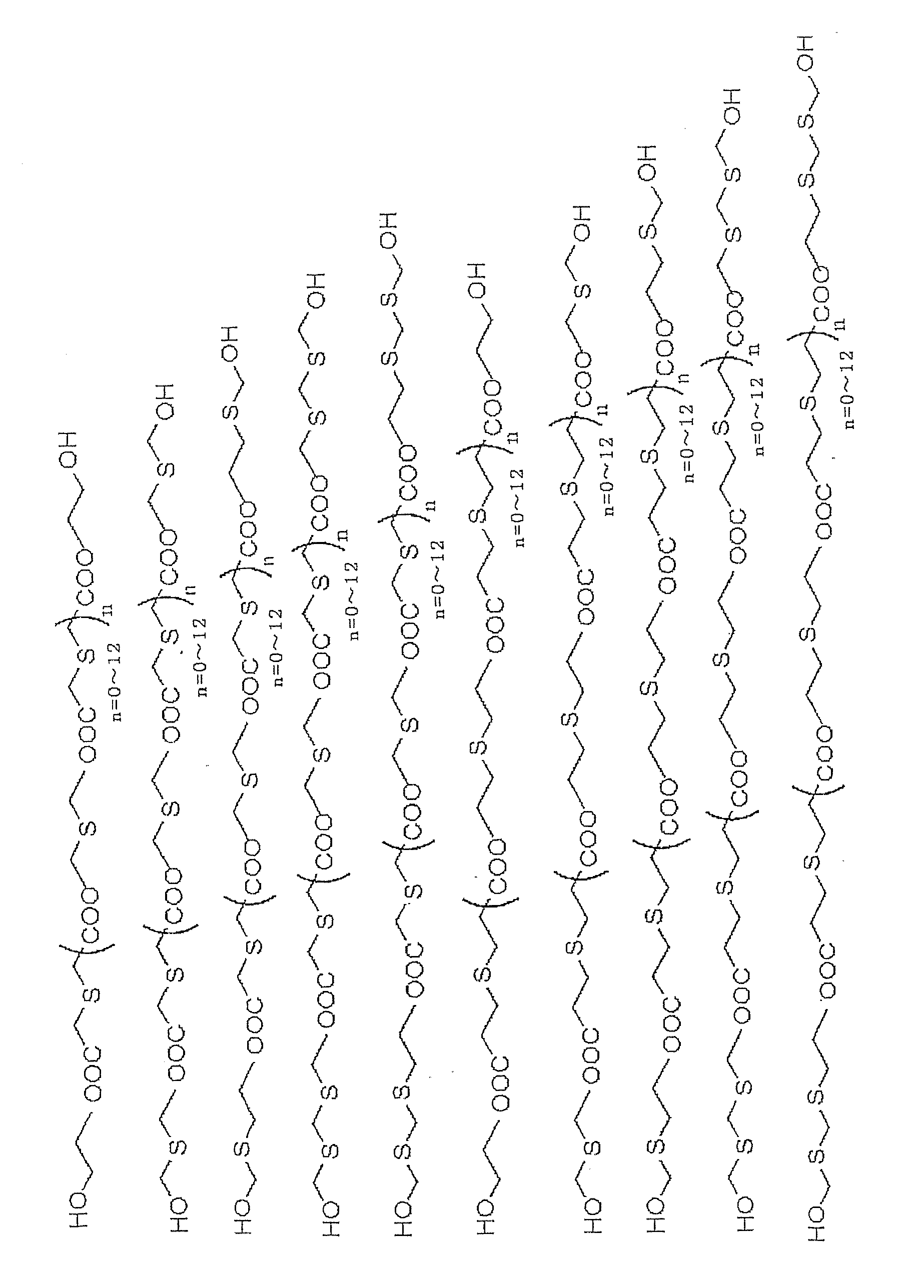

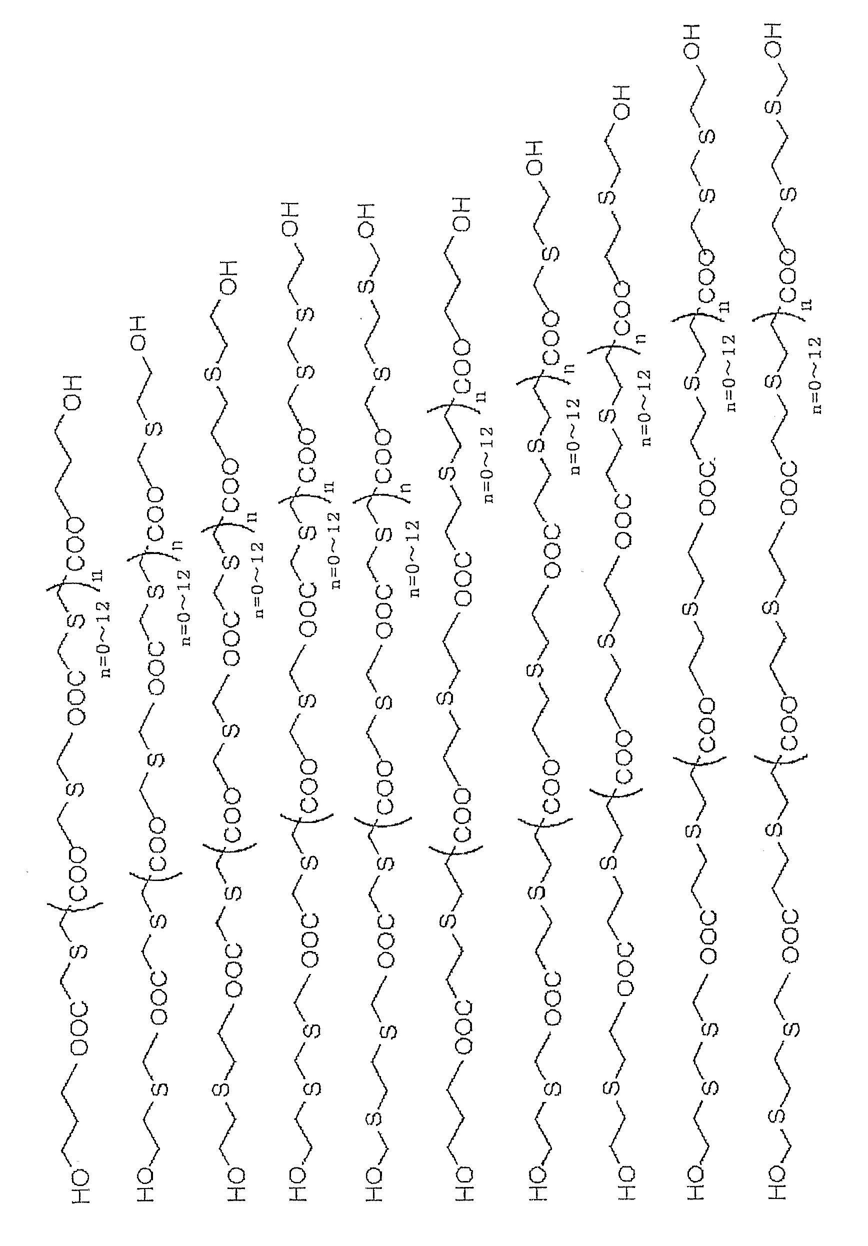

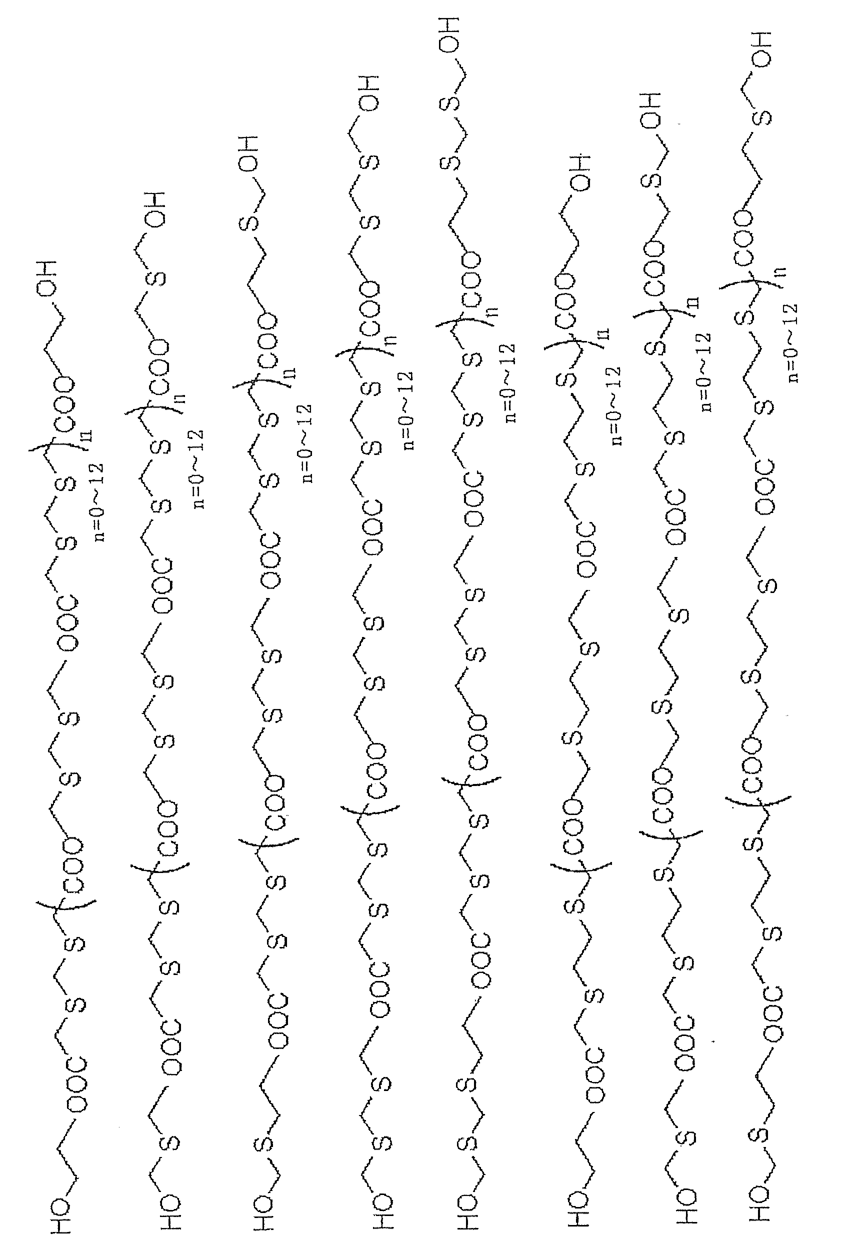

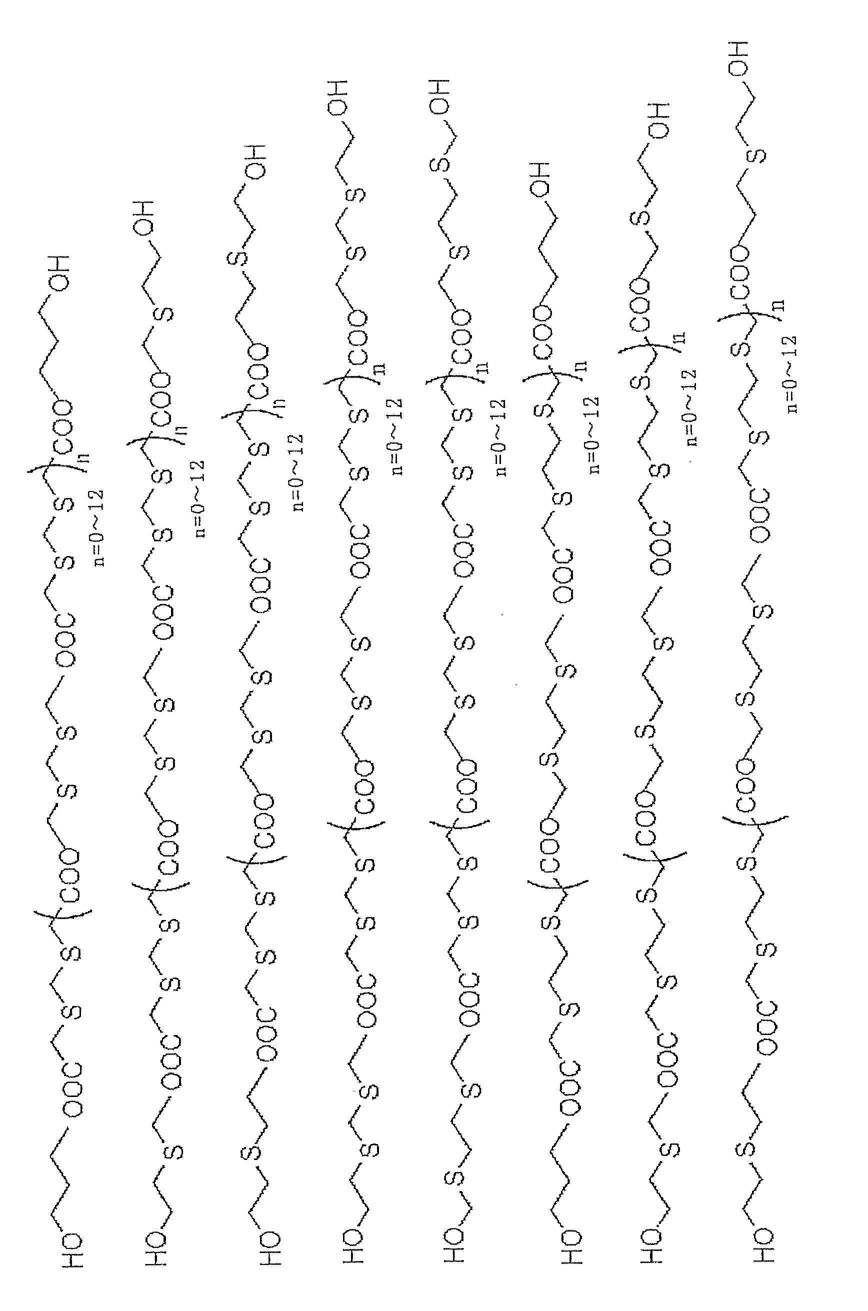

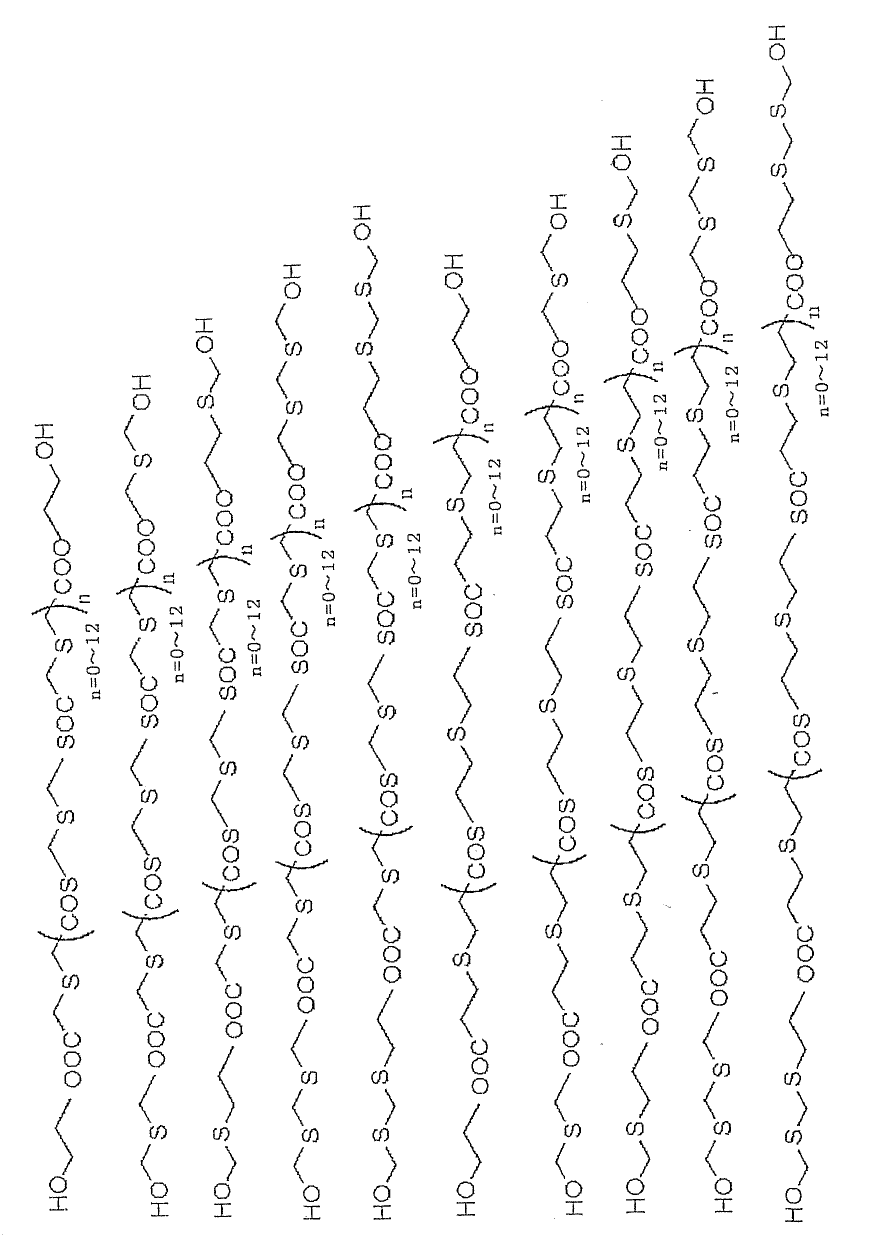

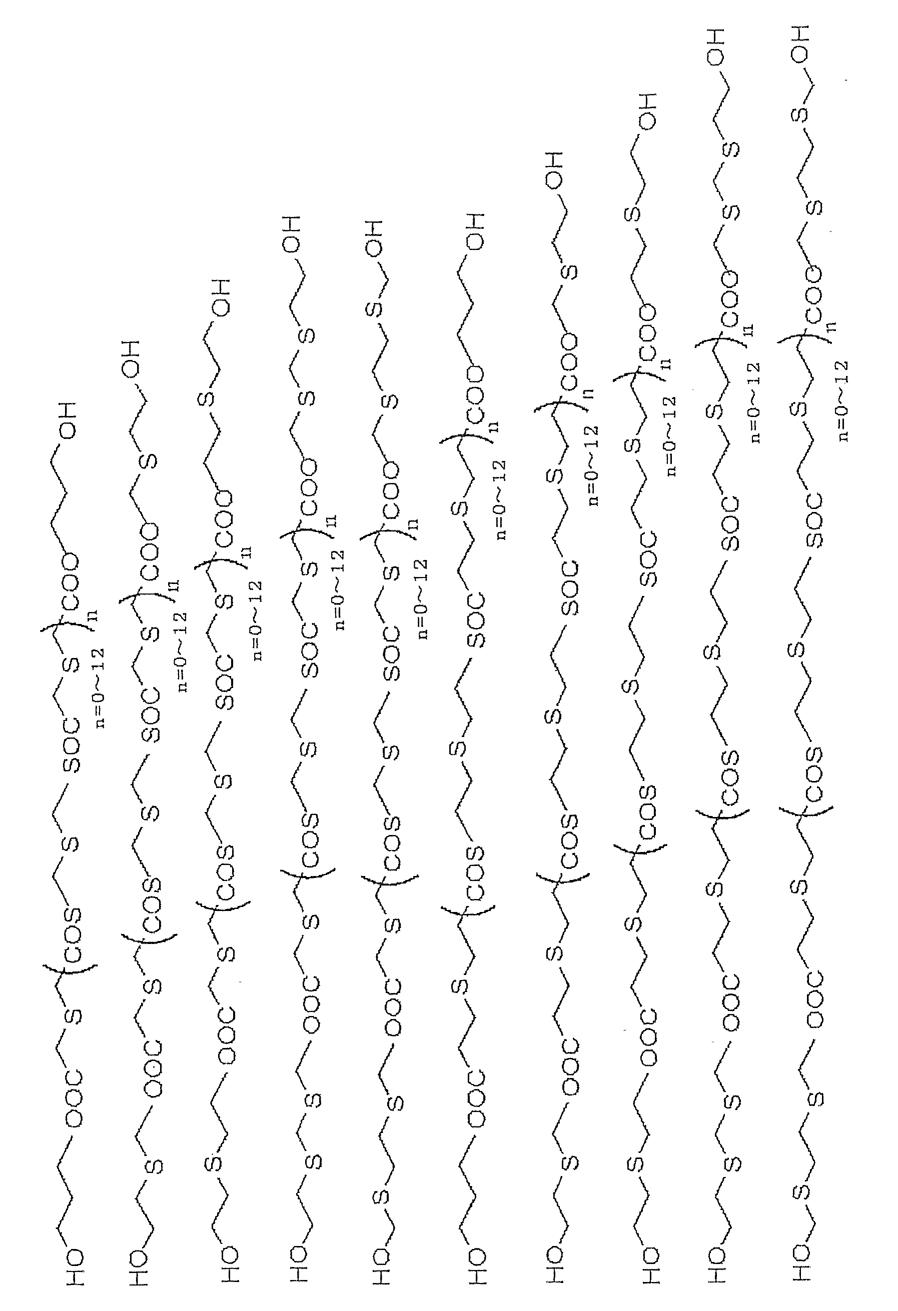

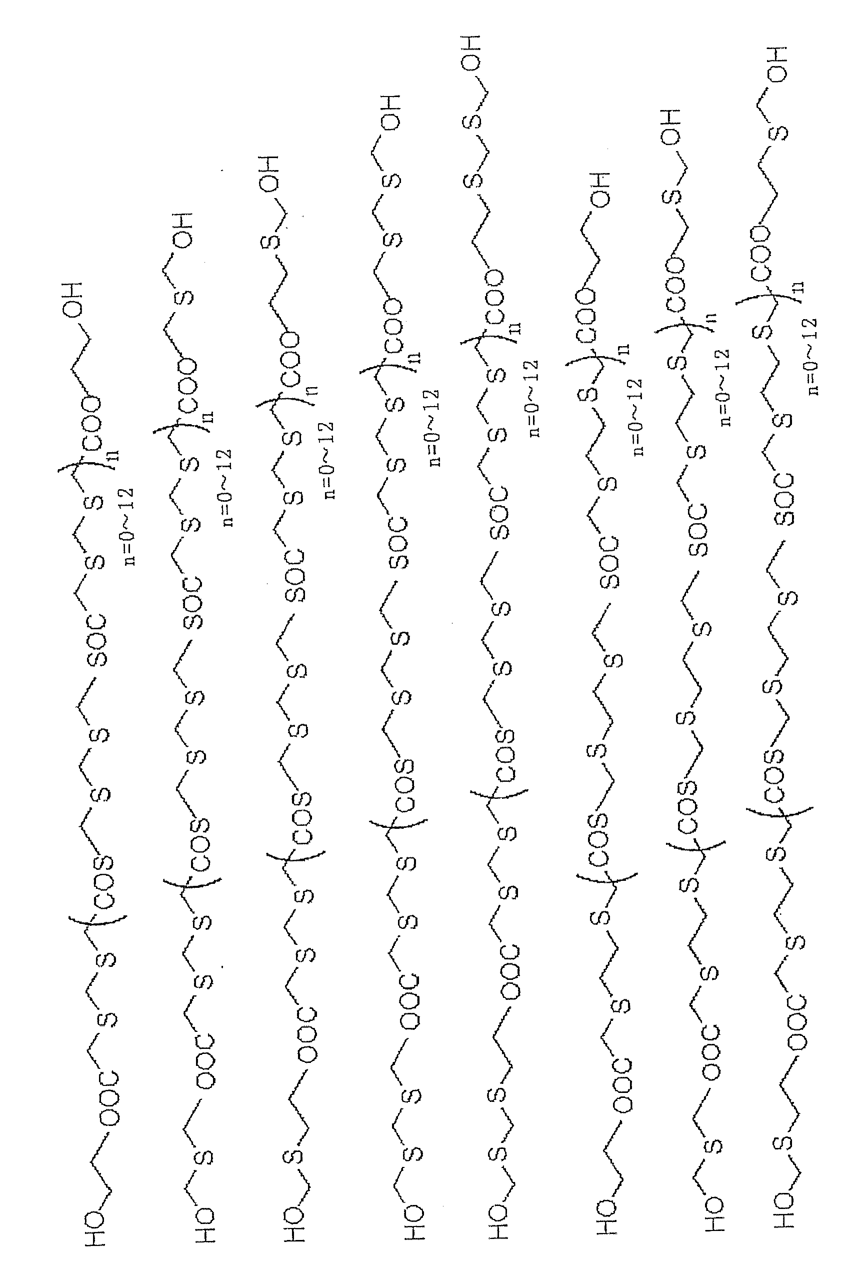

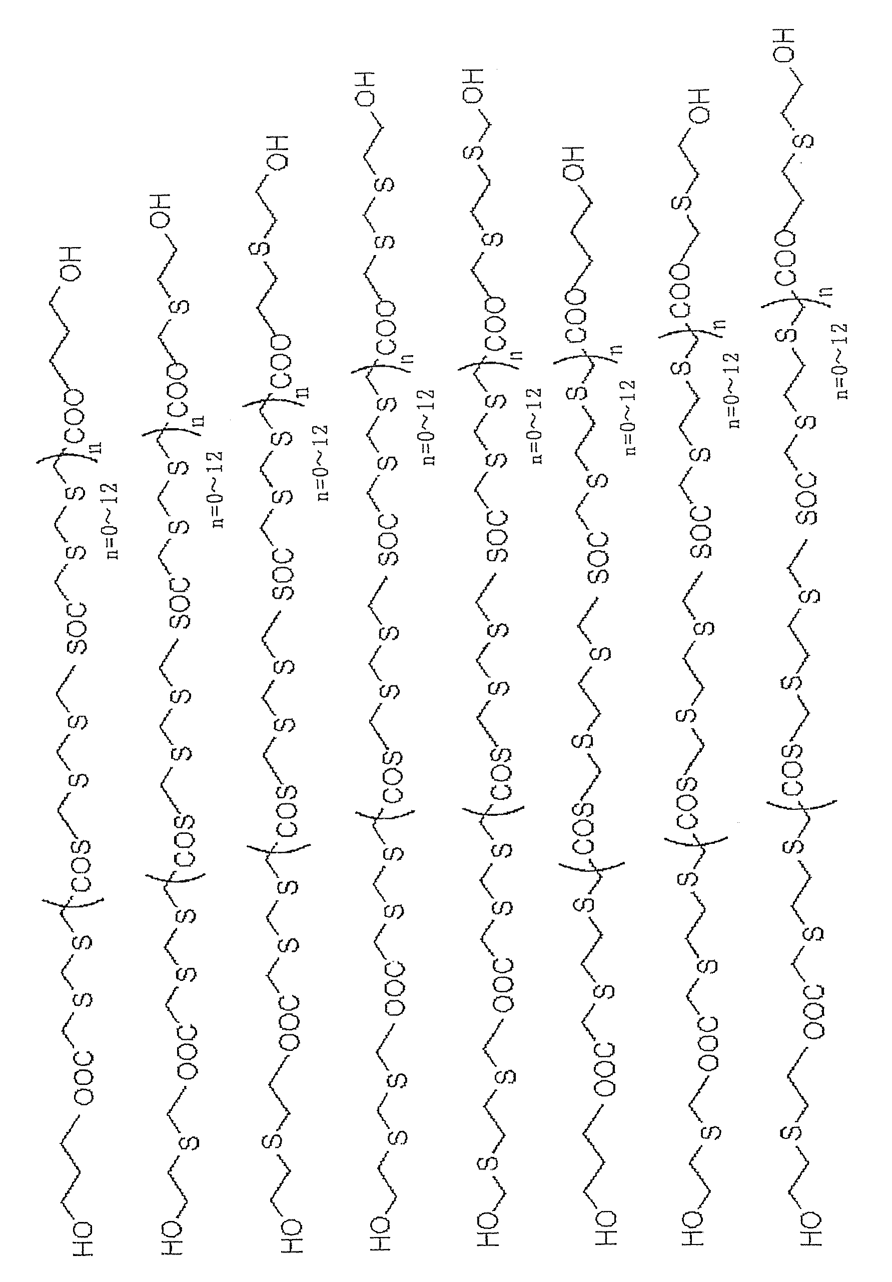

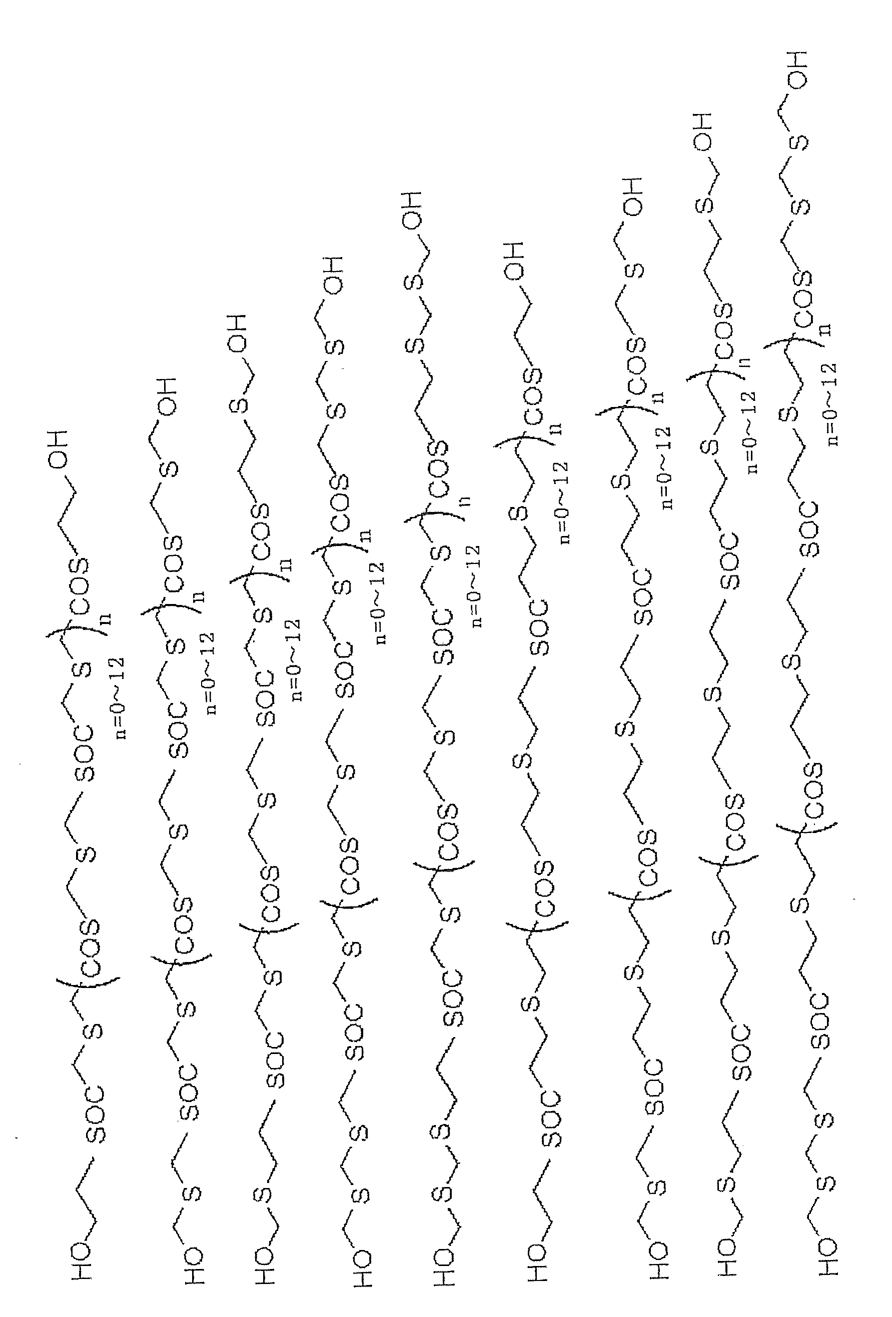

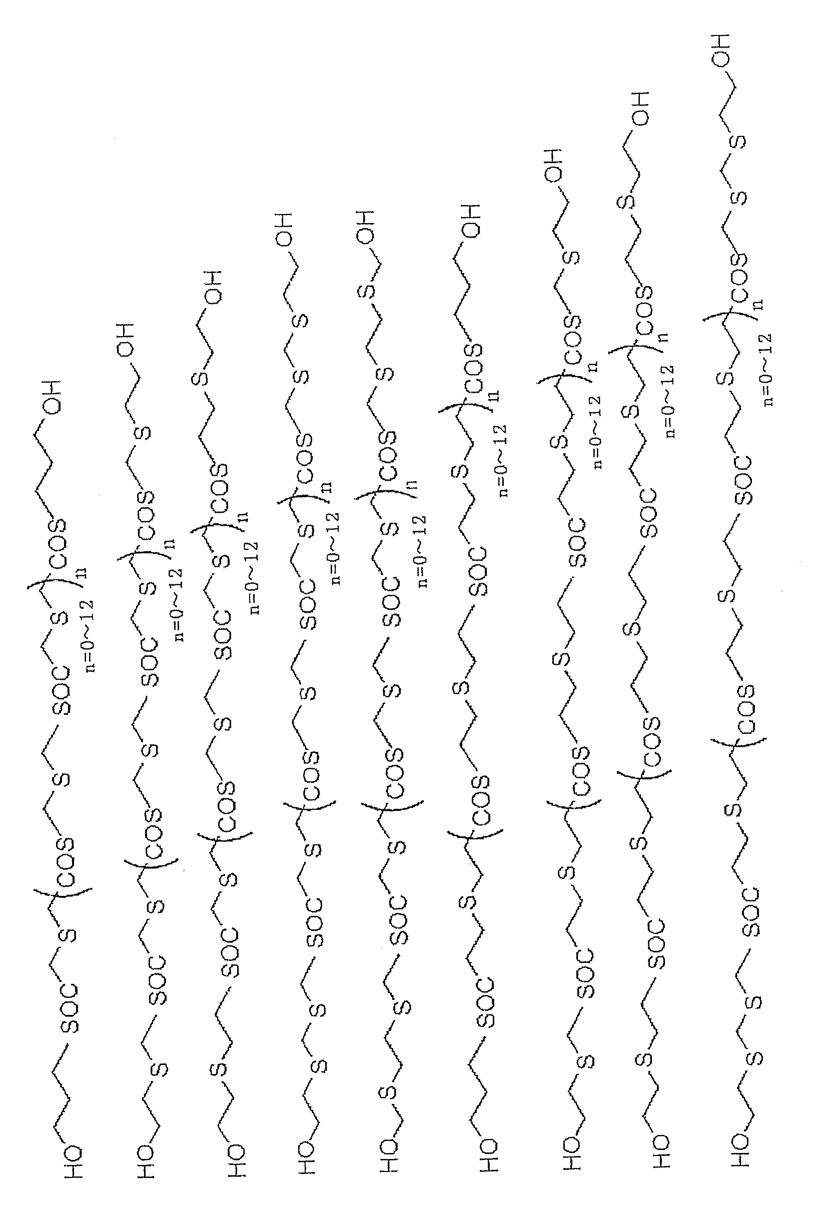

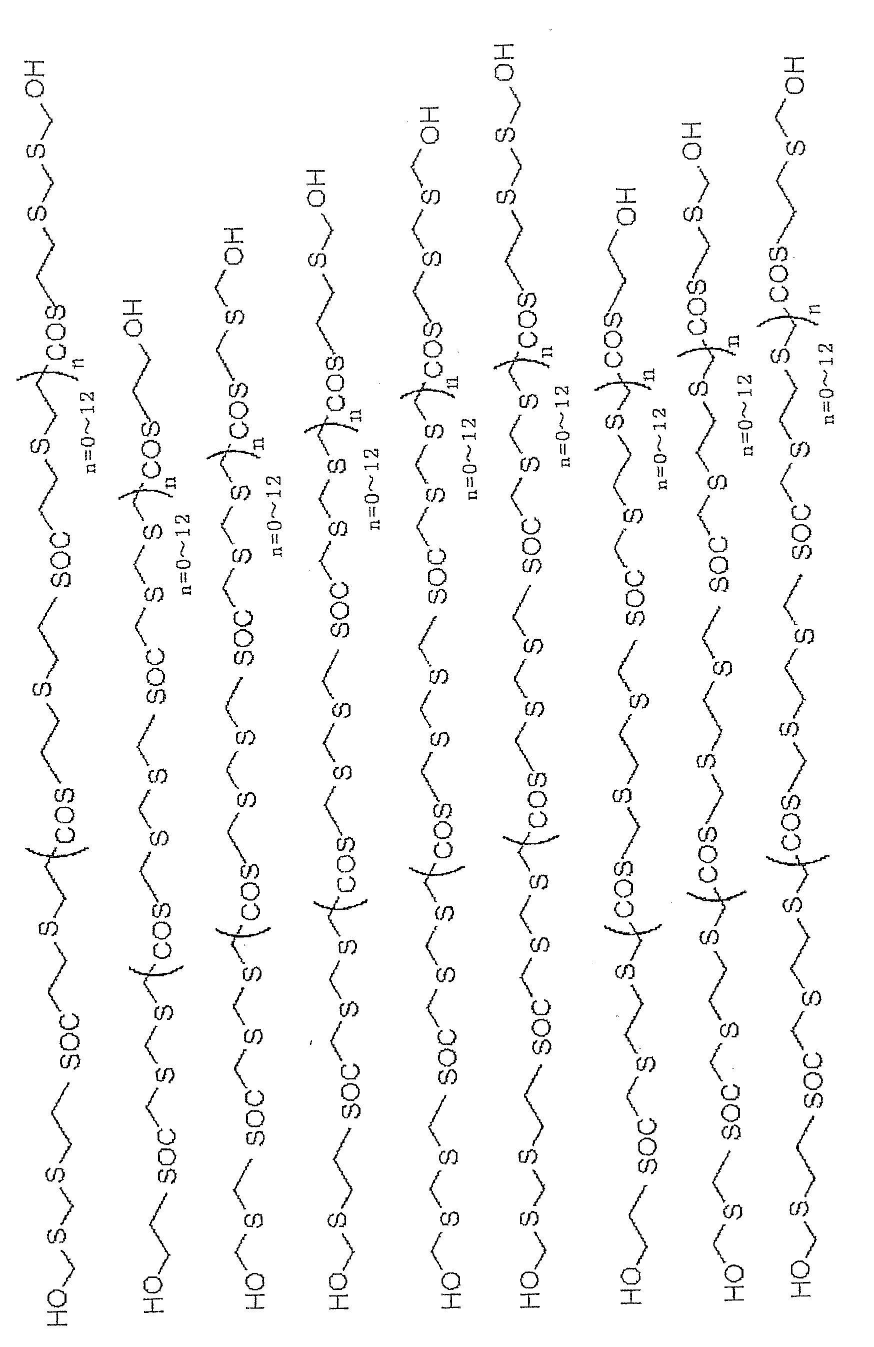

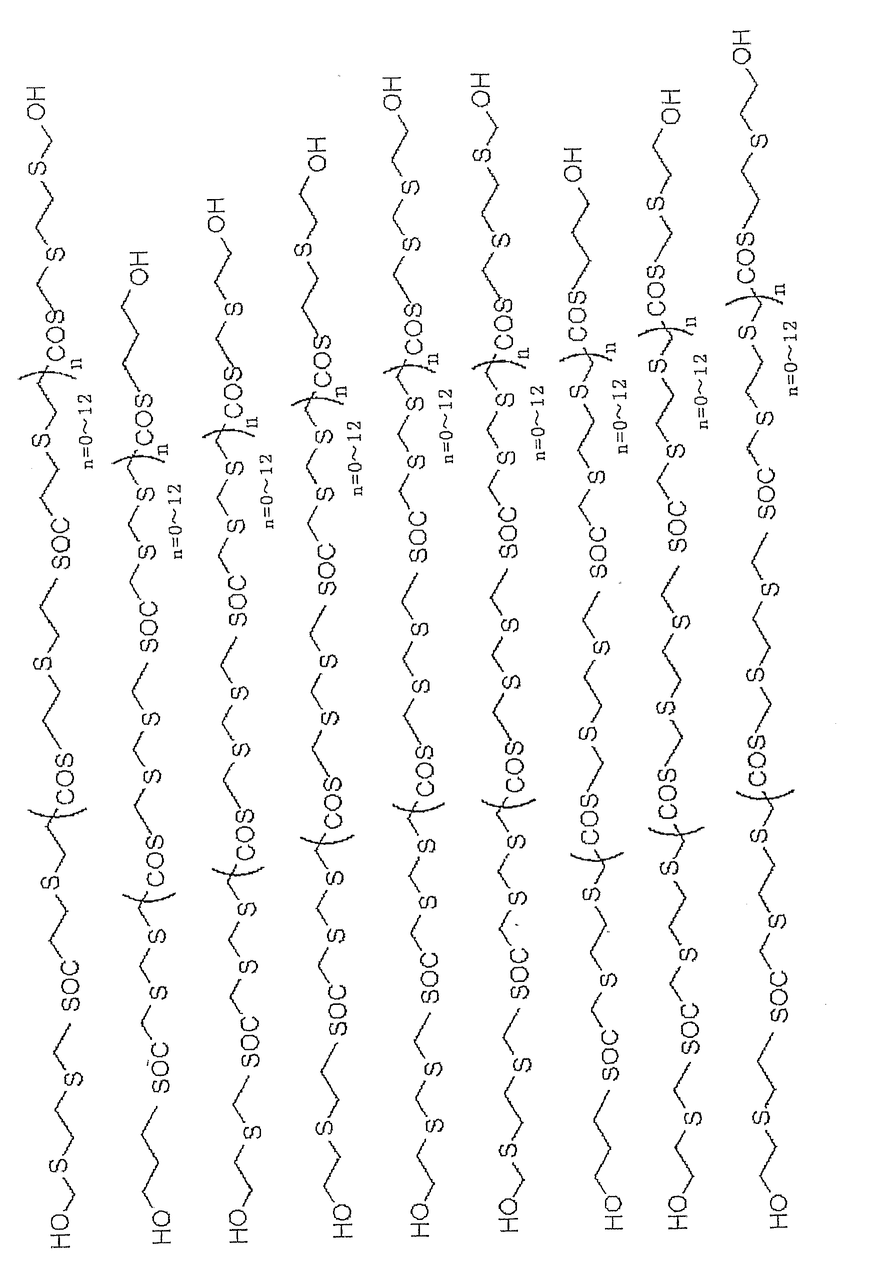

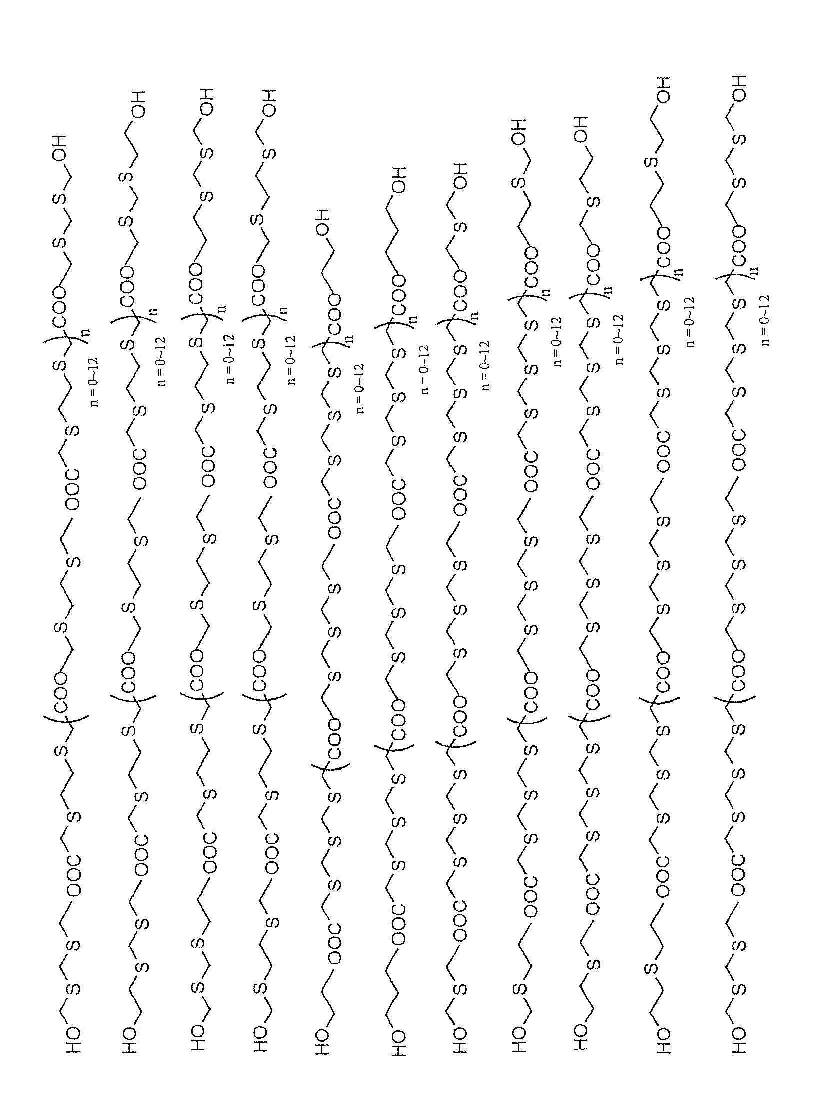

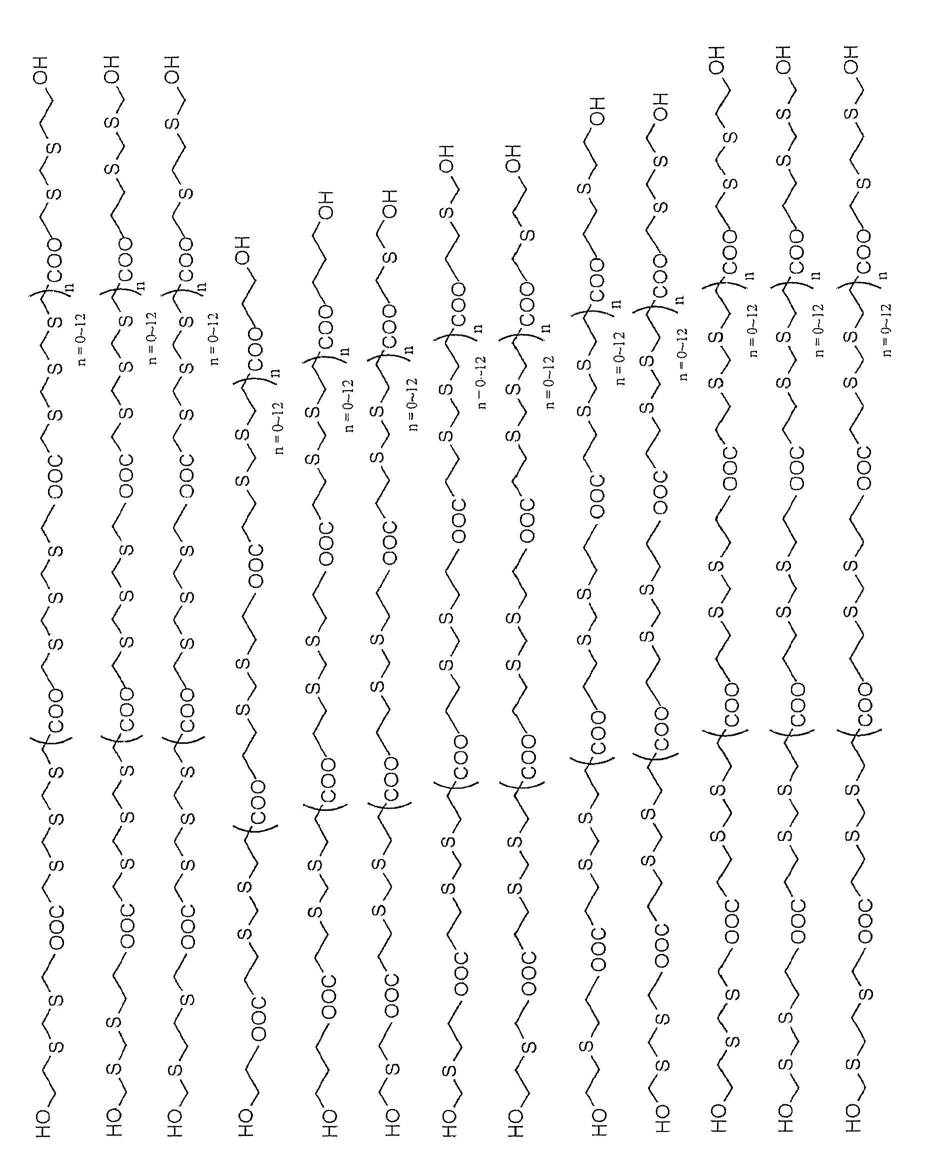

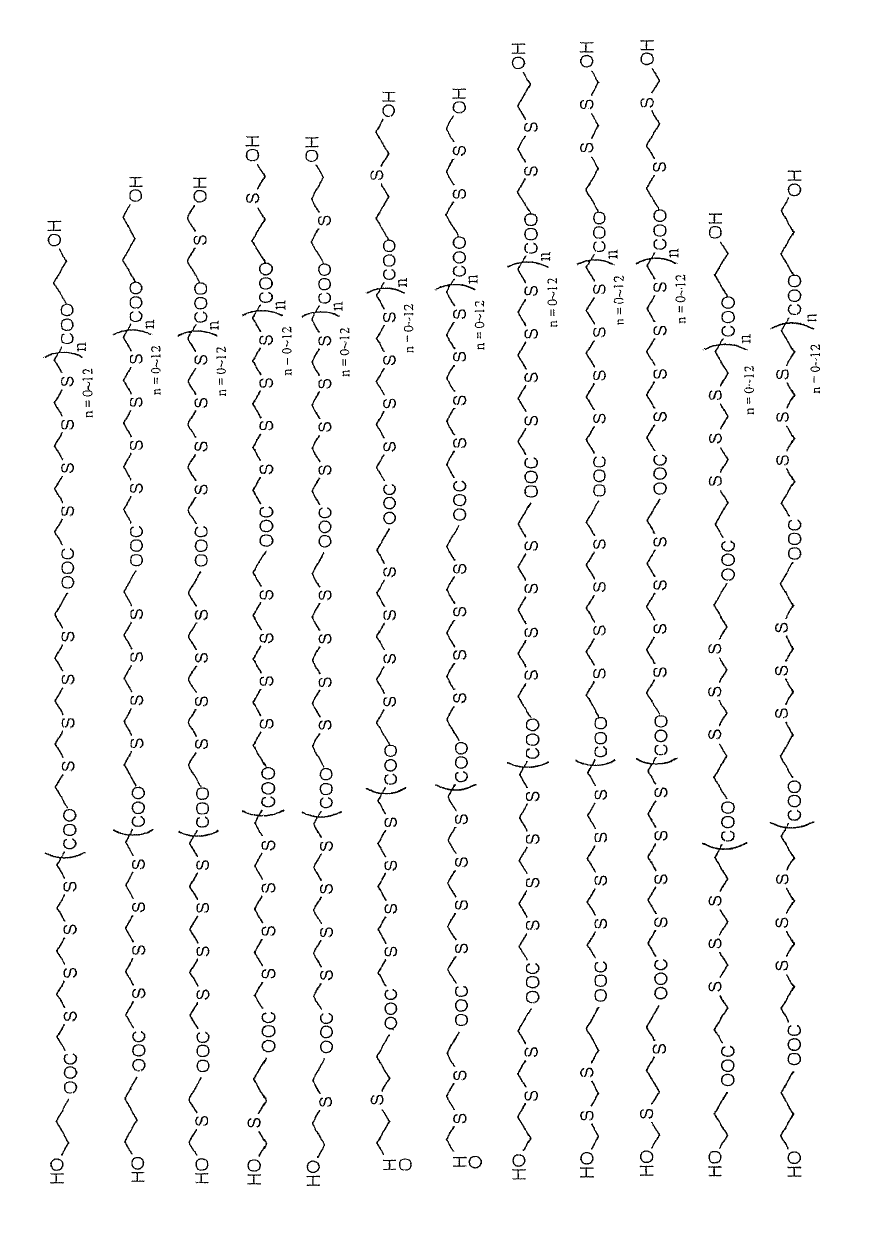

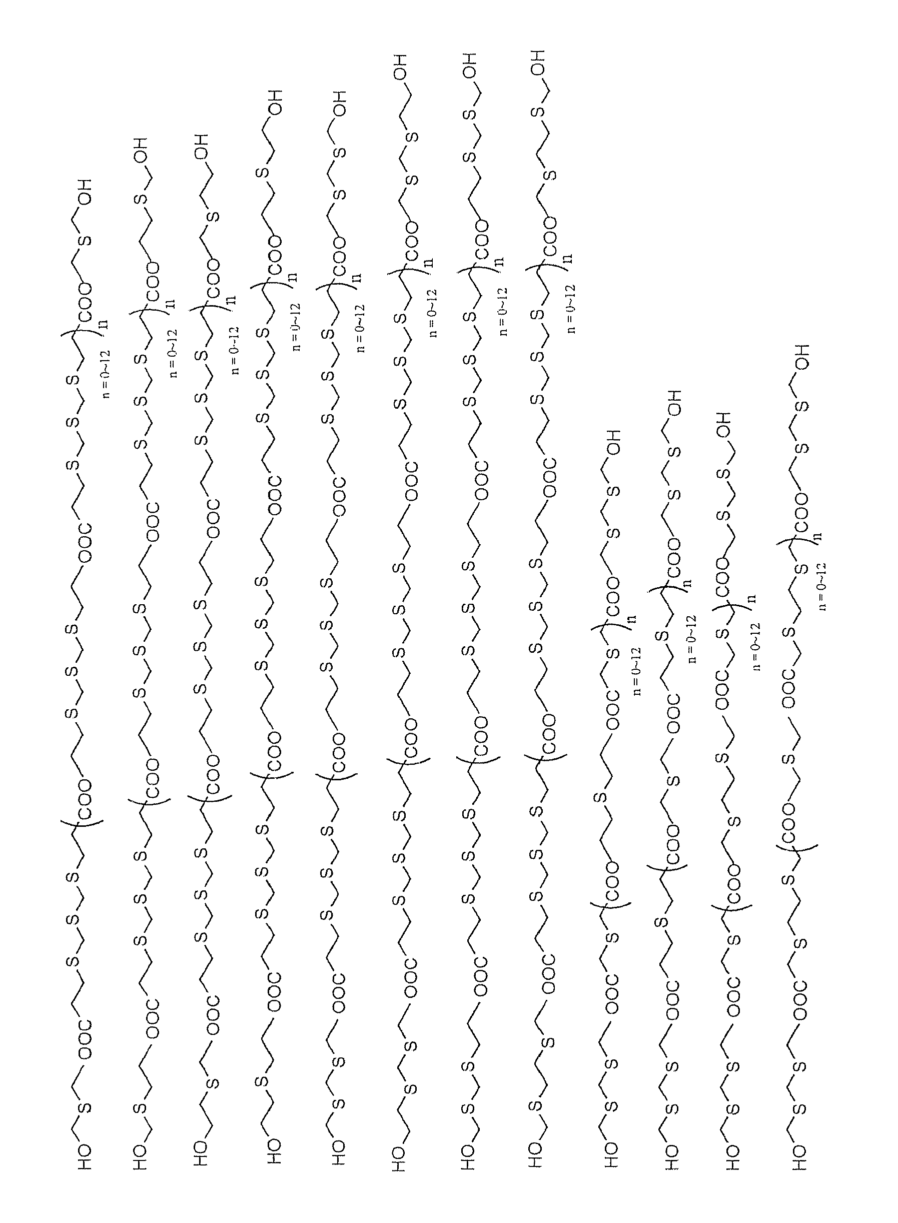

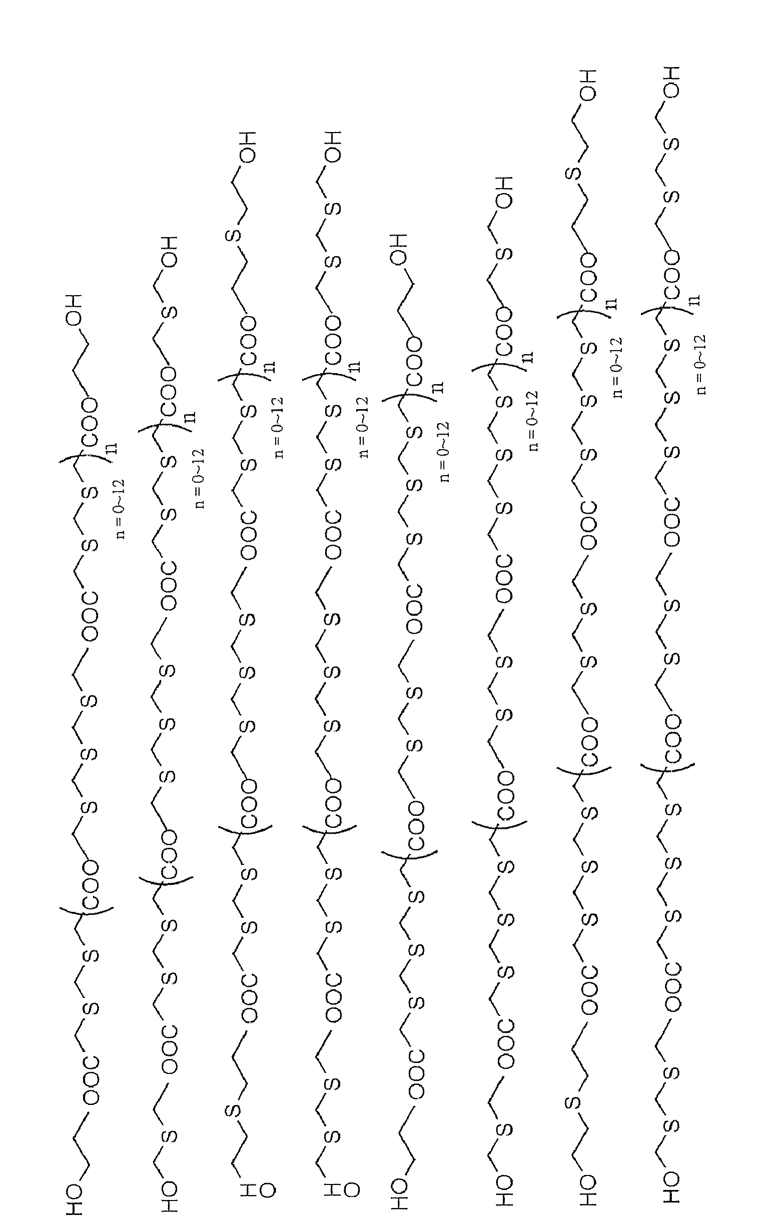

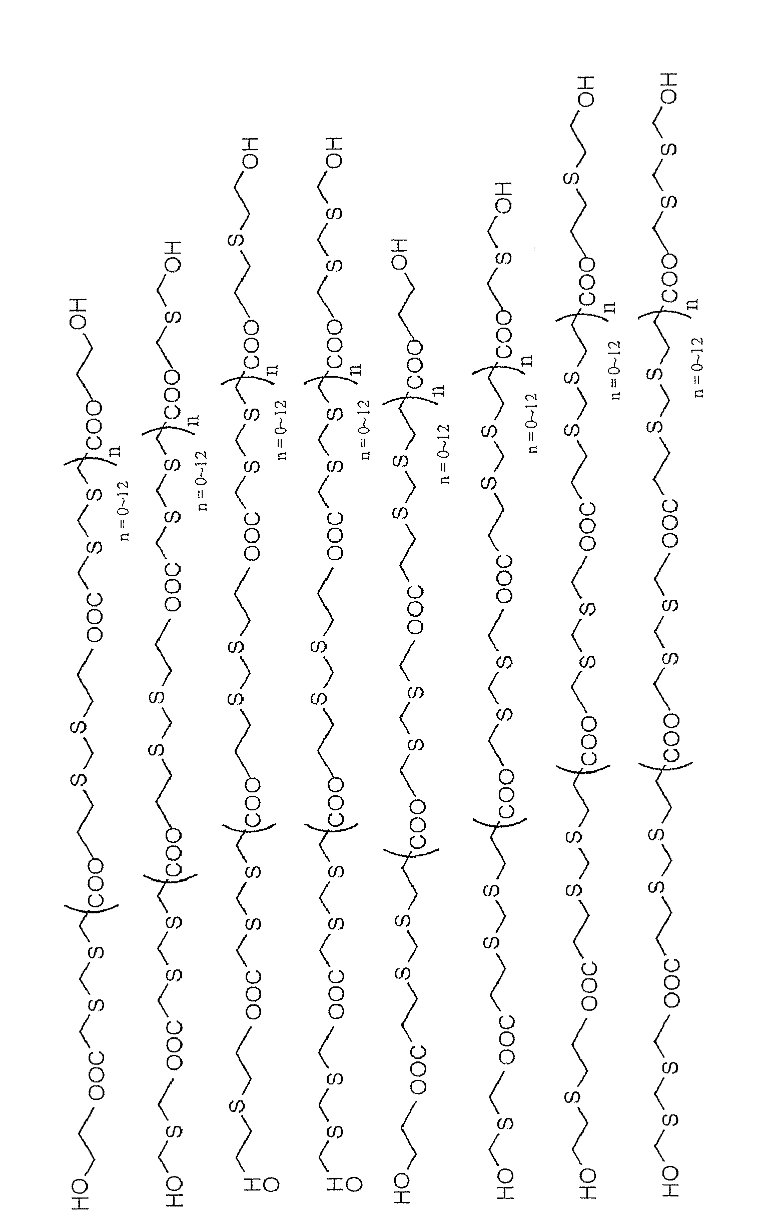

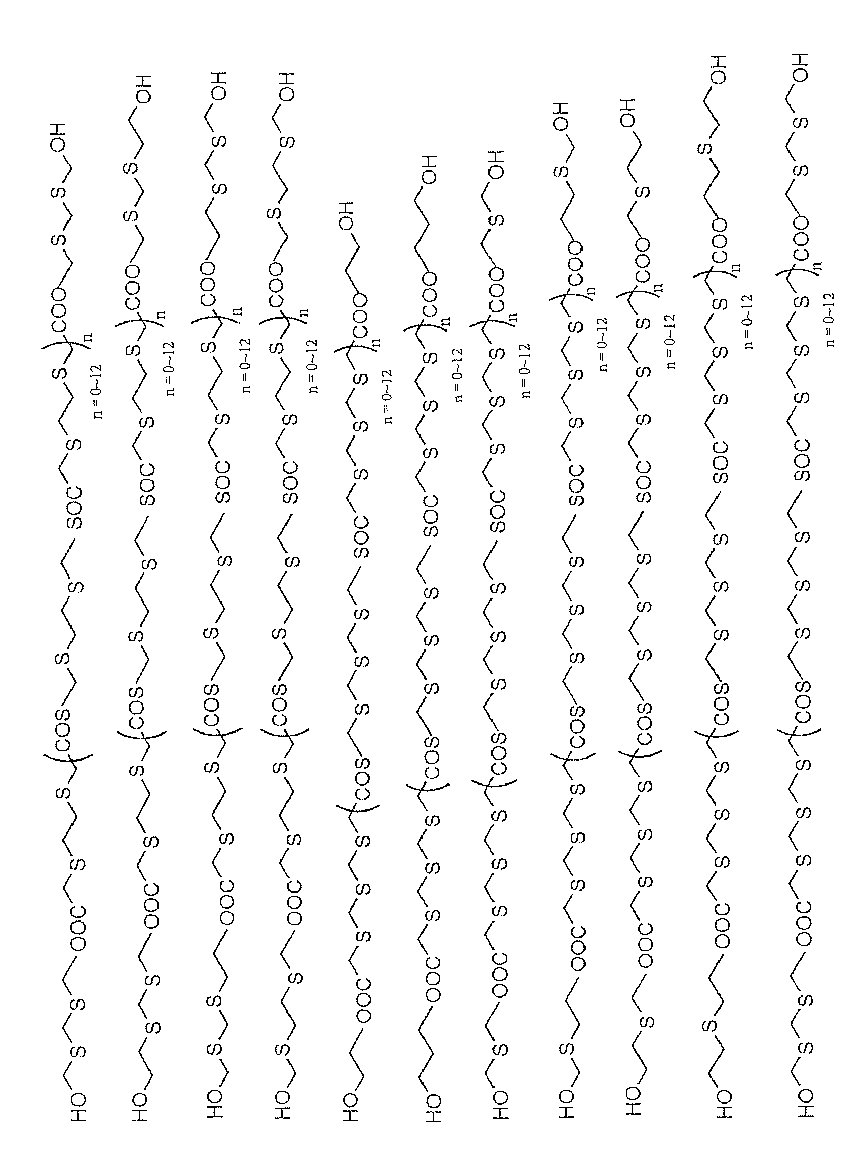

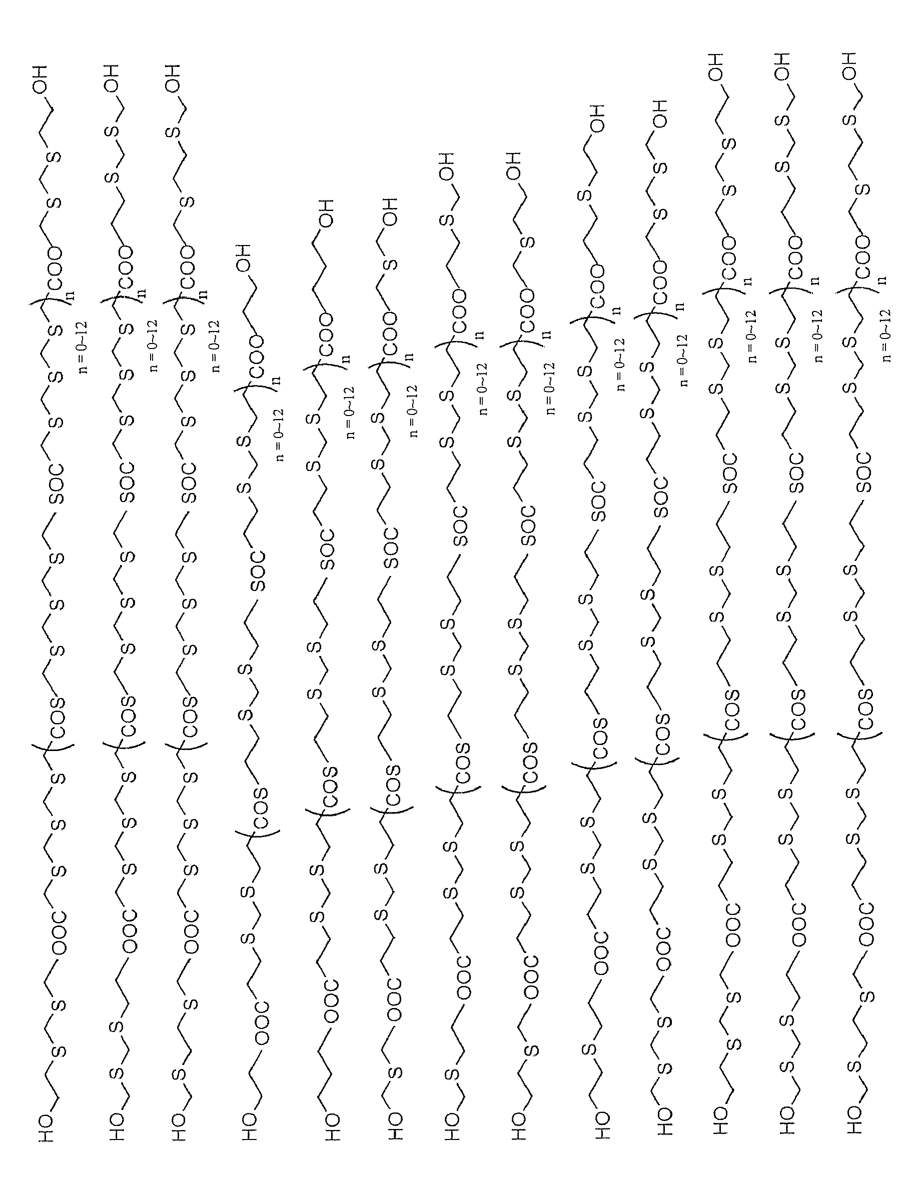

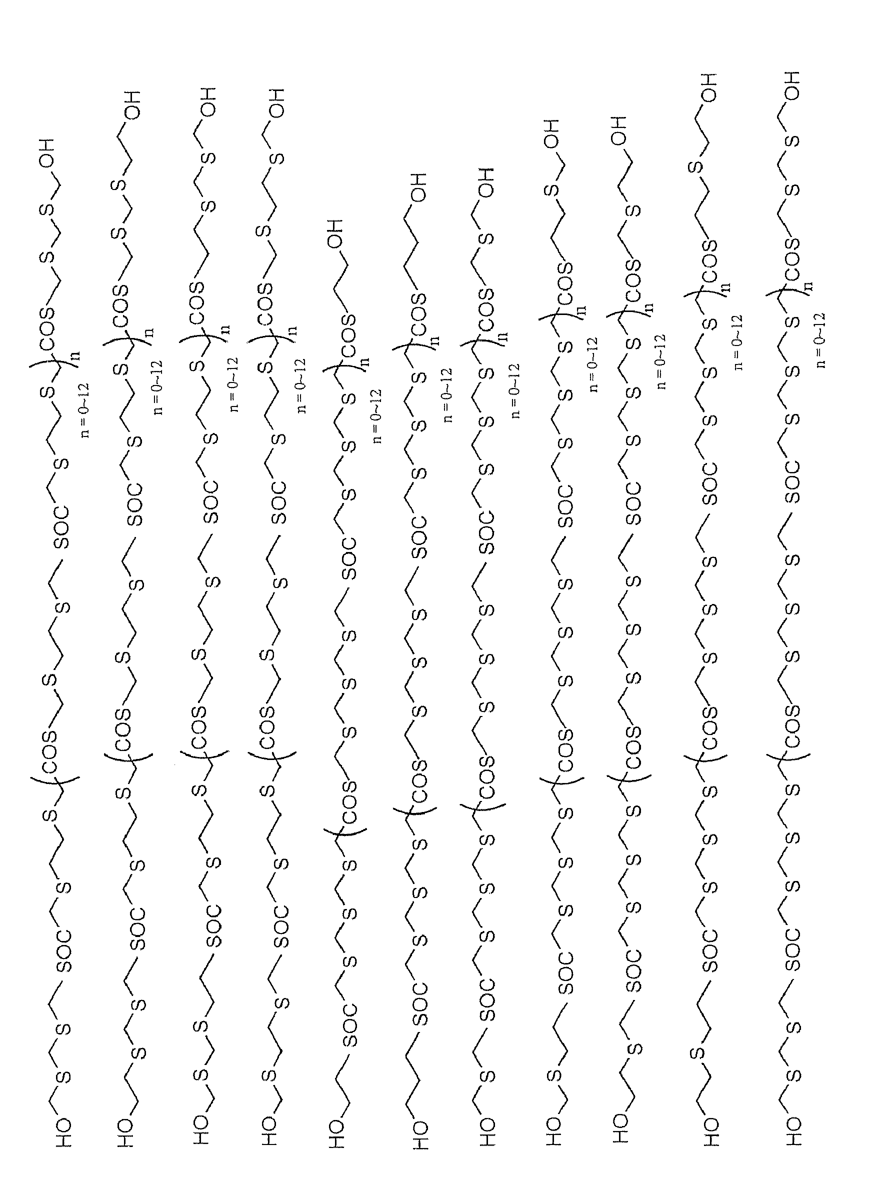

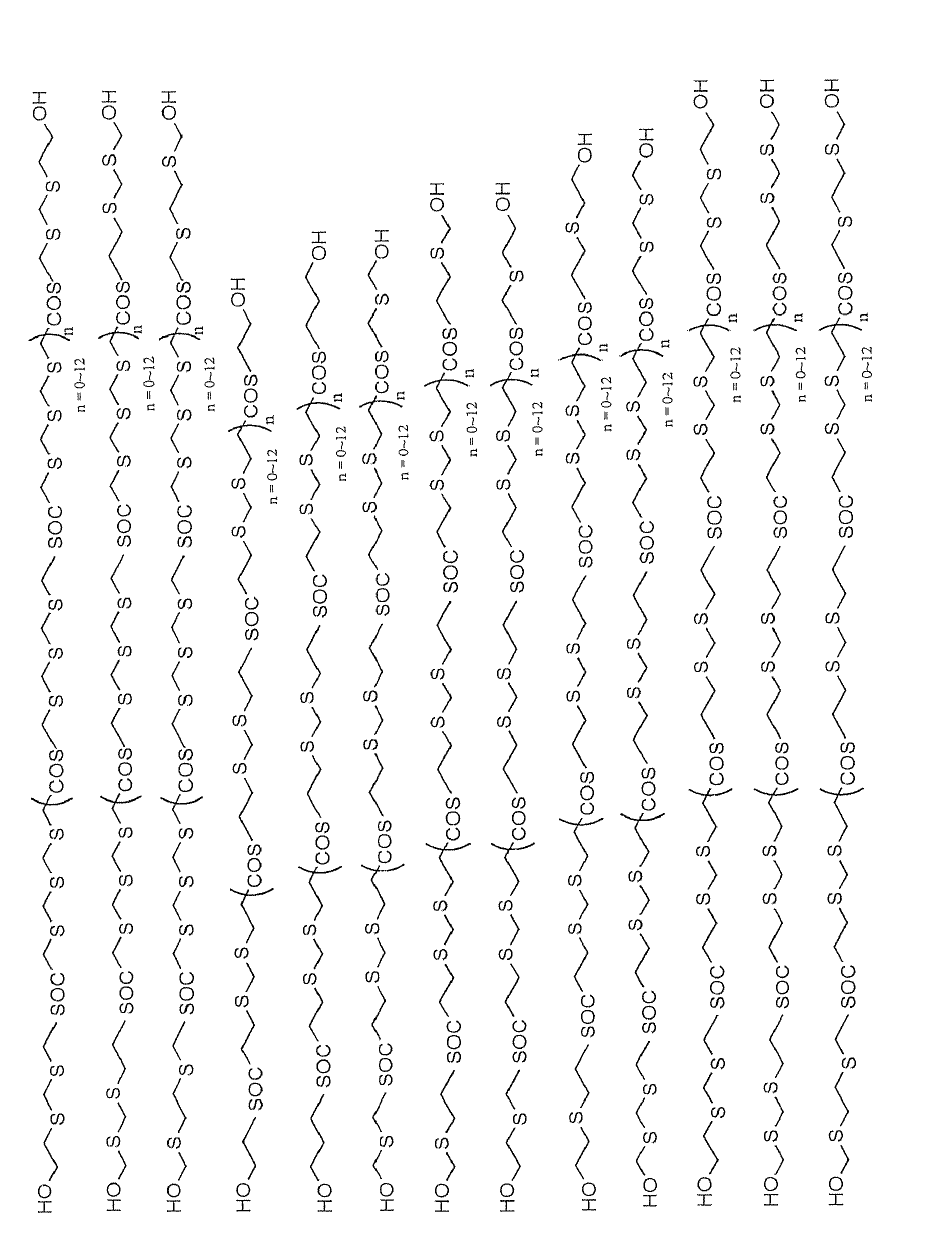

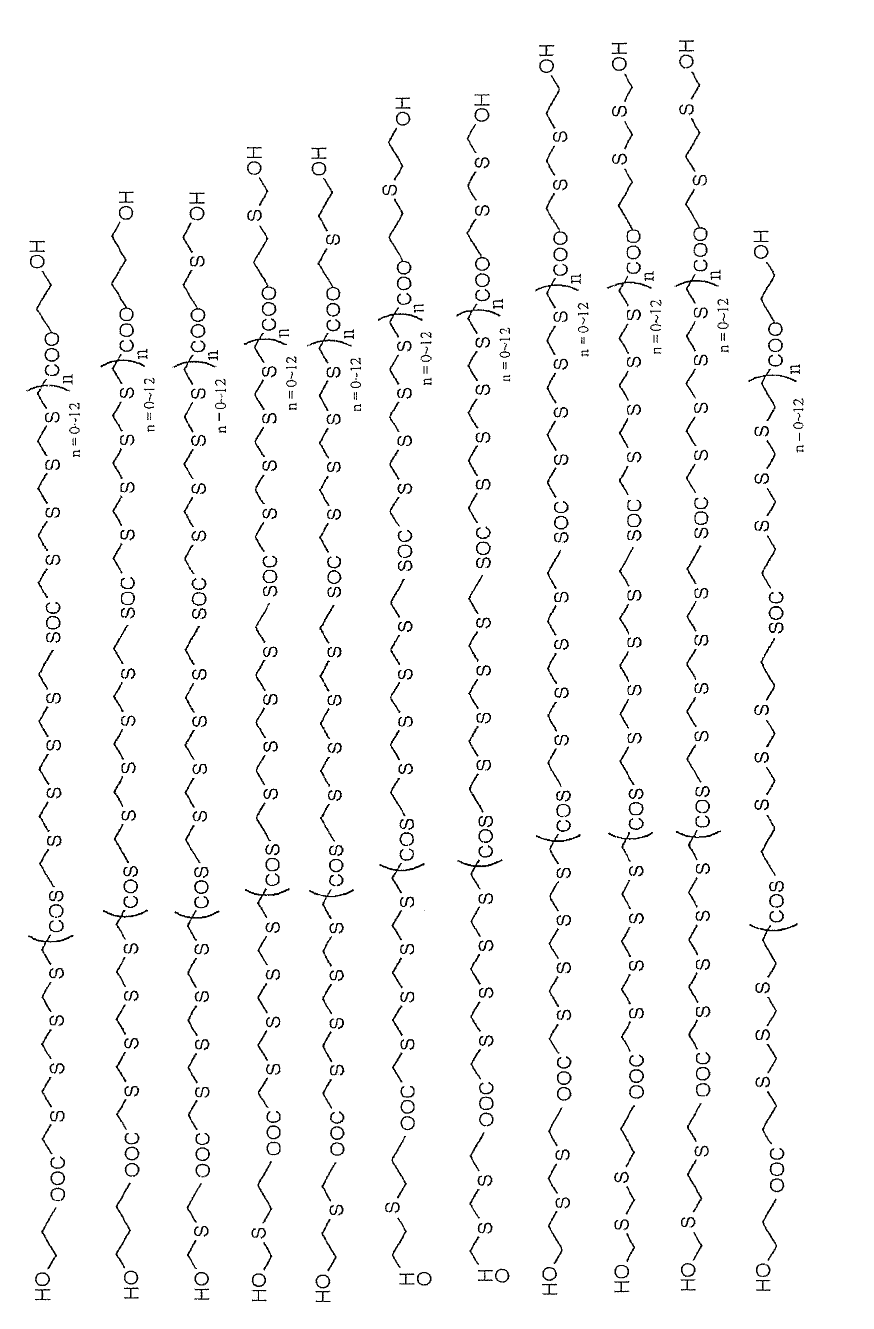

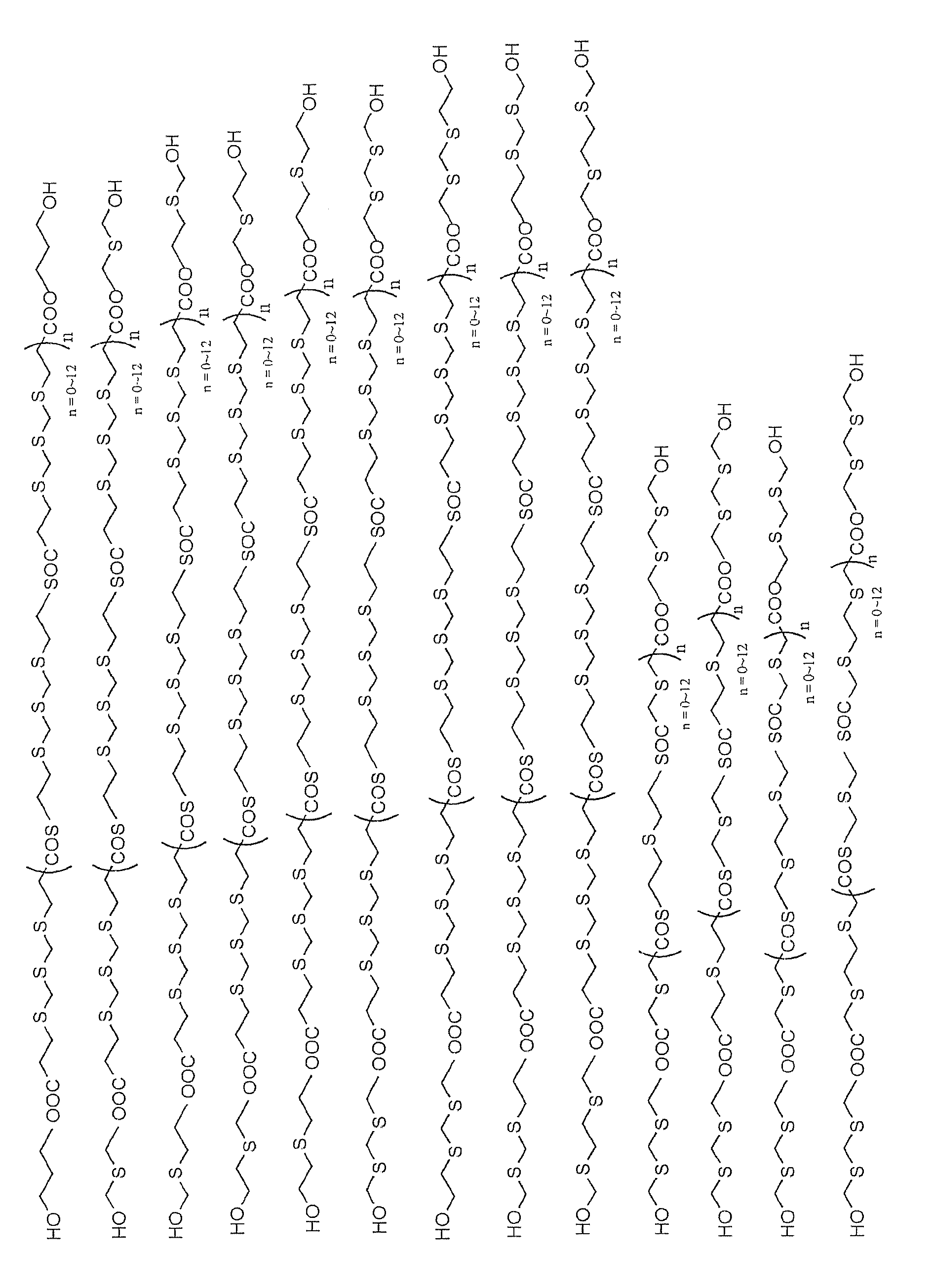

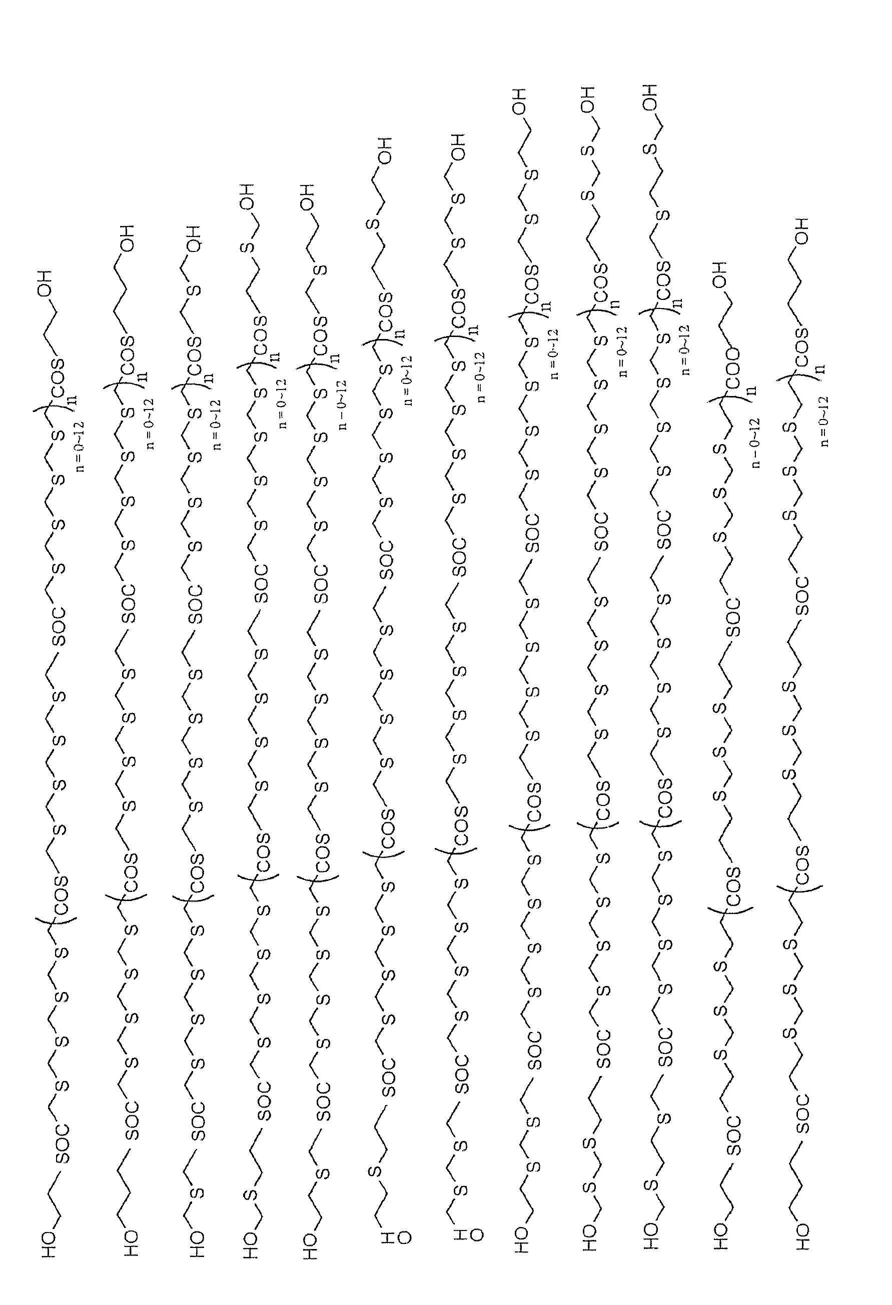

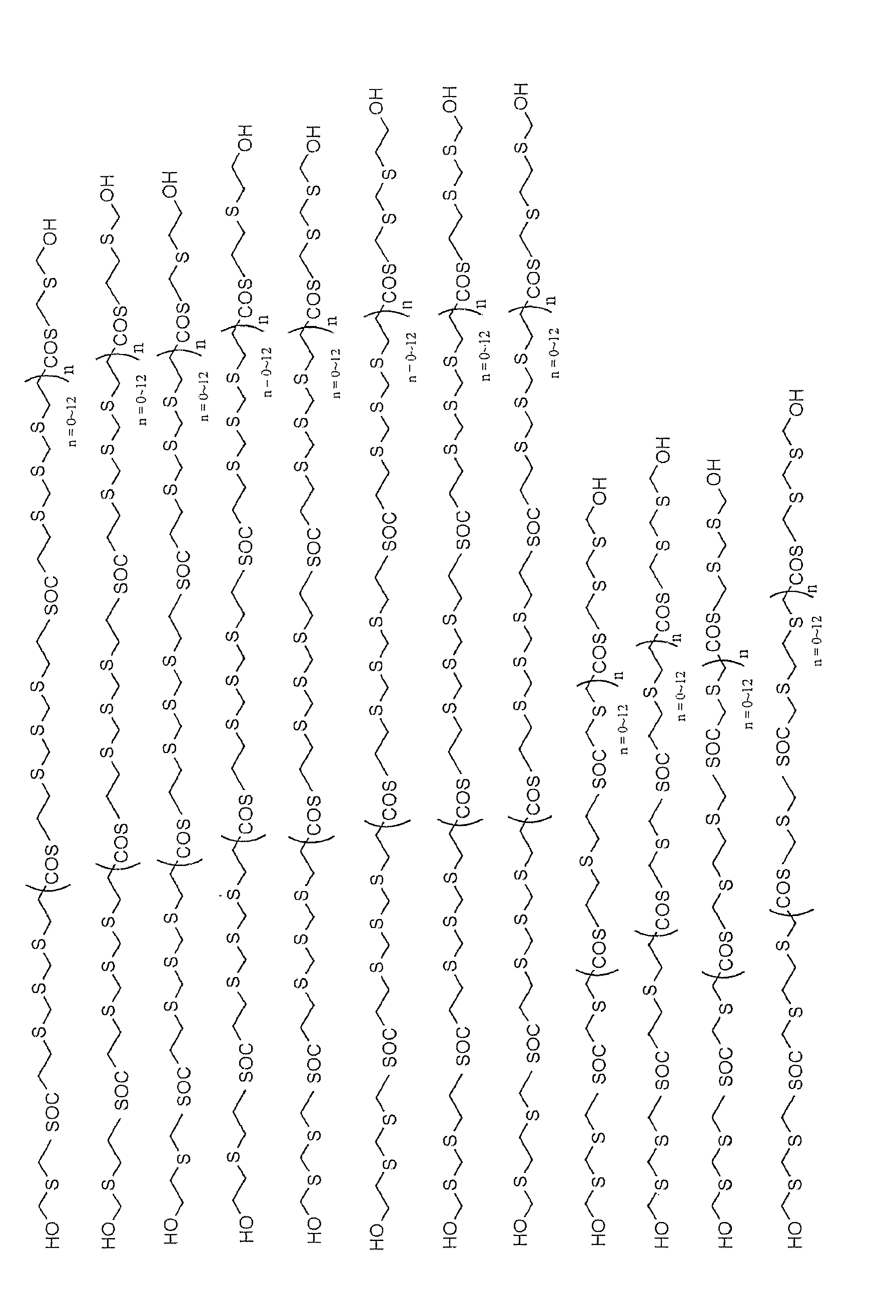

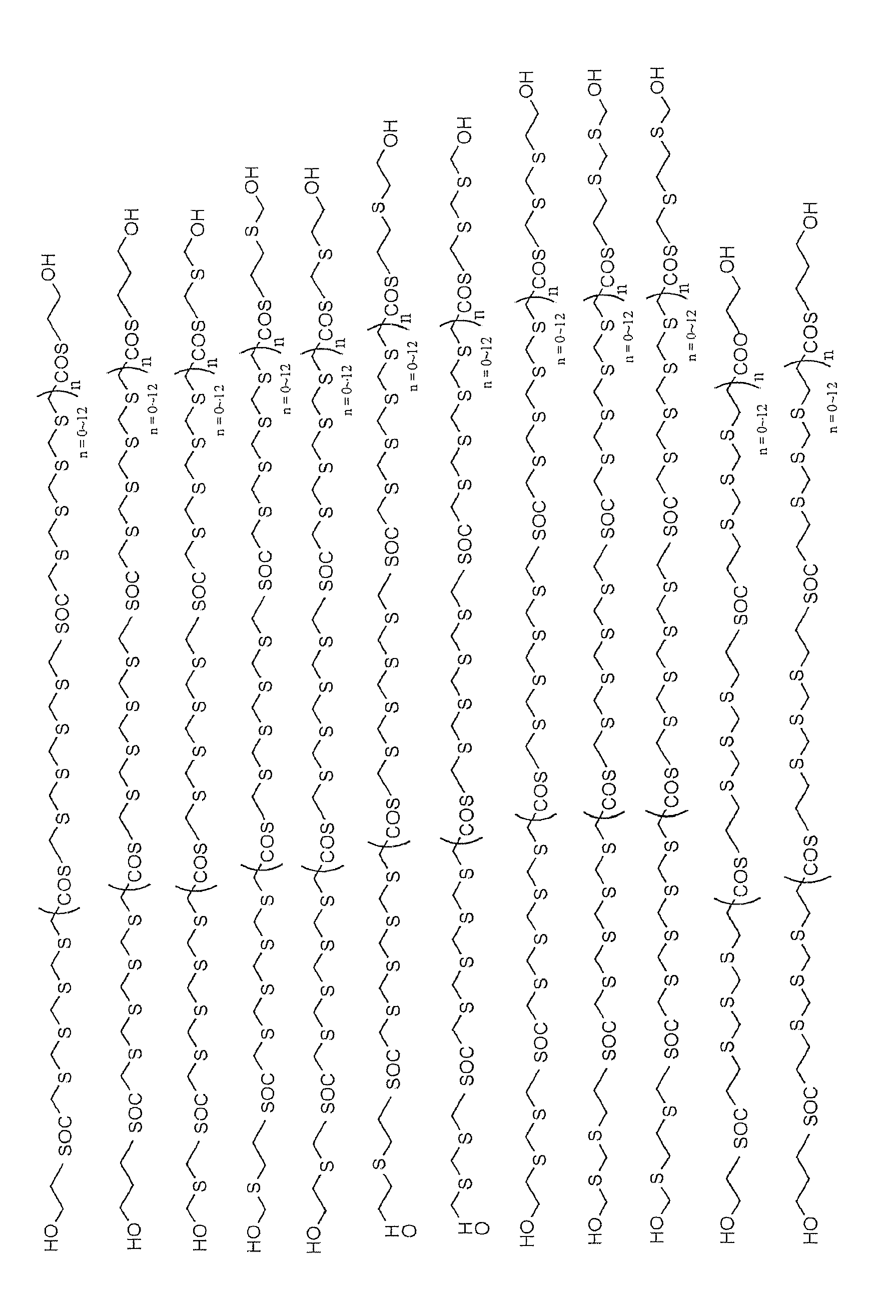

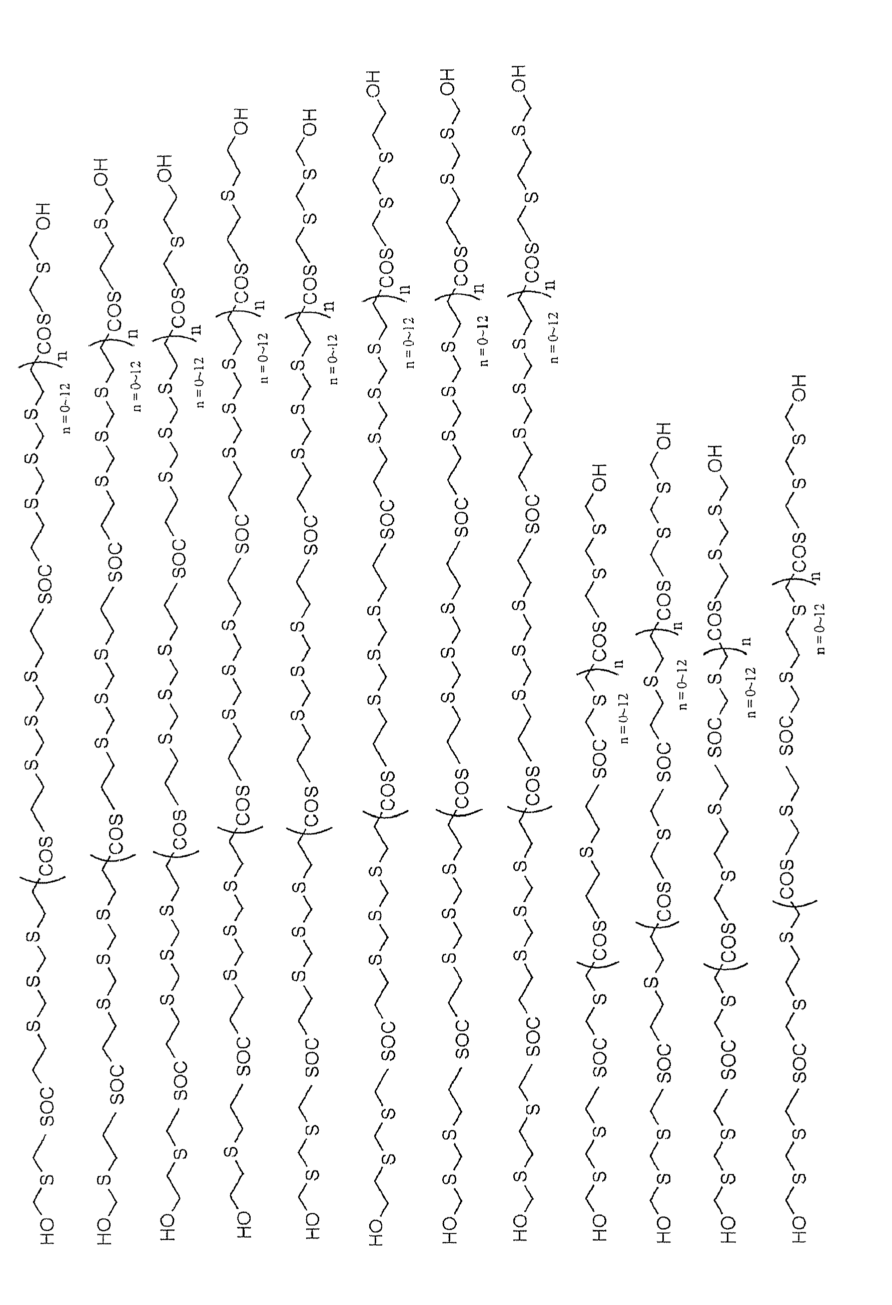

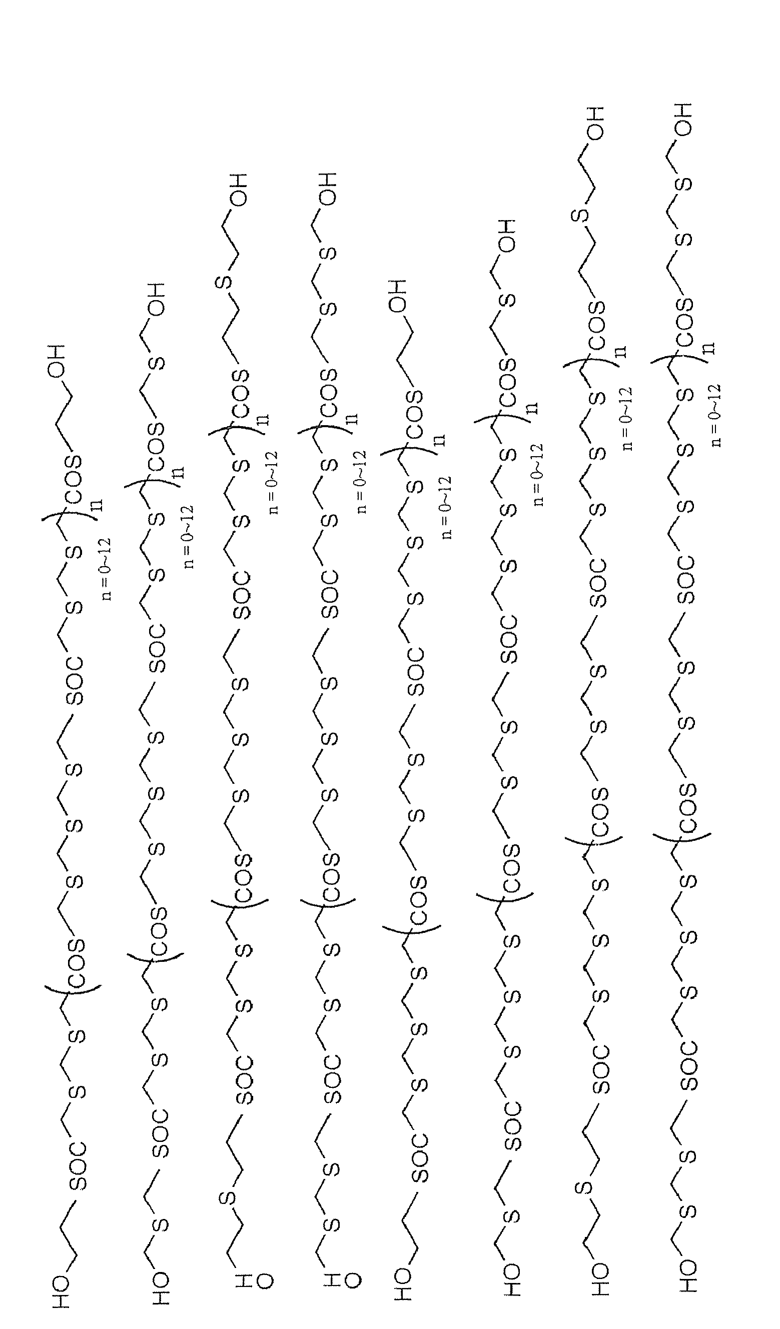

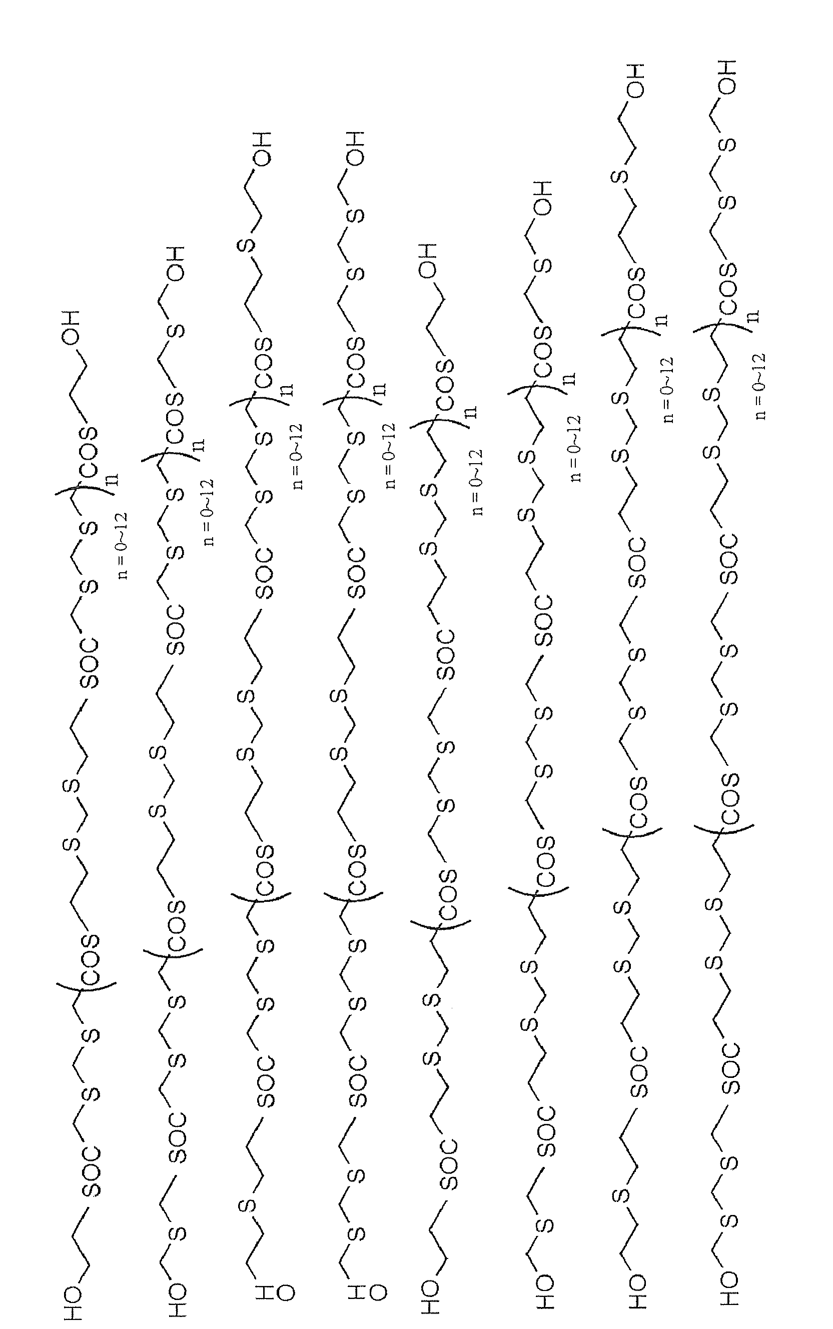

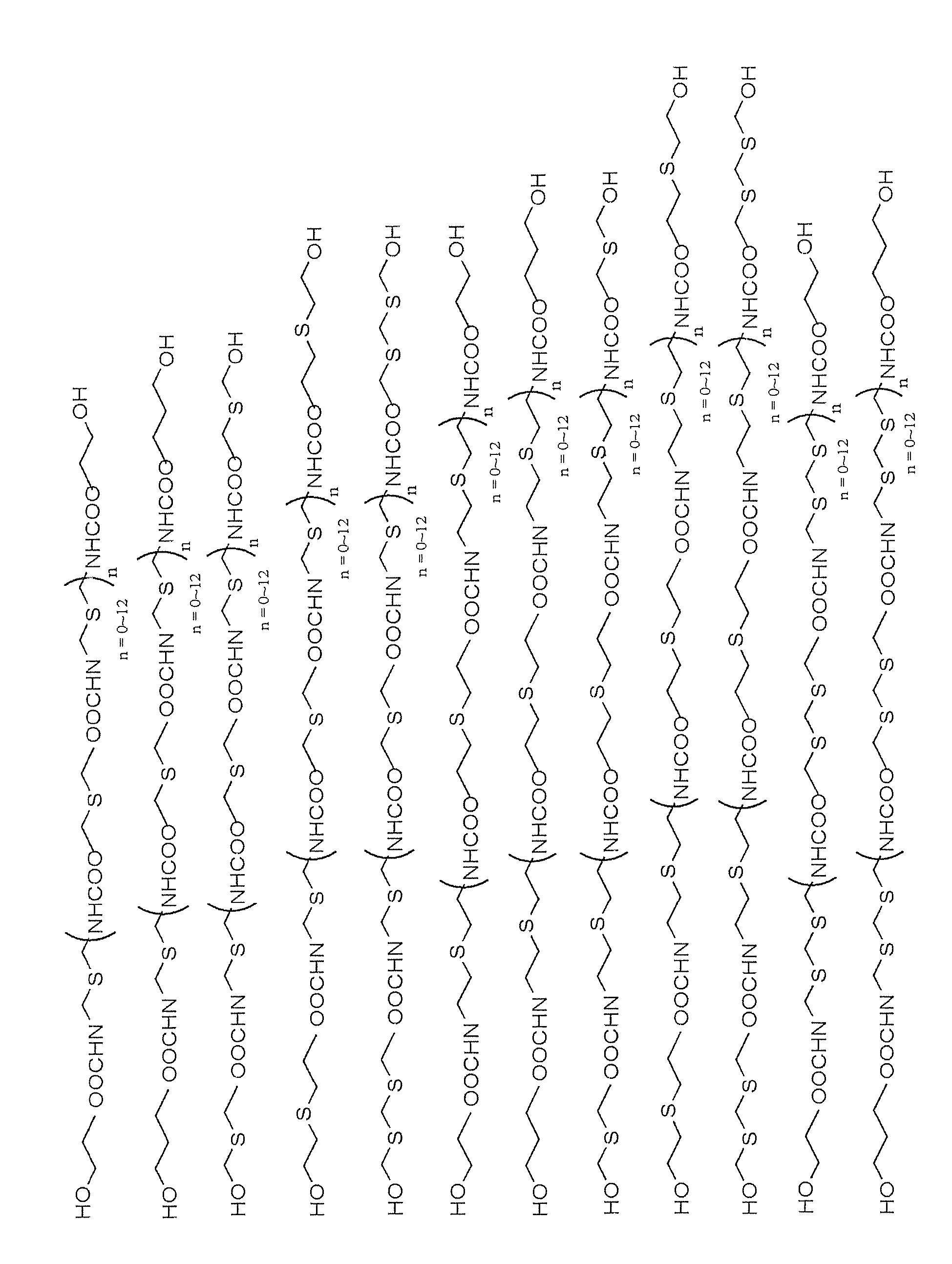

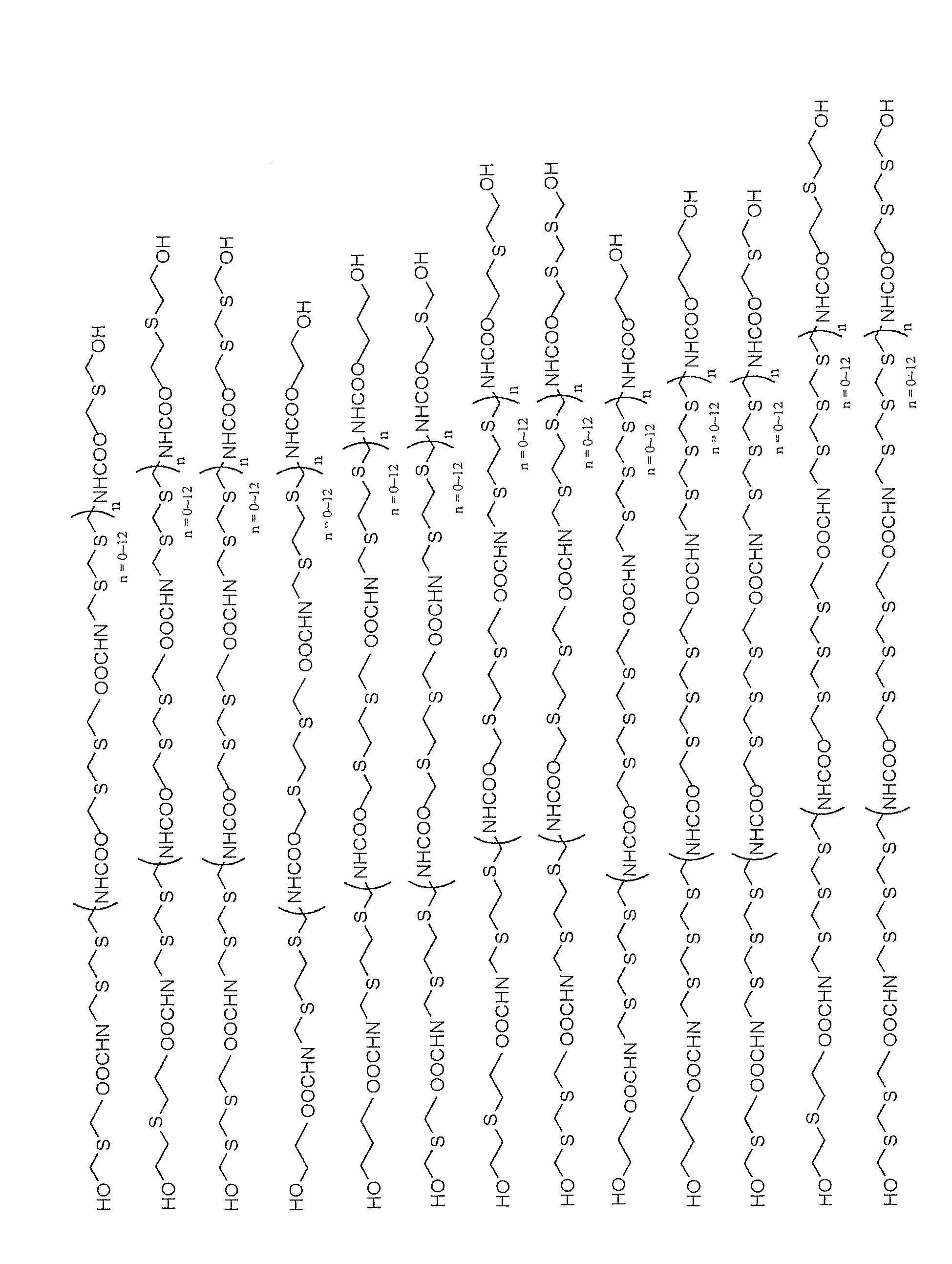

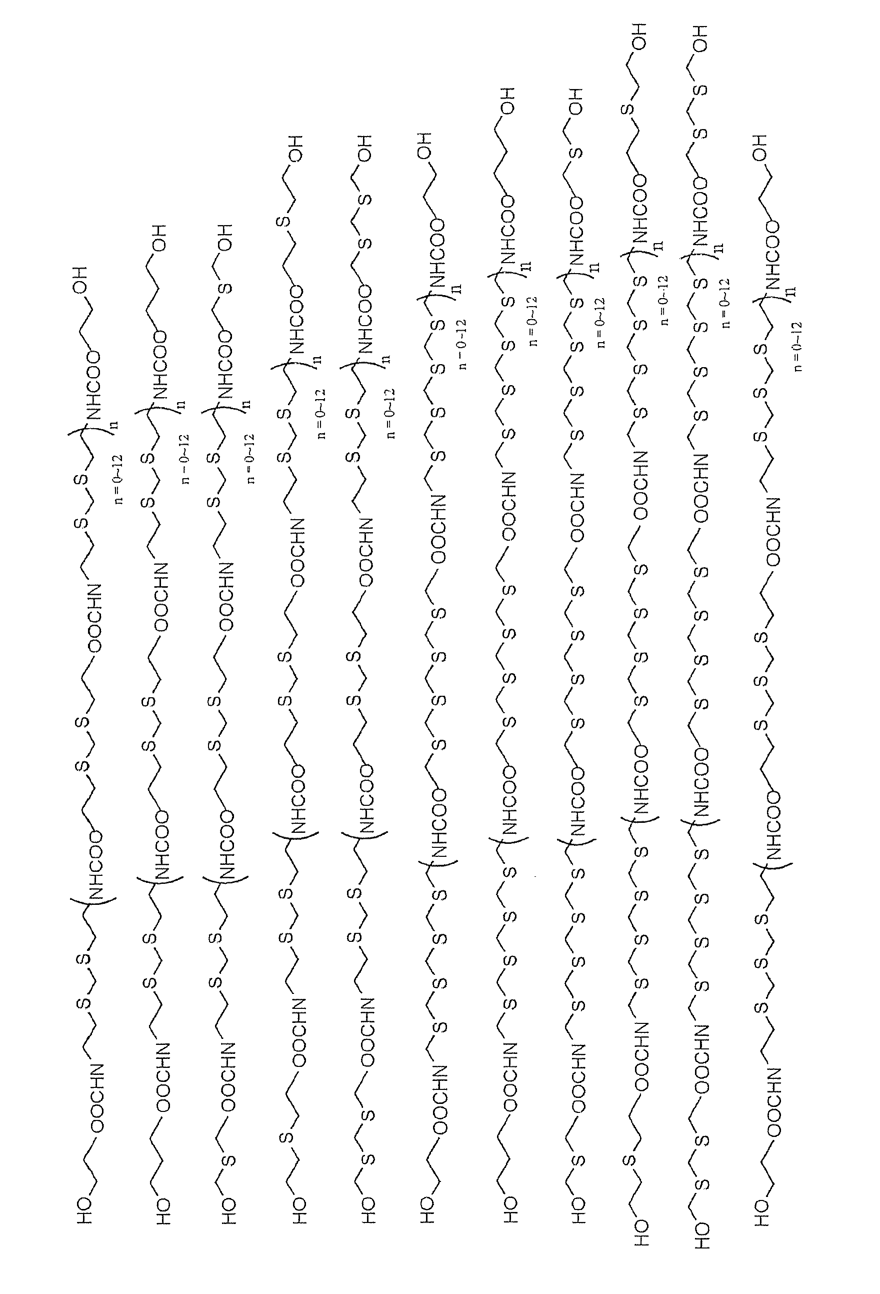

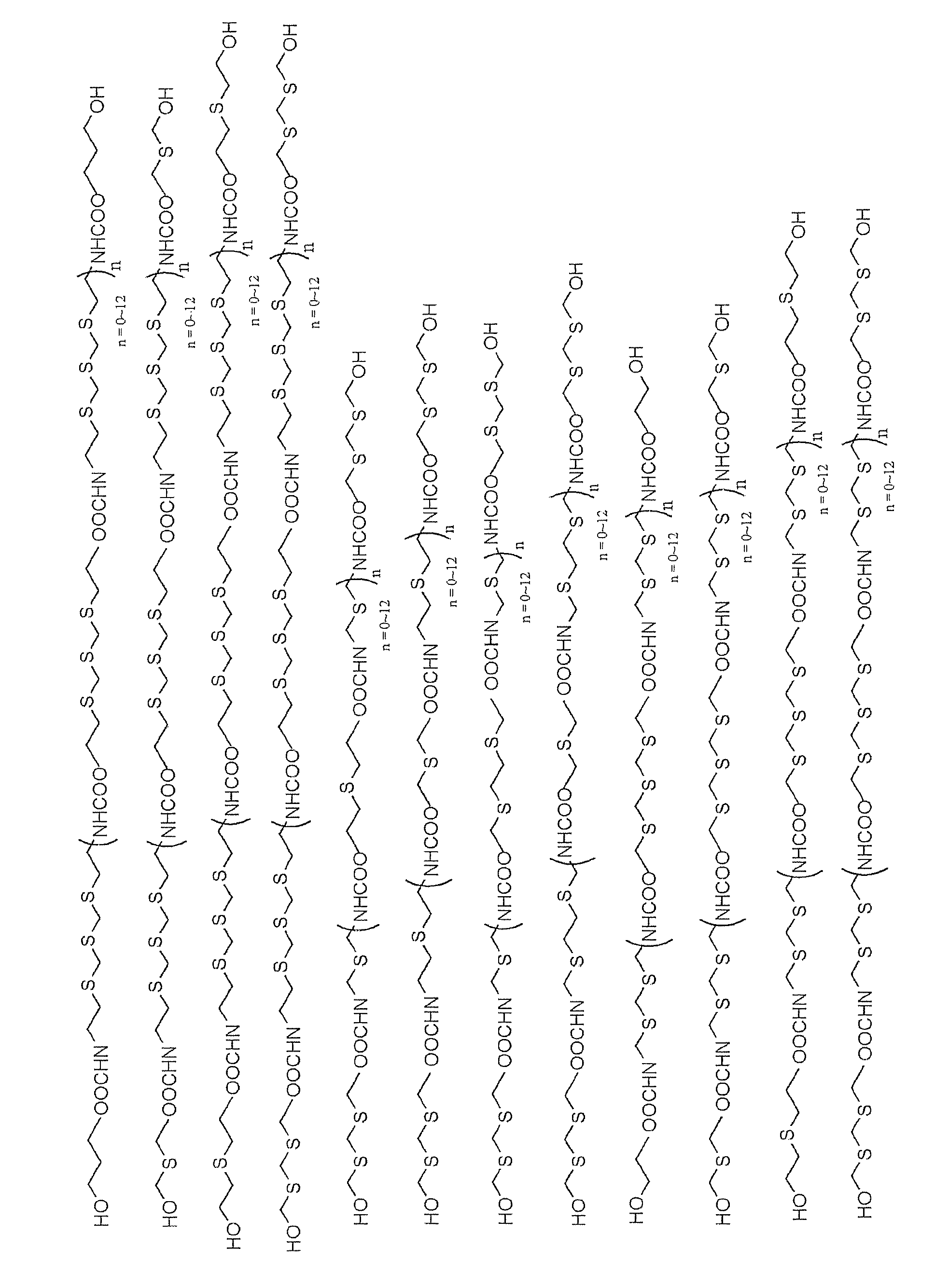

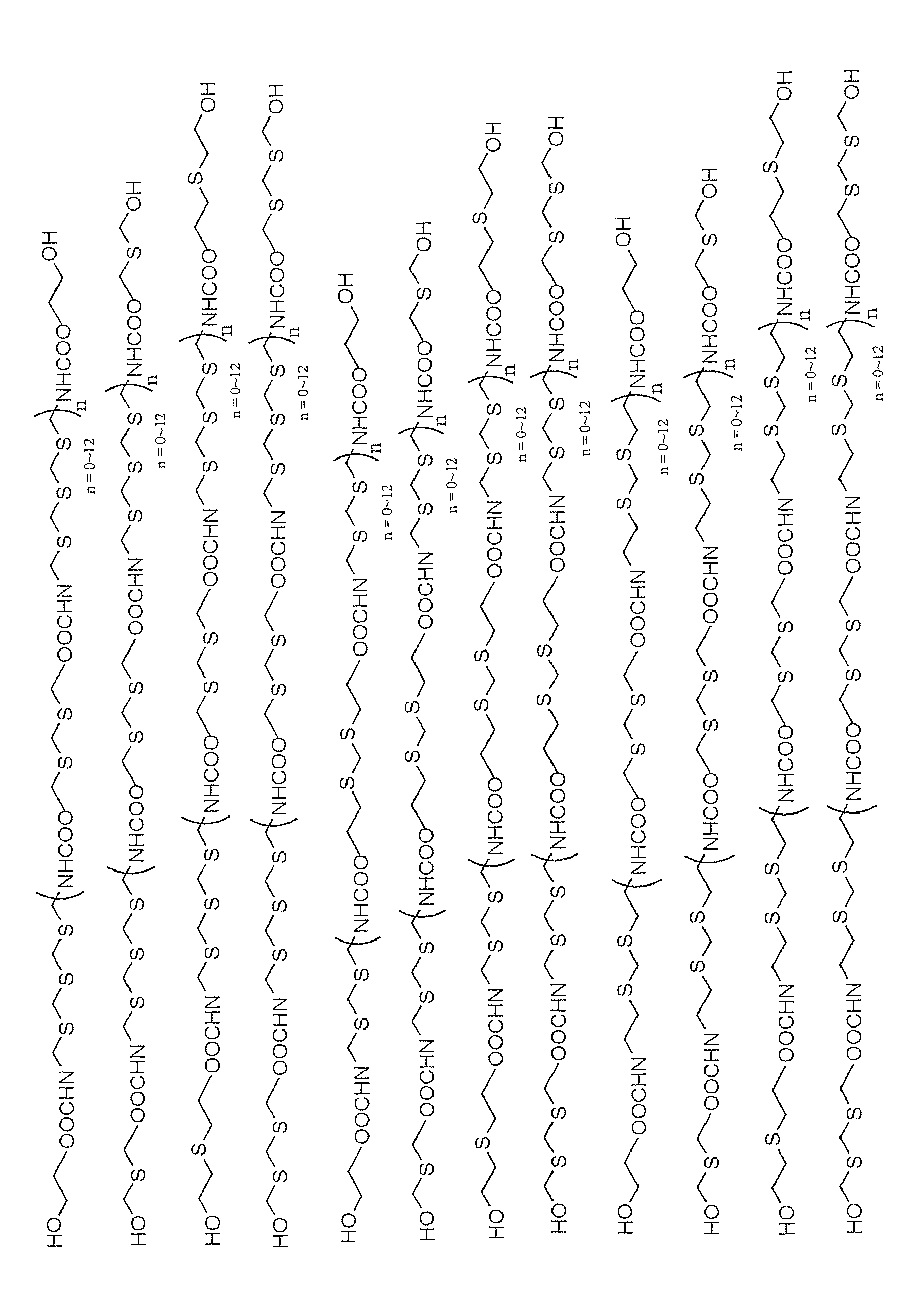

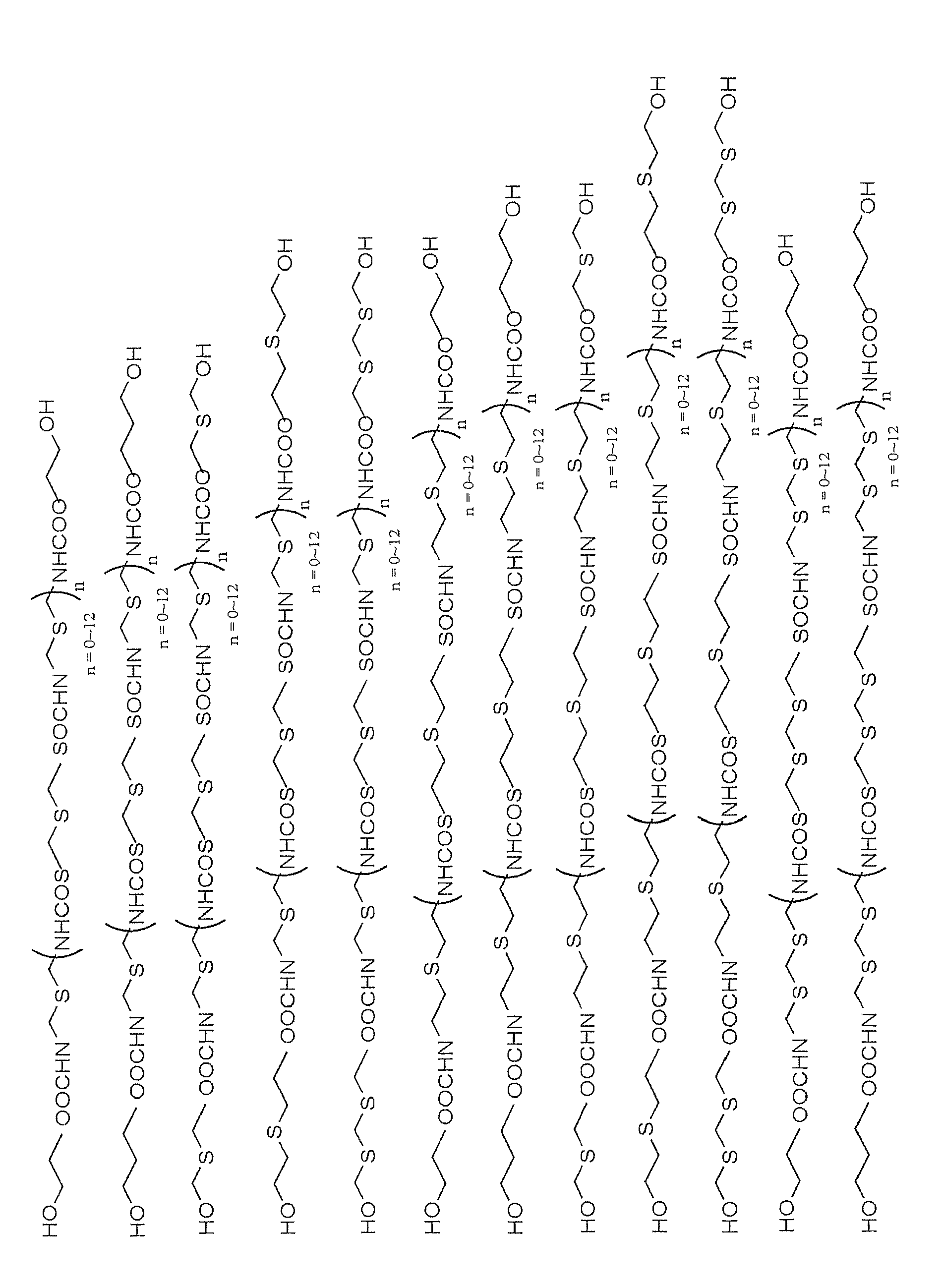

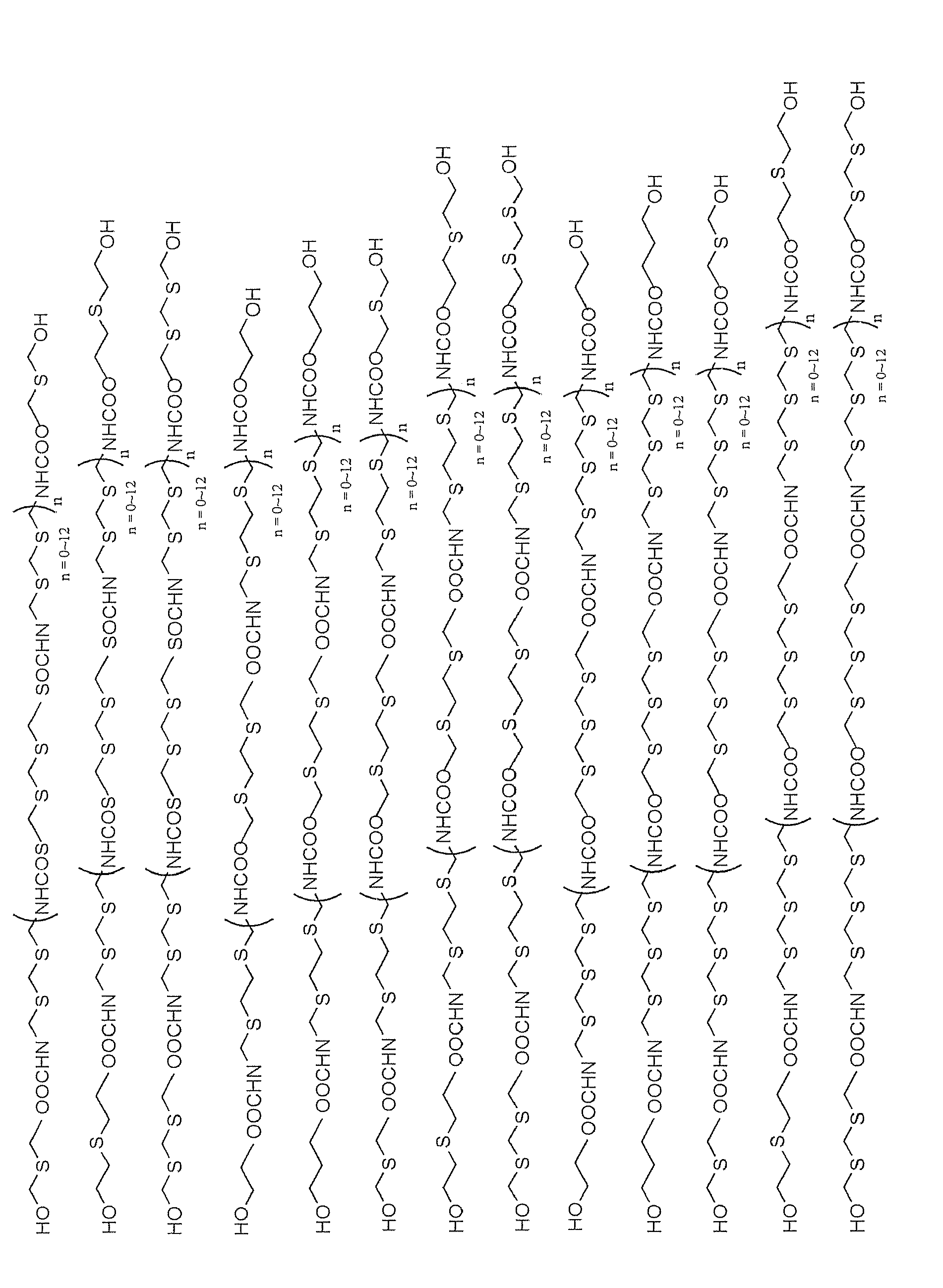

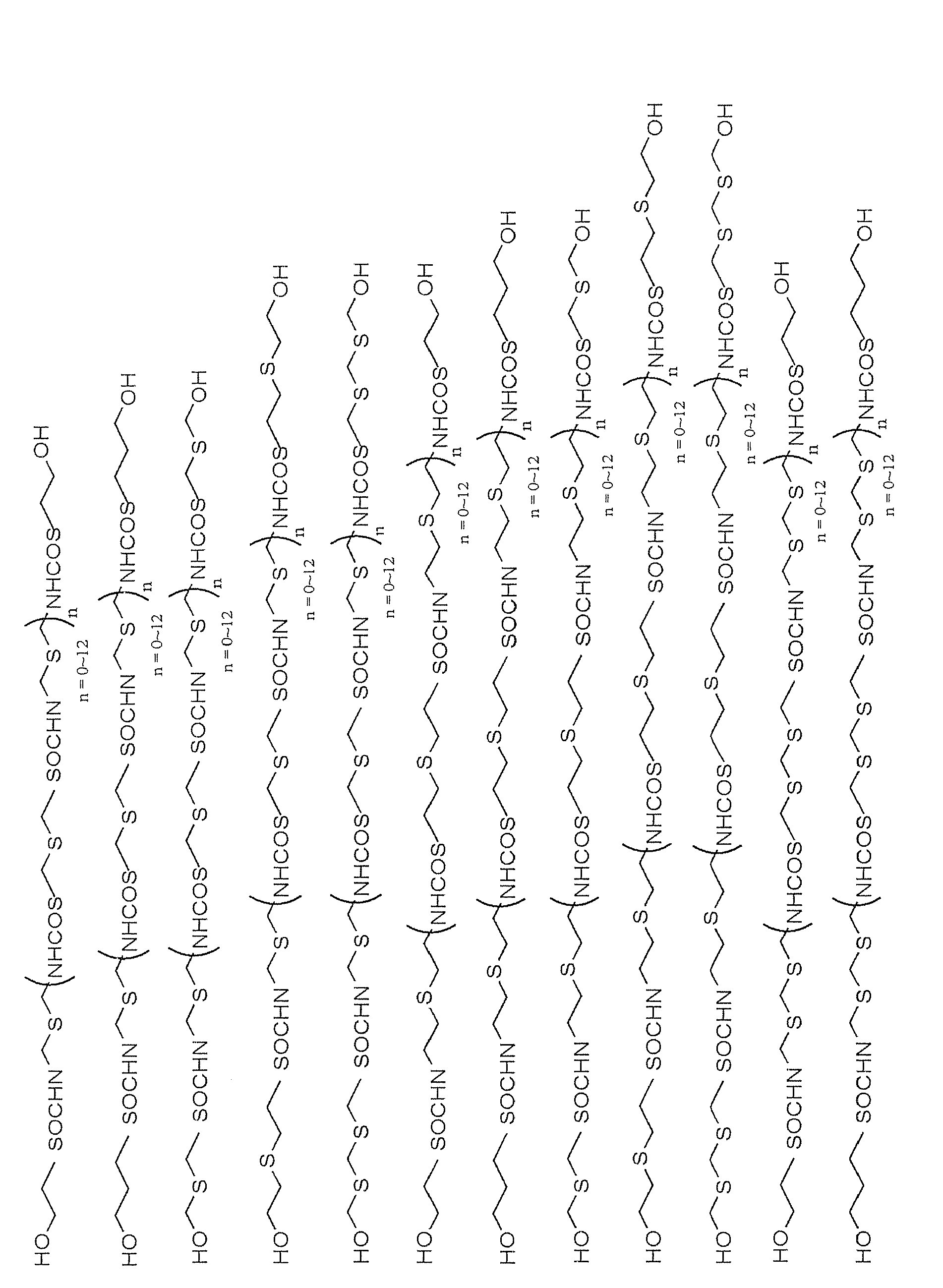

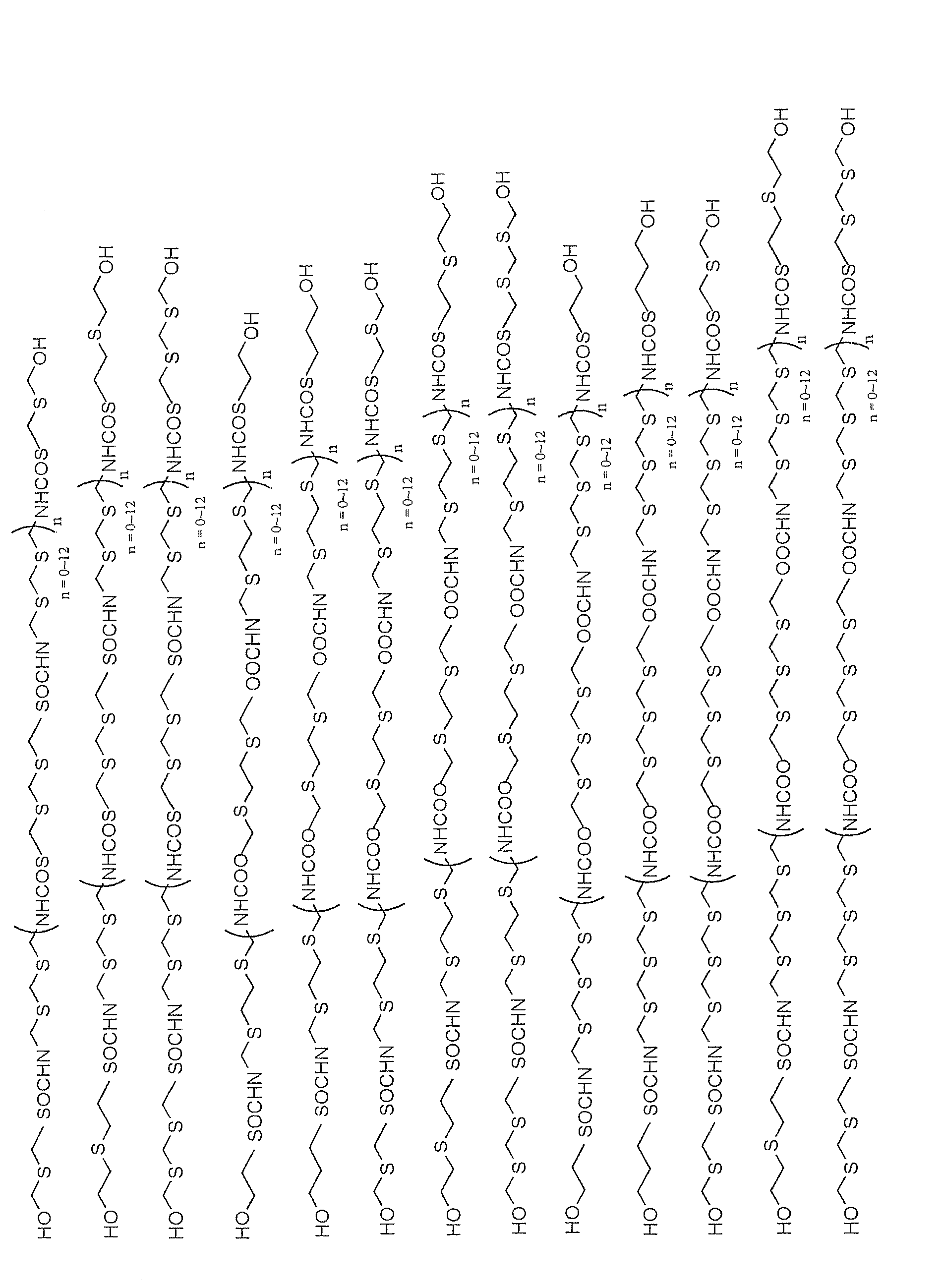

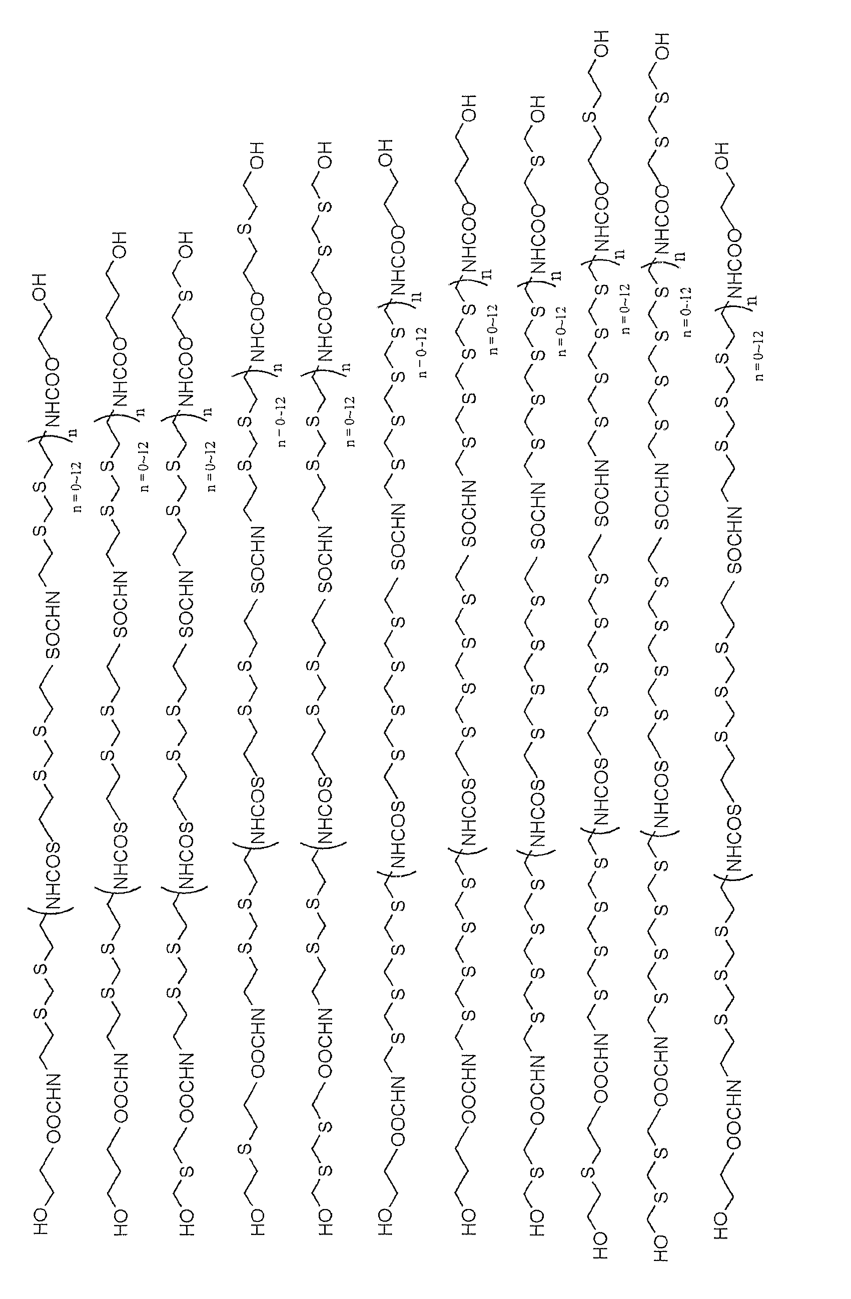

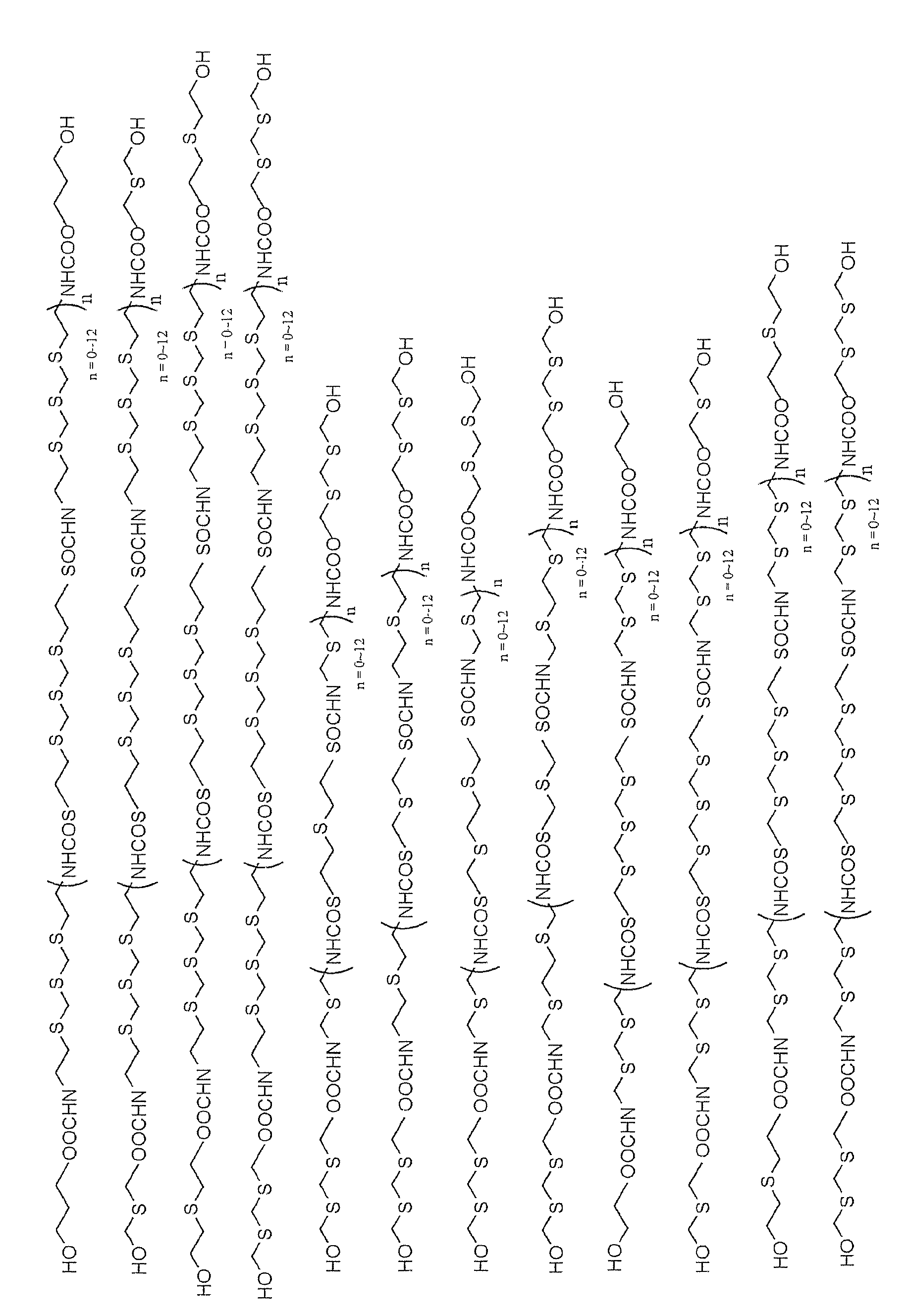

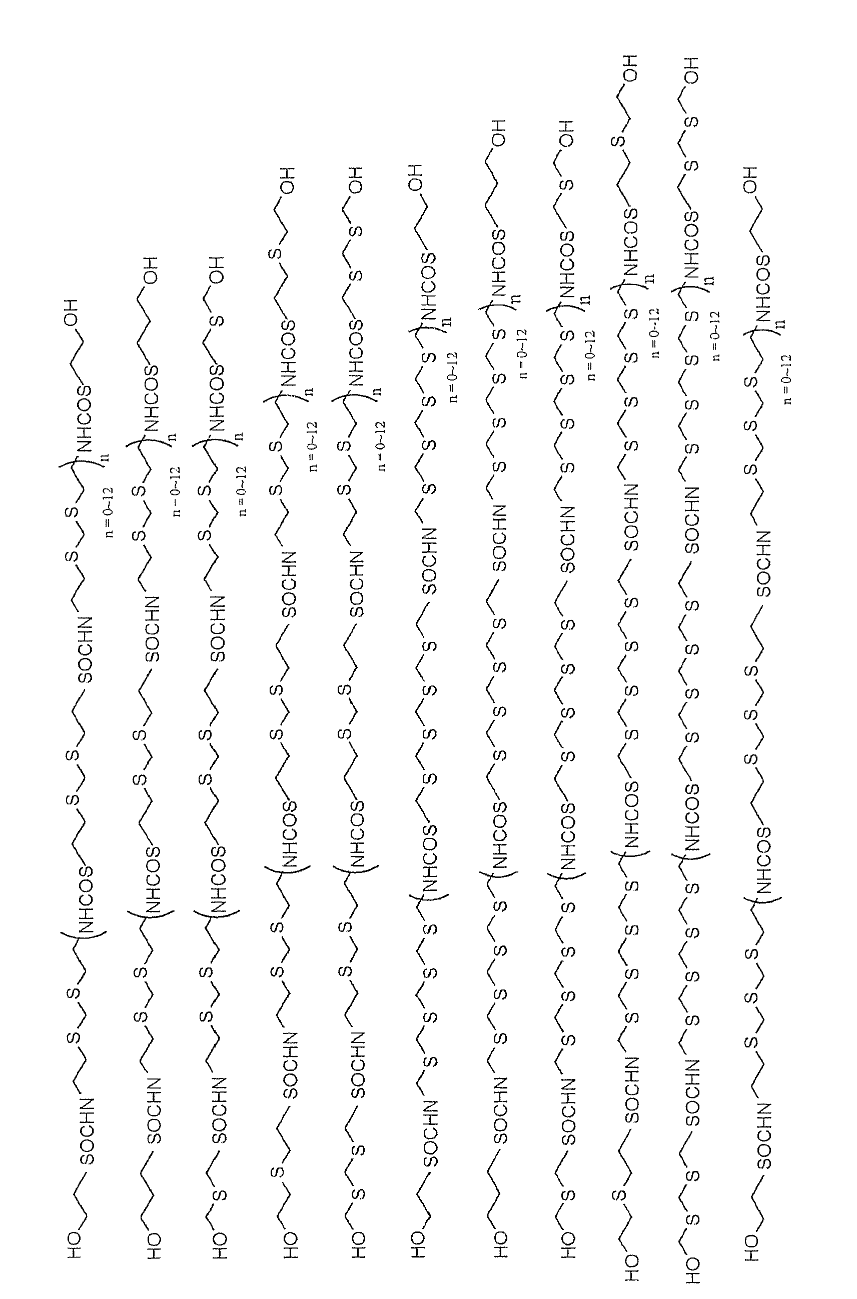

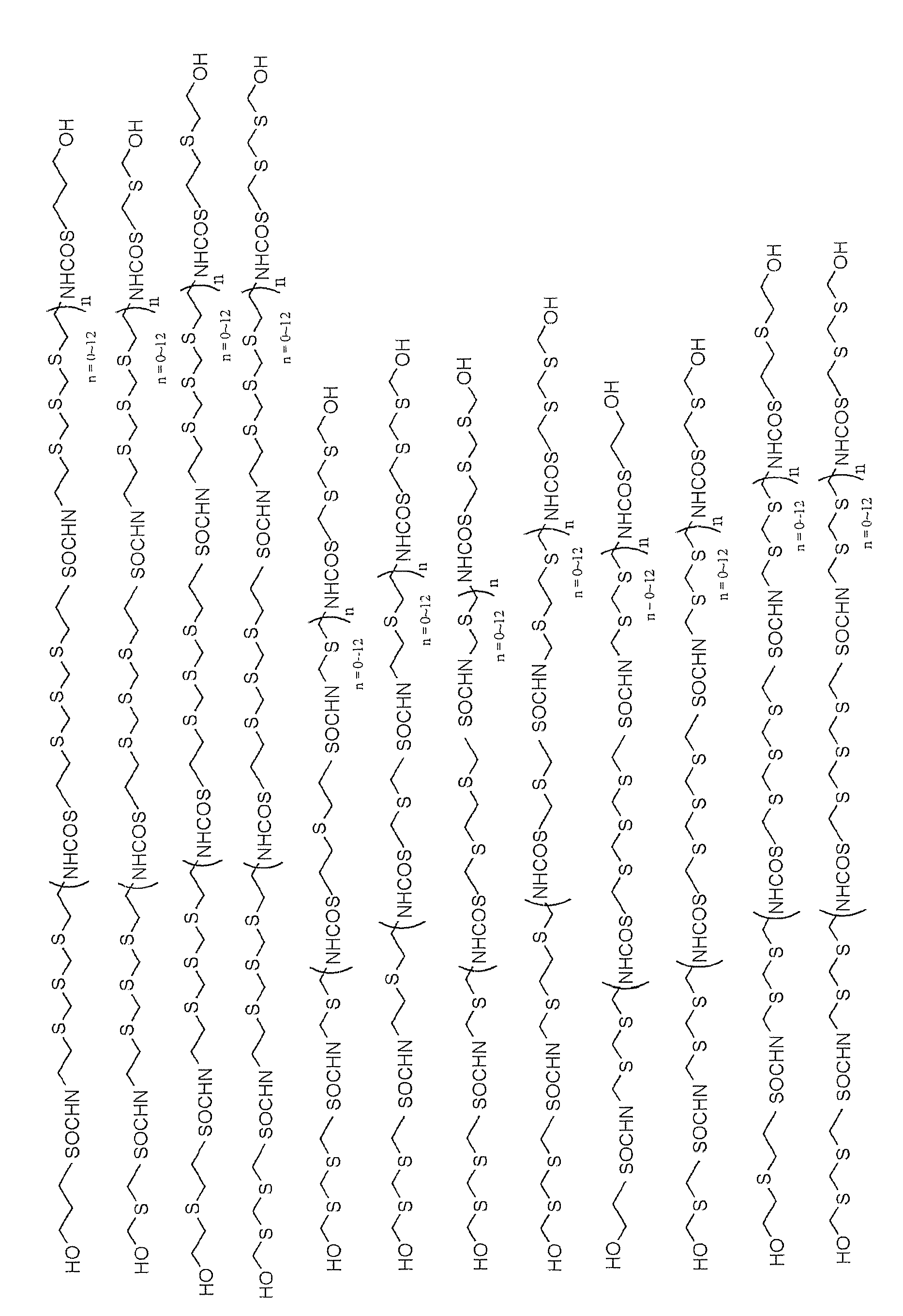

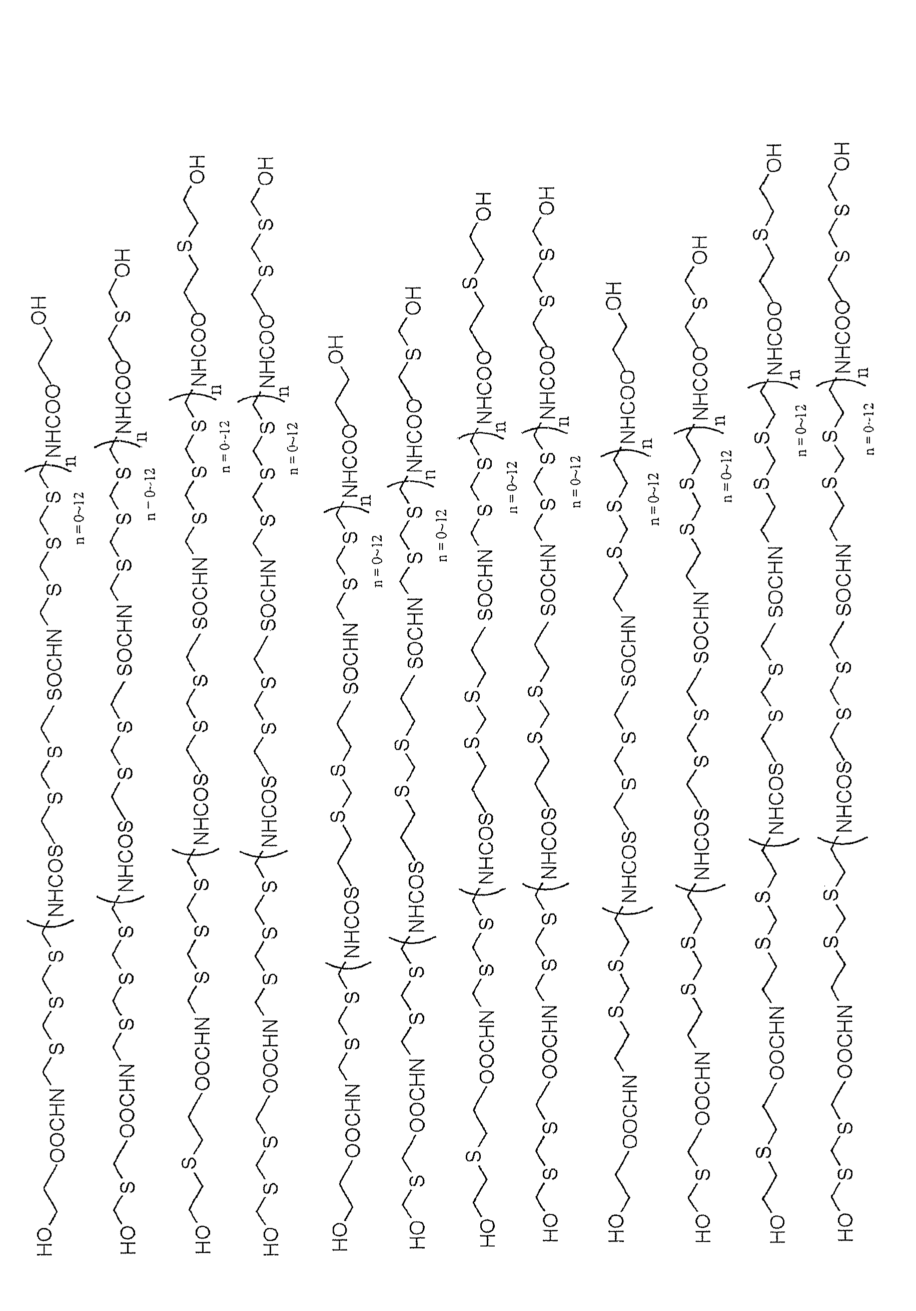

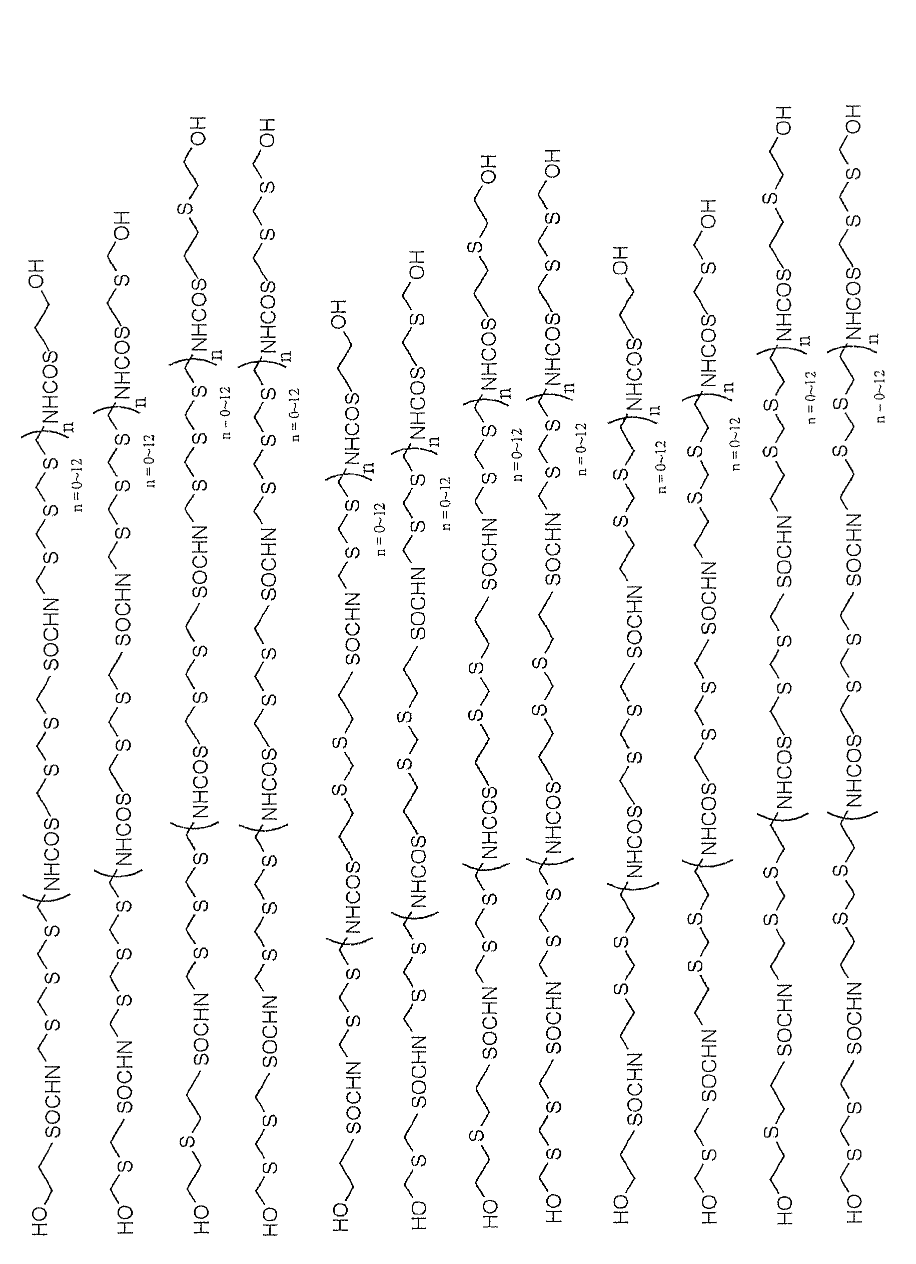

From the perspective of imparting excellent optical characteristics and good mechanical characteristics in a balanced manner to a transparent molded article obtained by polymerizing the novel polyol compound of the present invention, preferred examples are as follows:

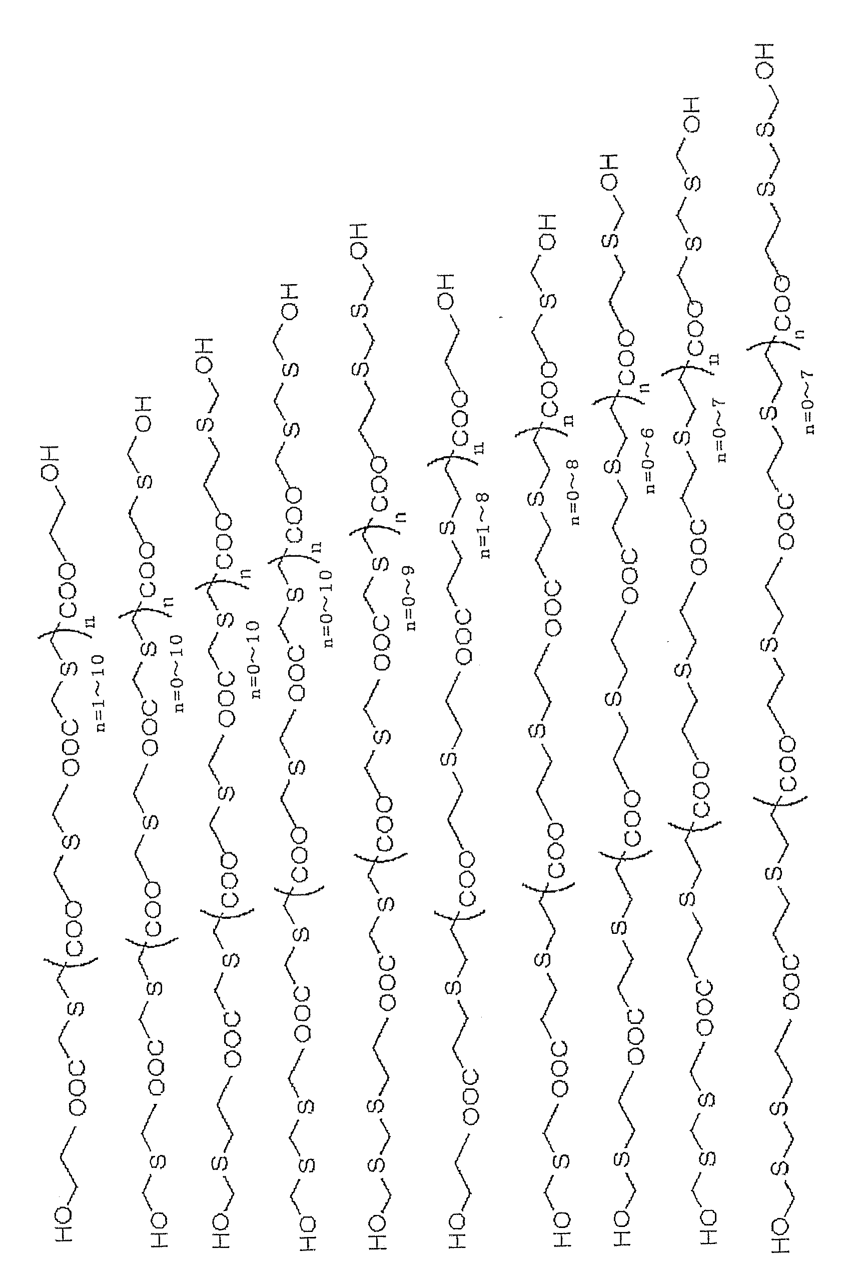

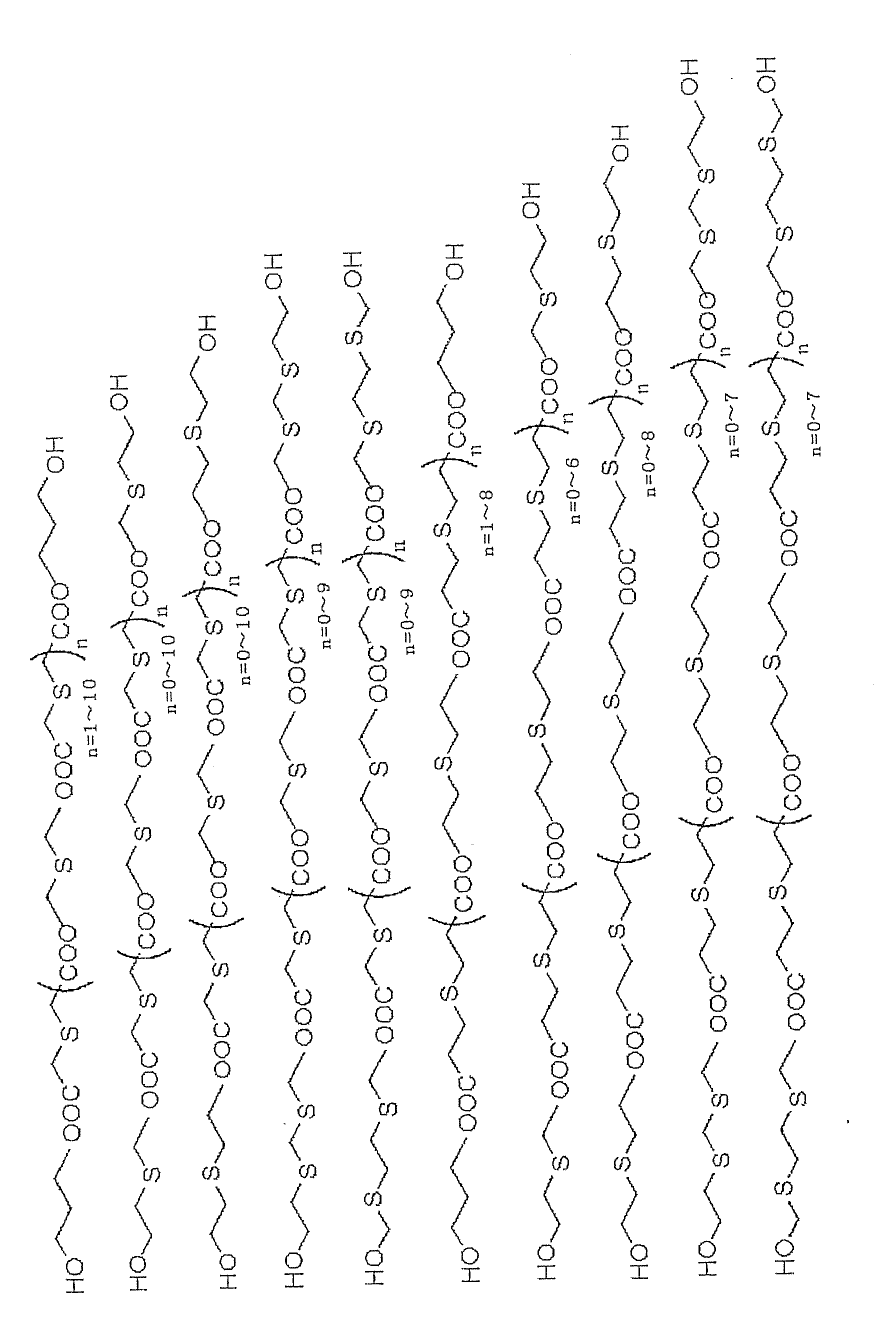

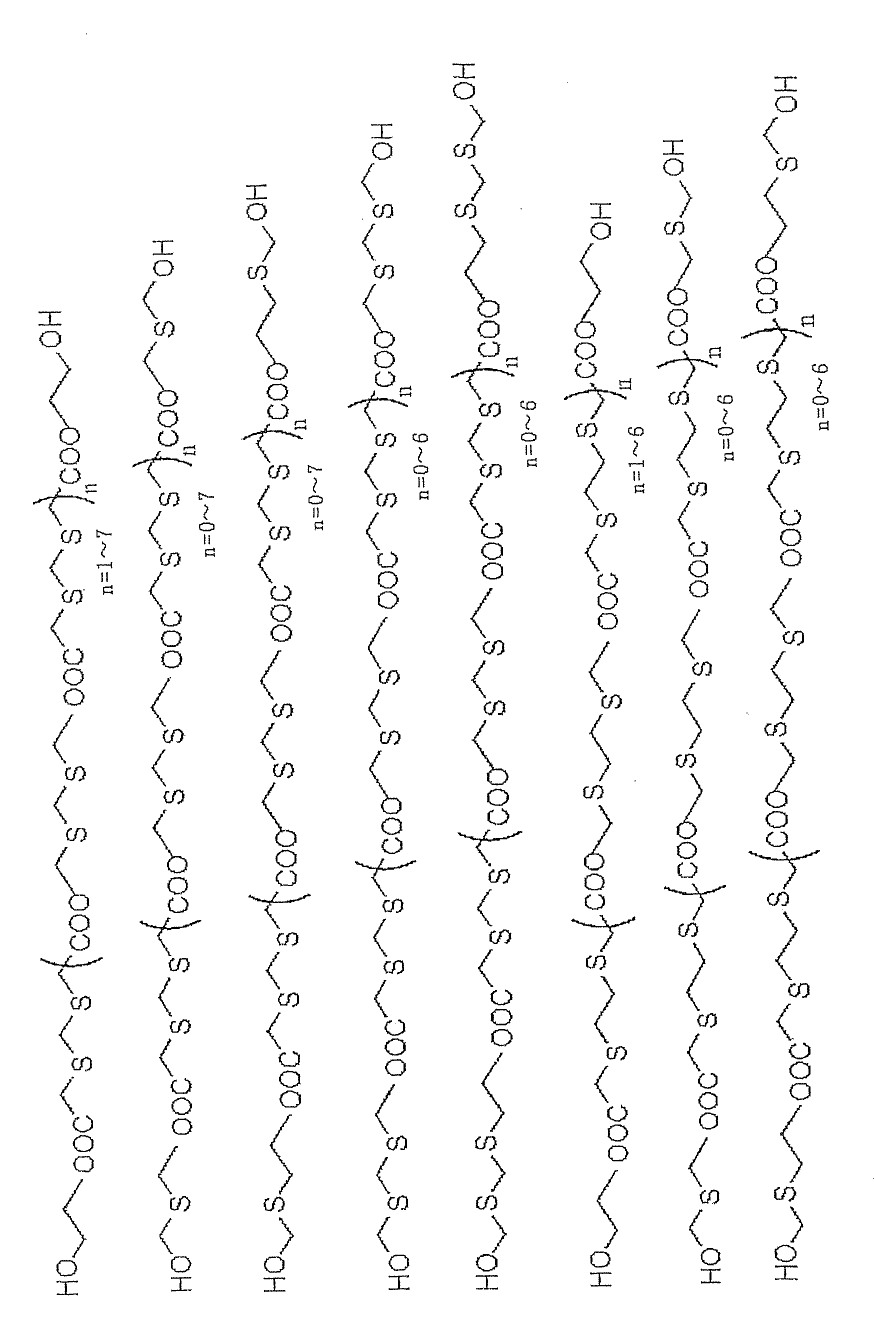

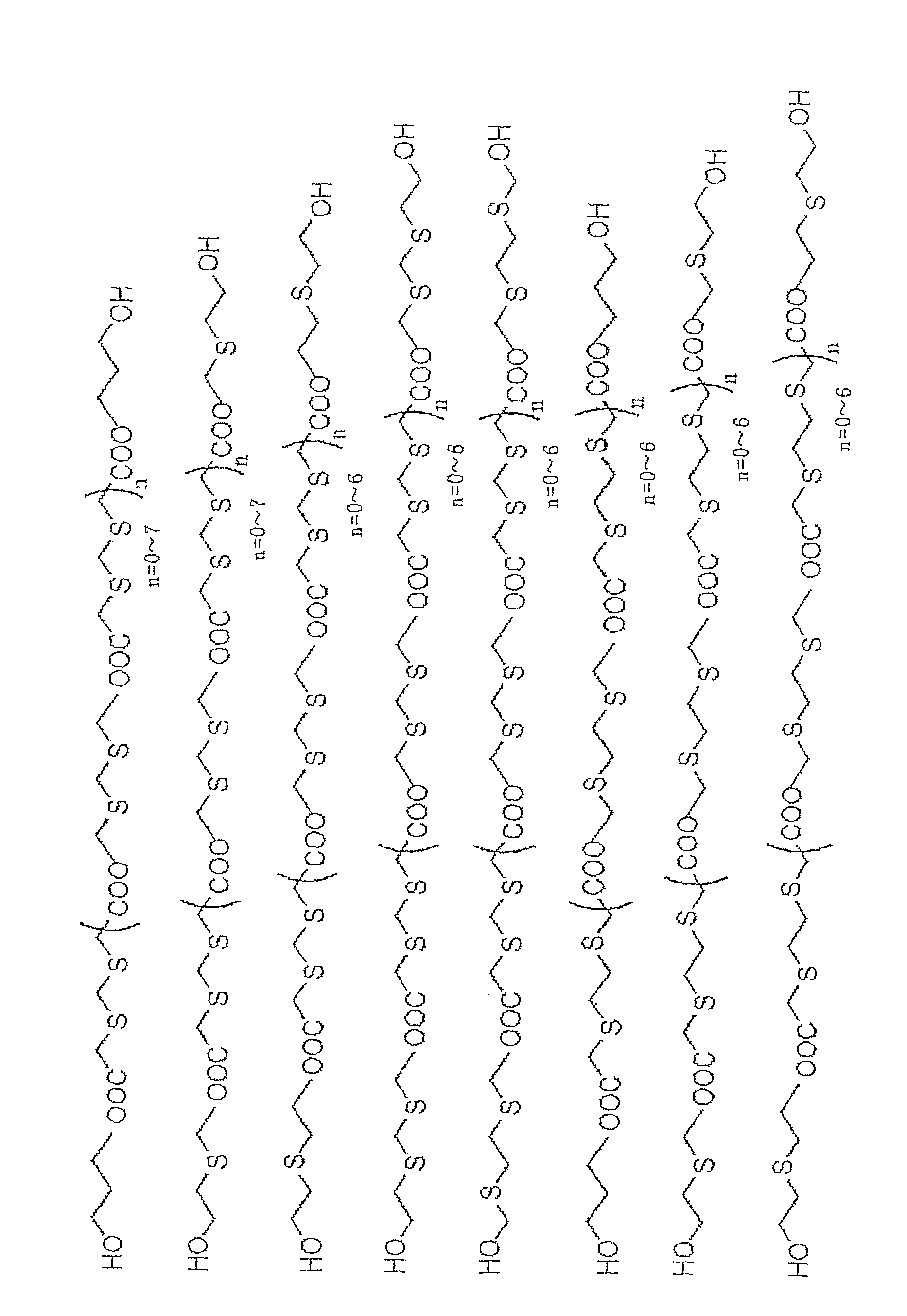

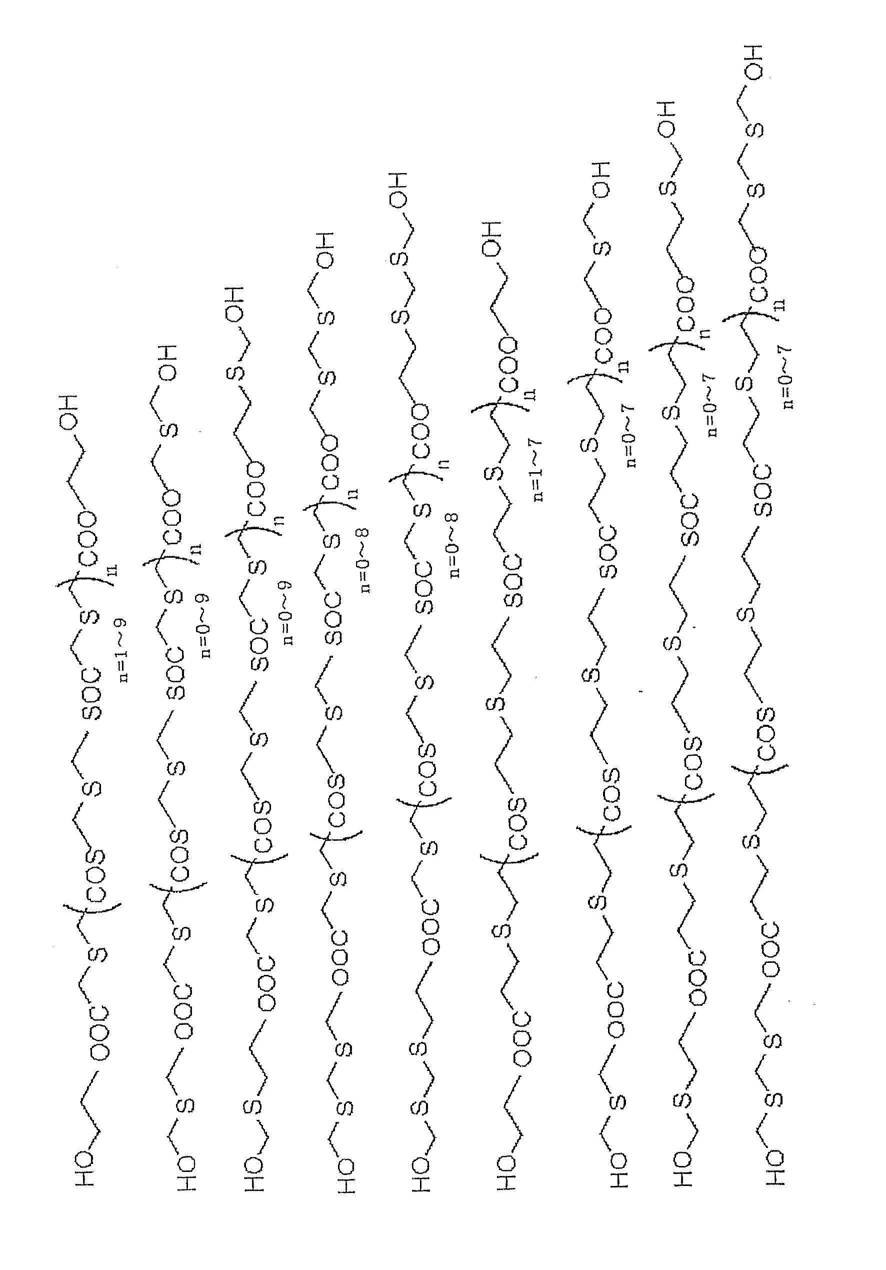

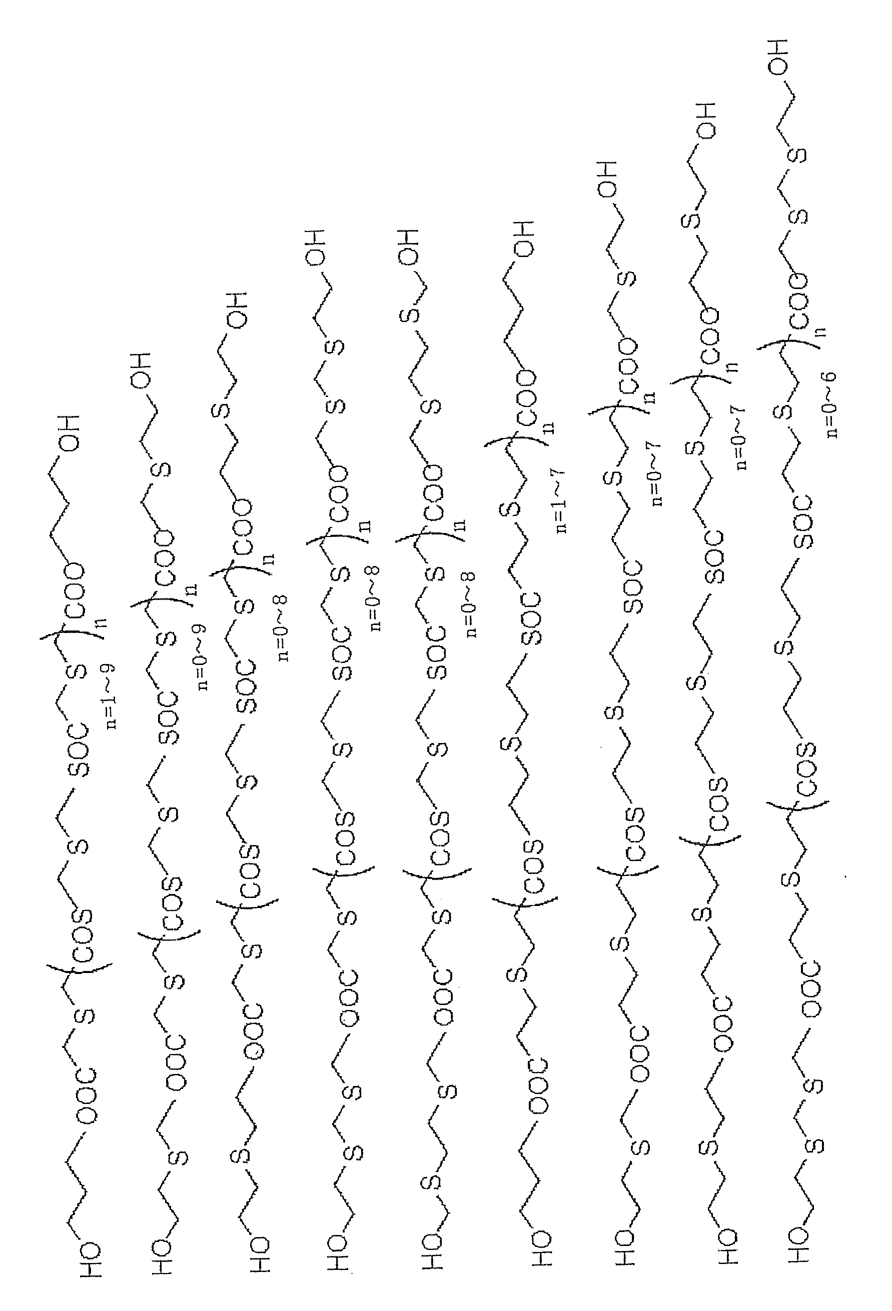

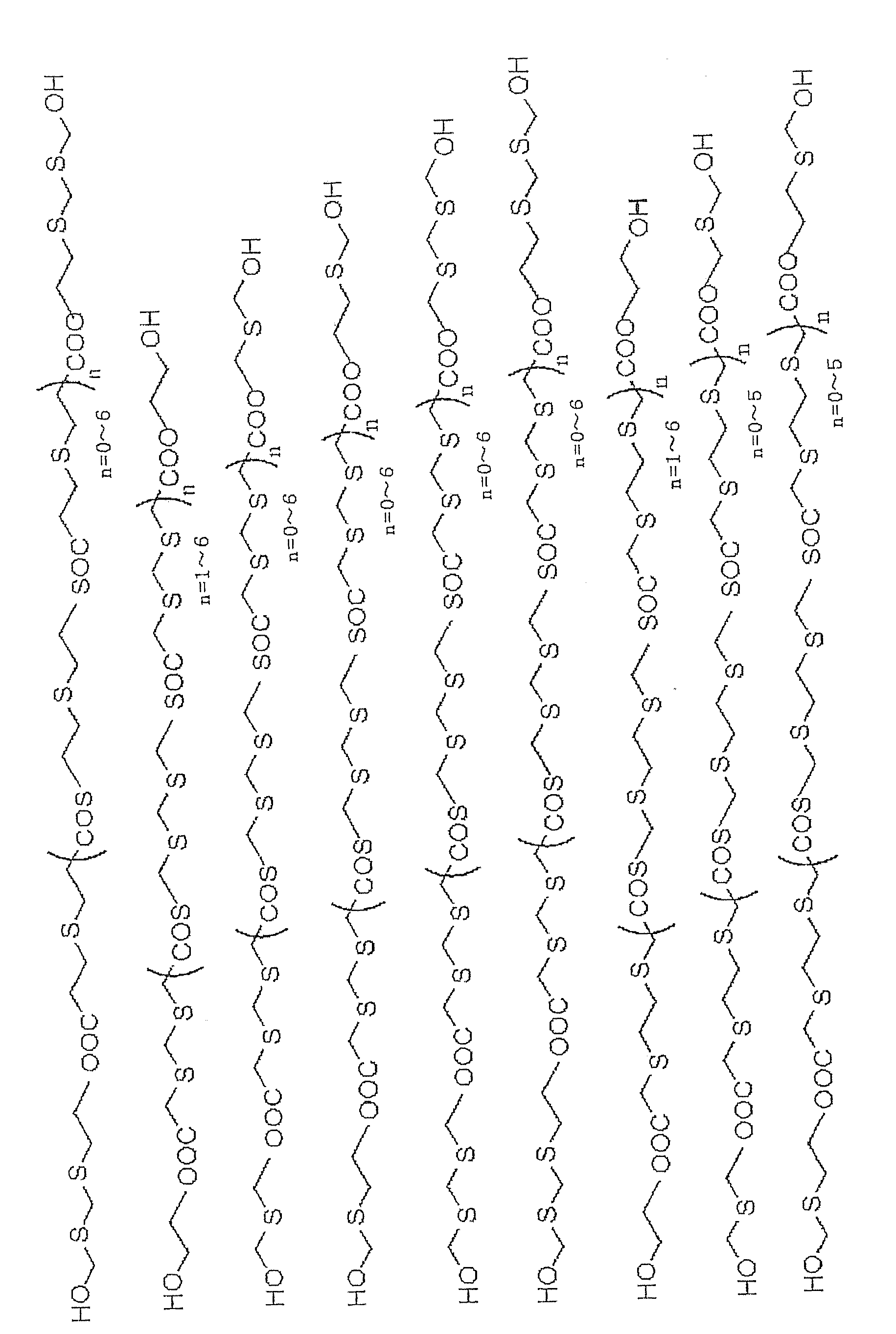

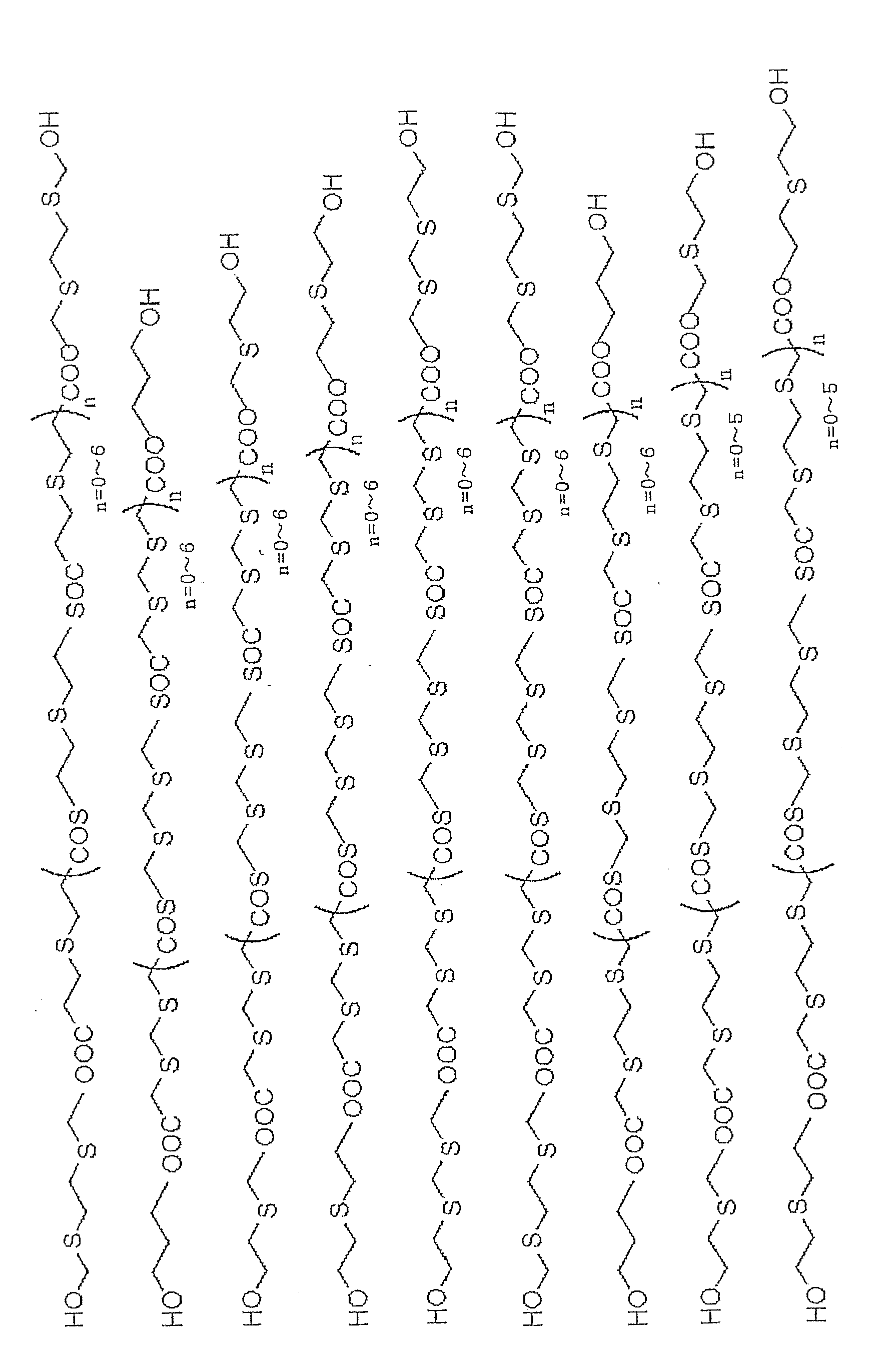

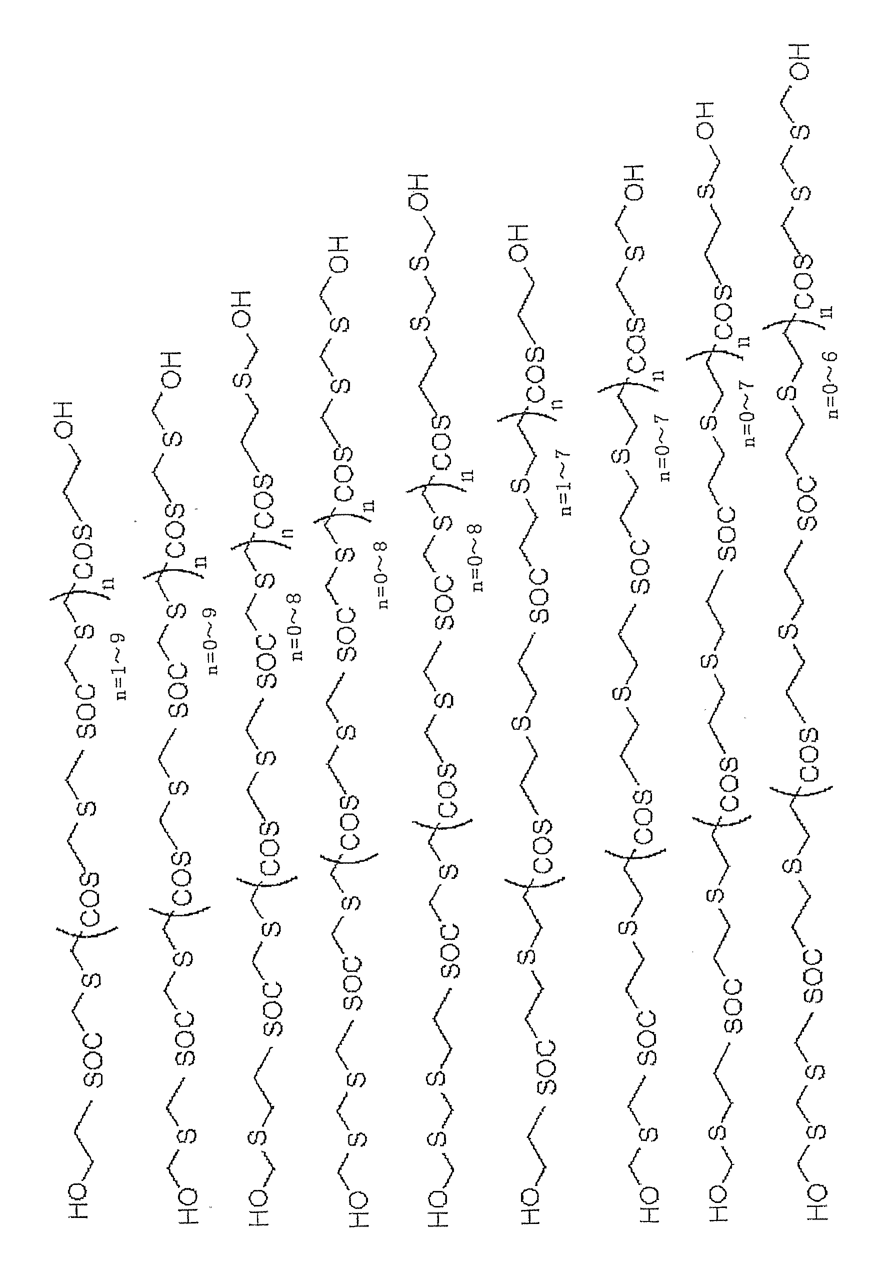

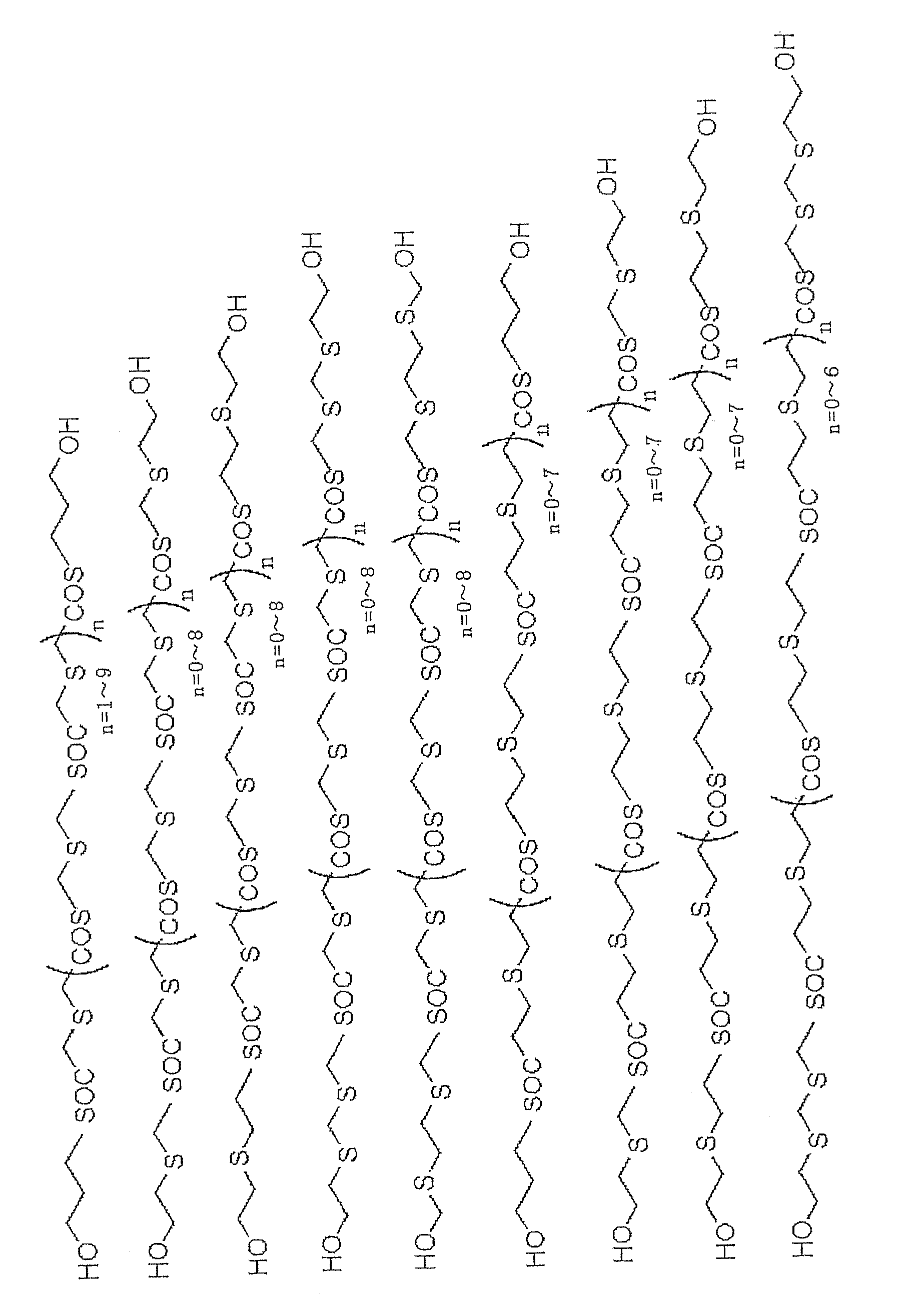

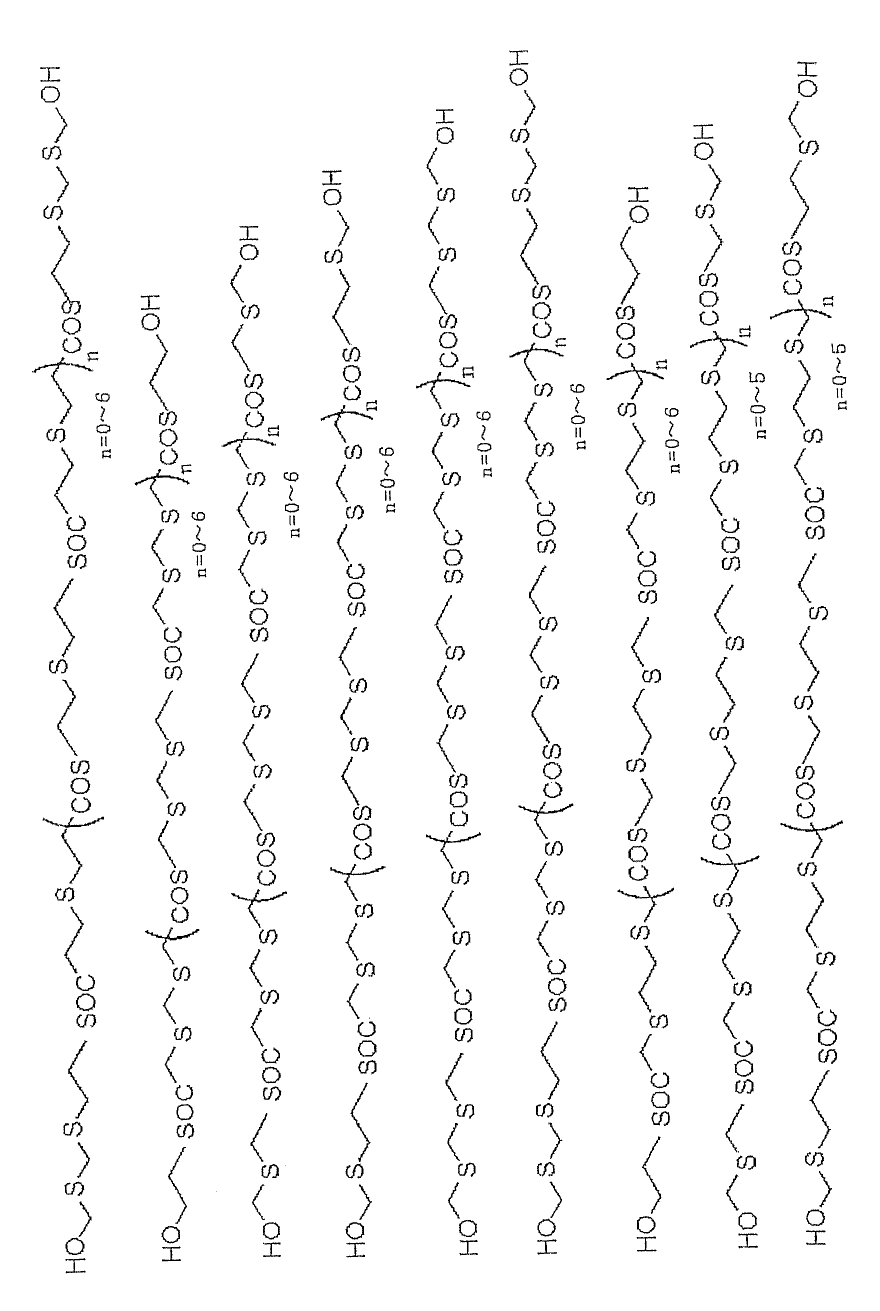

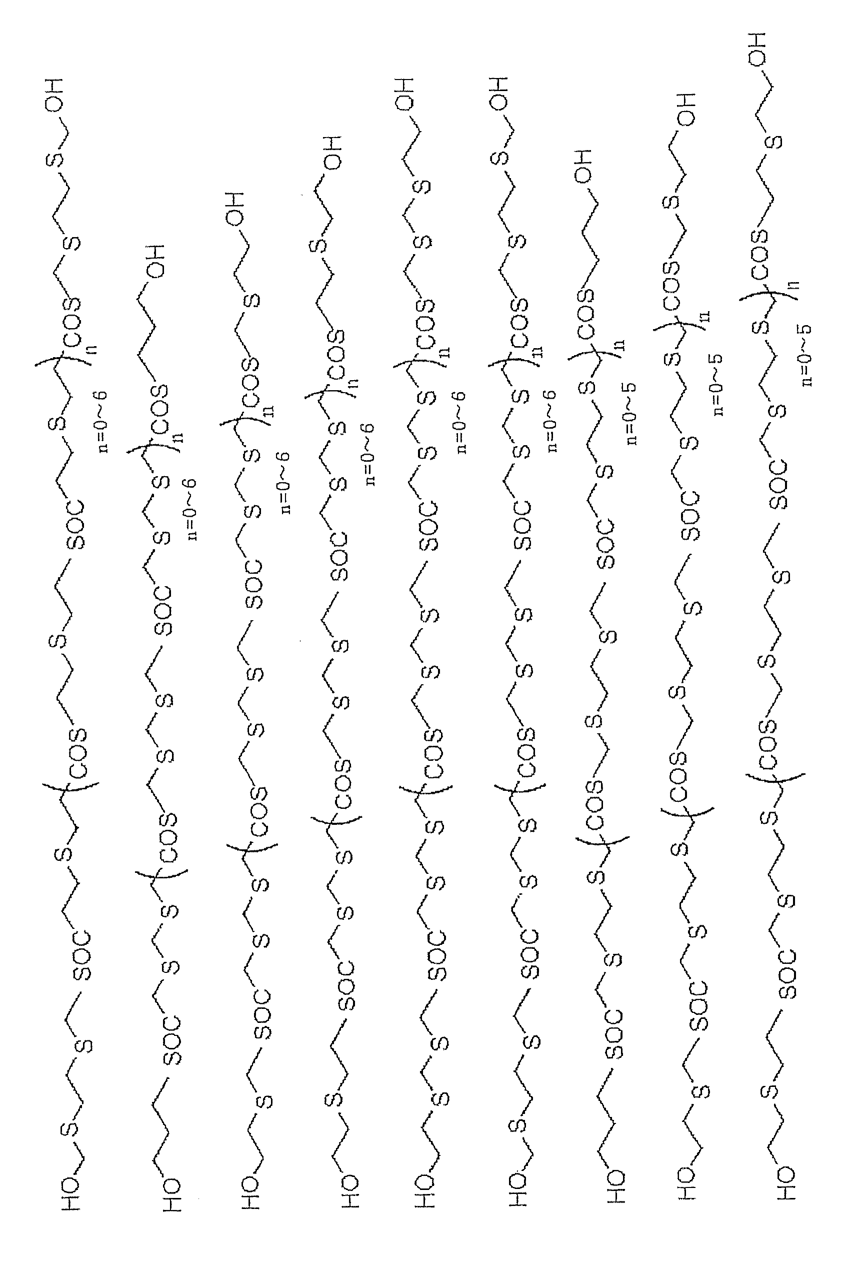

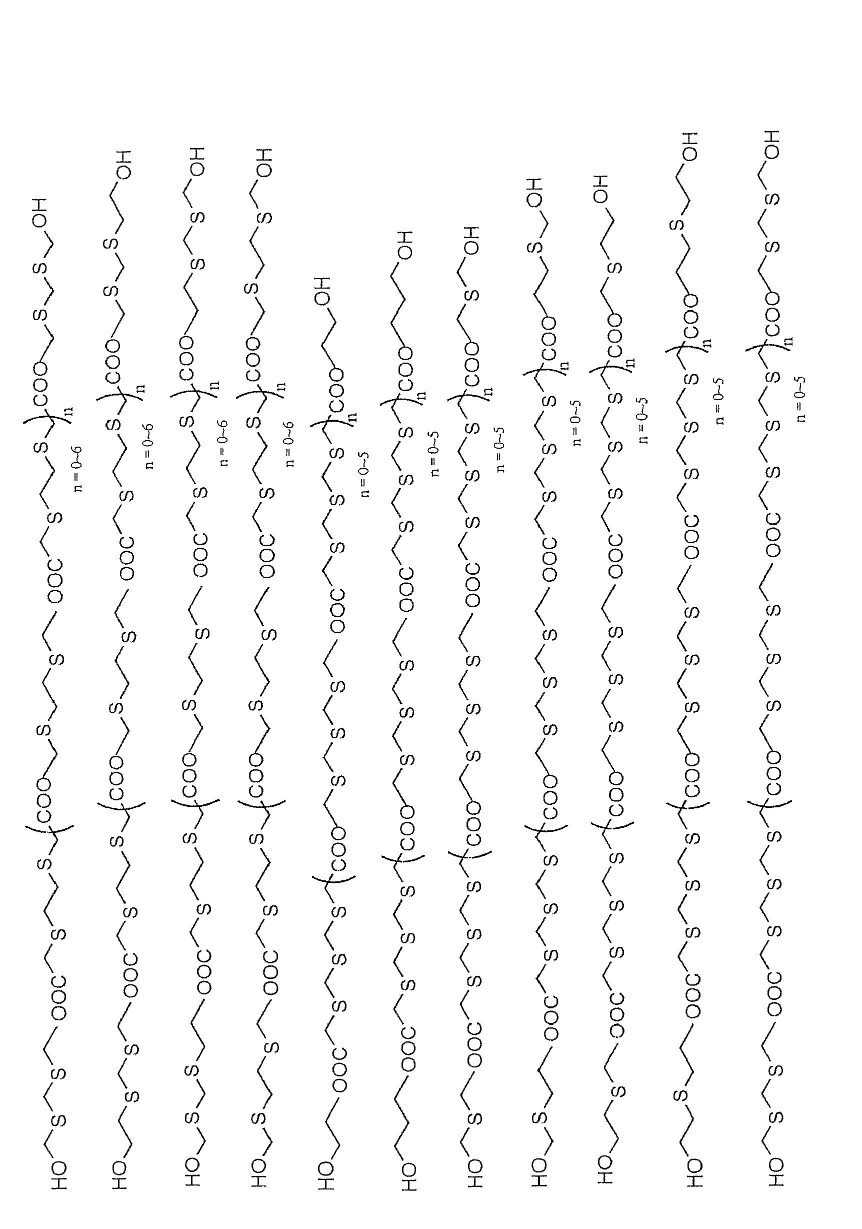

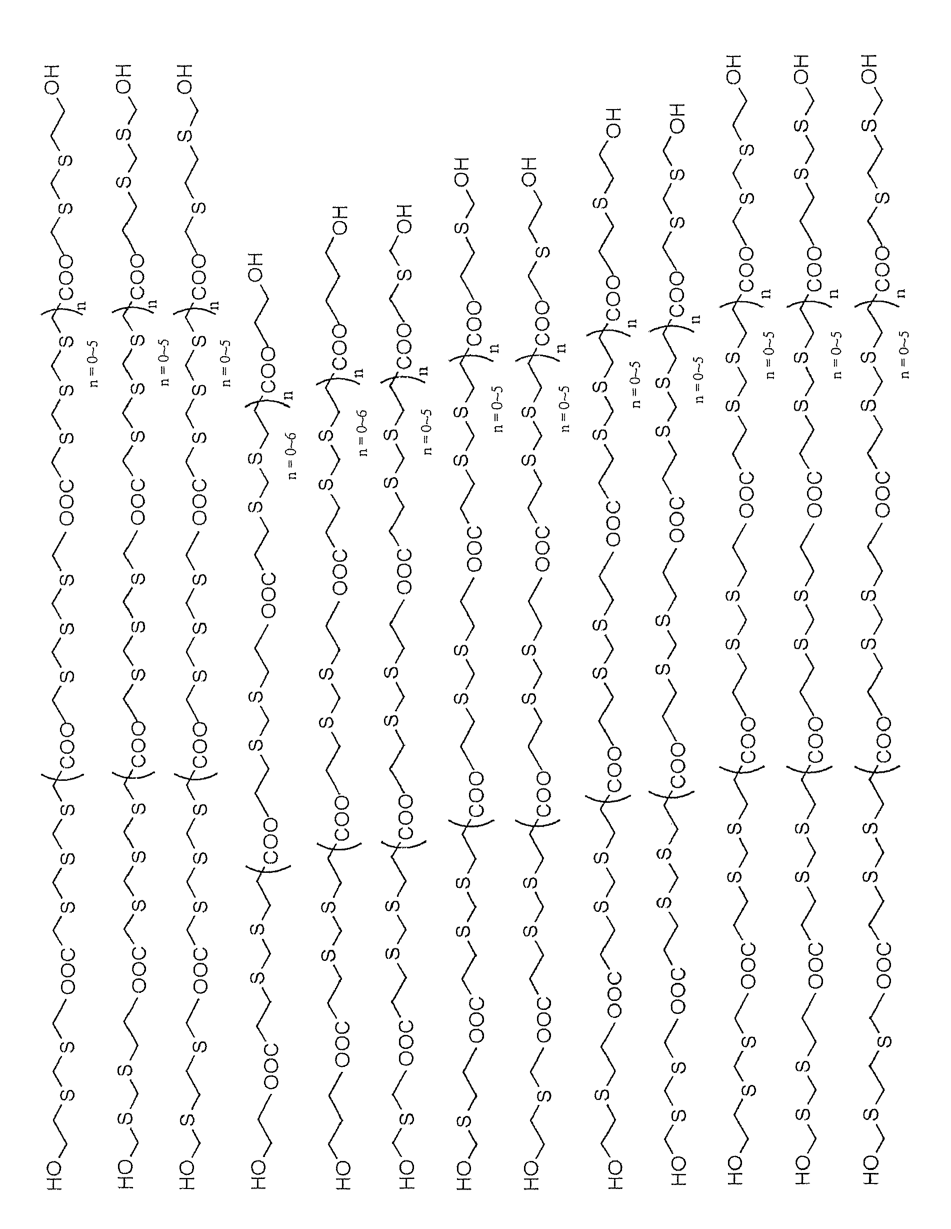

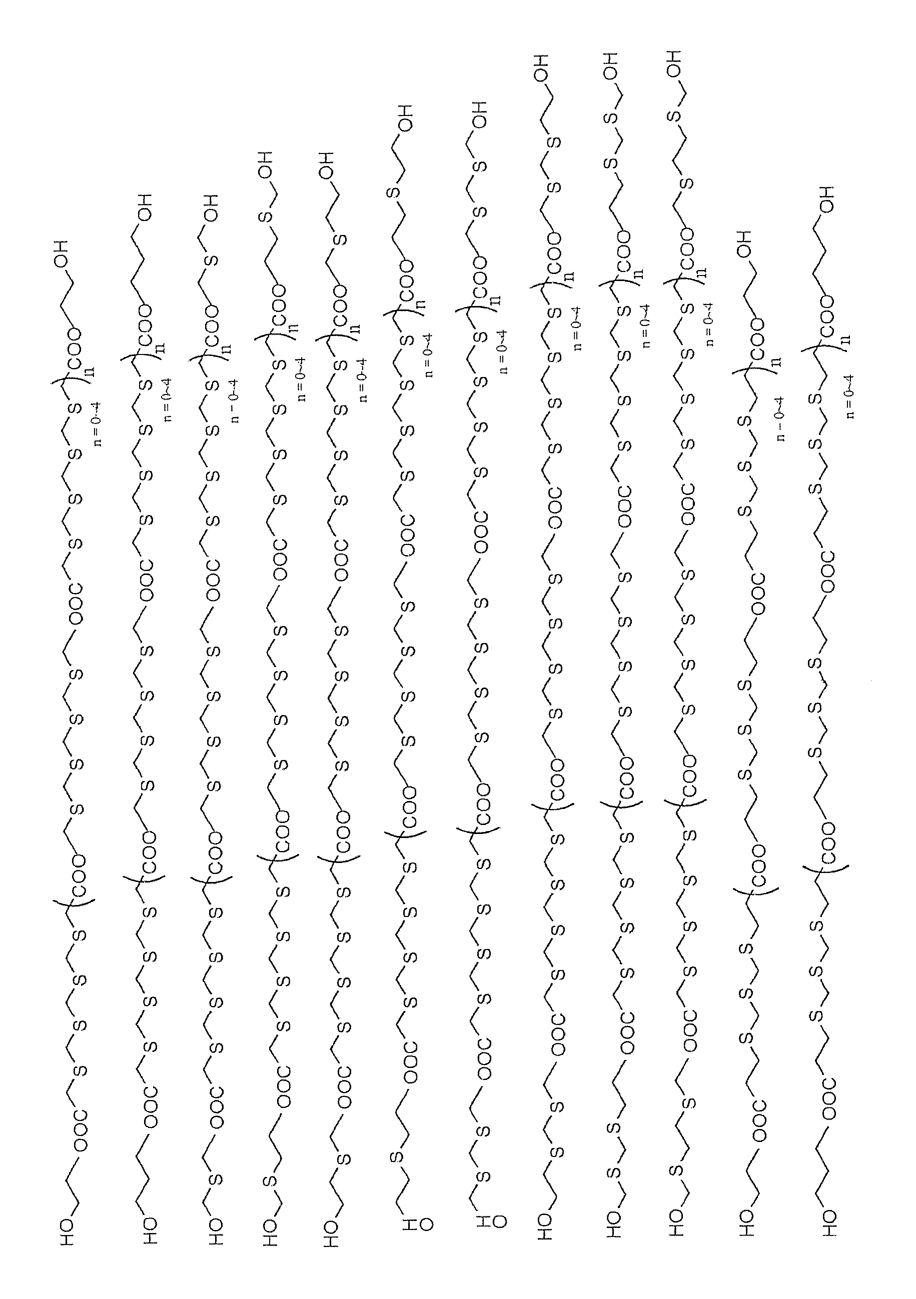

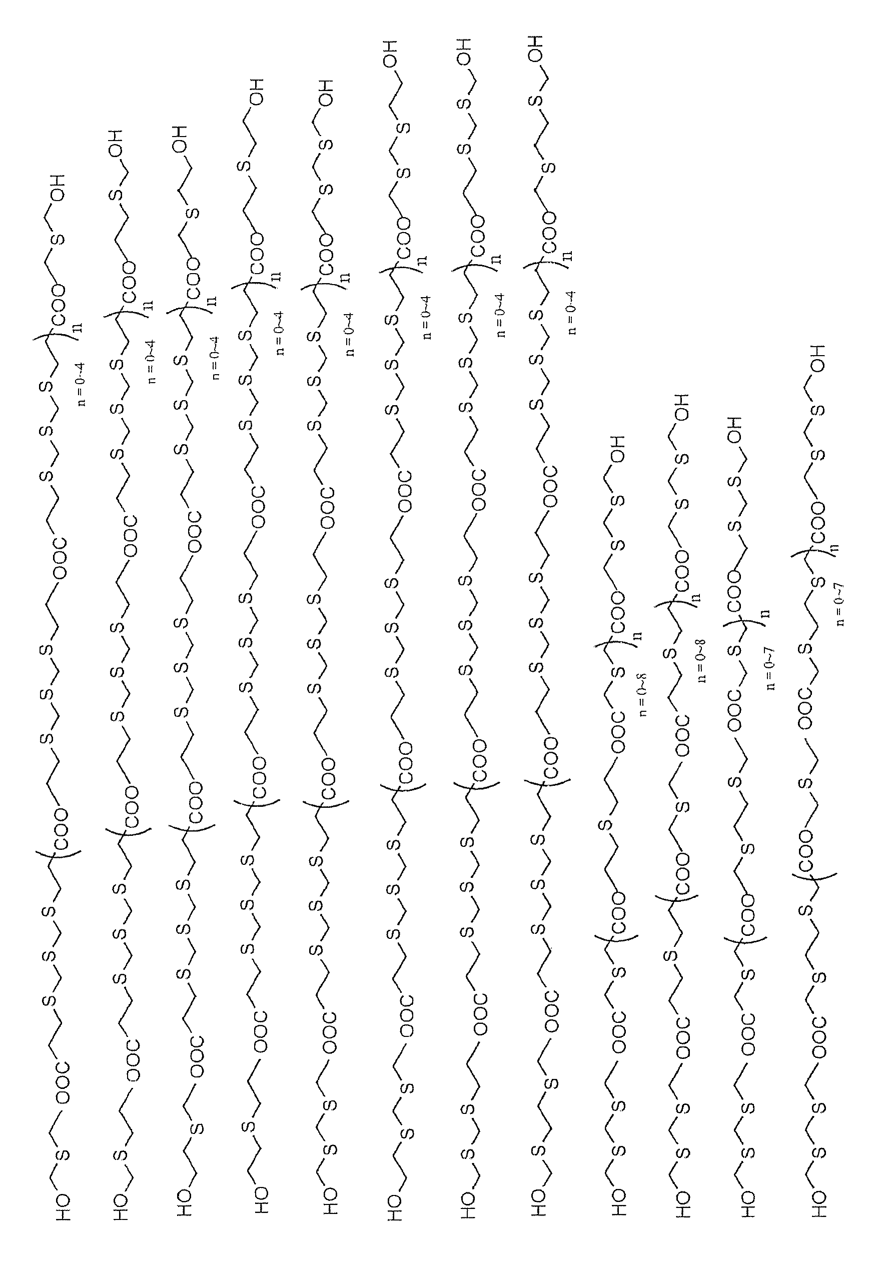

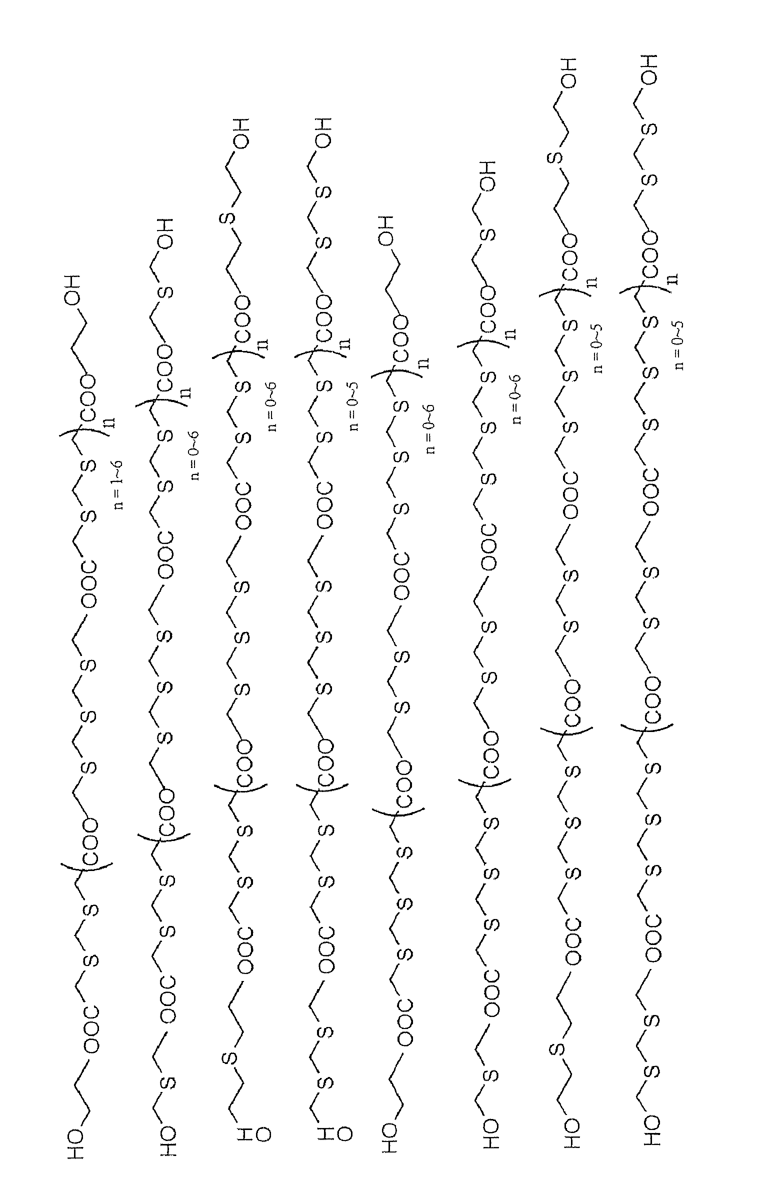

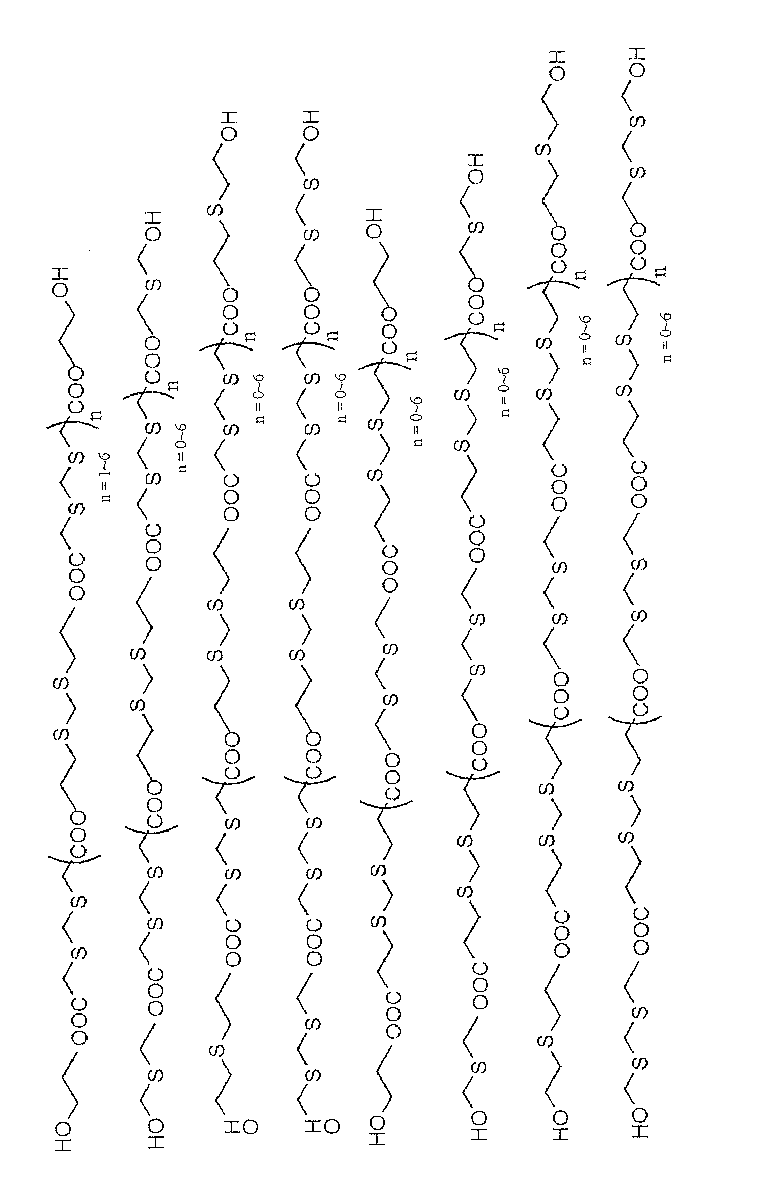

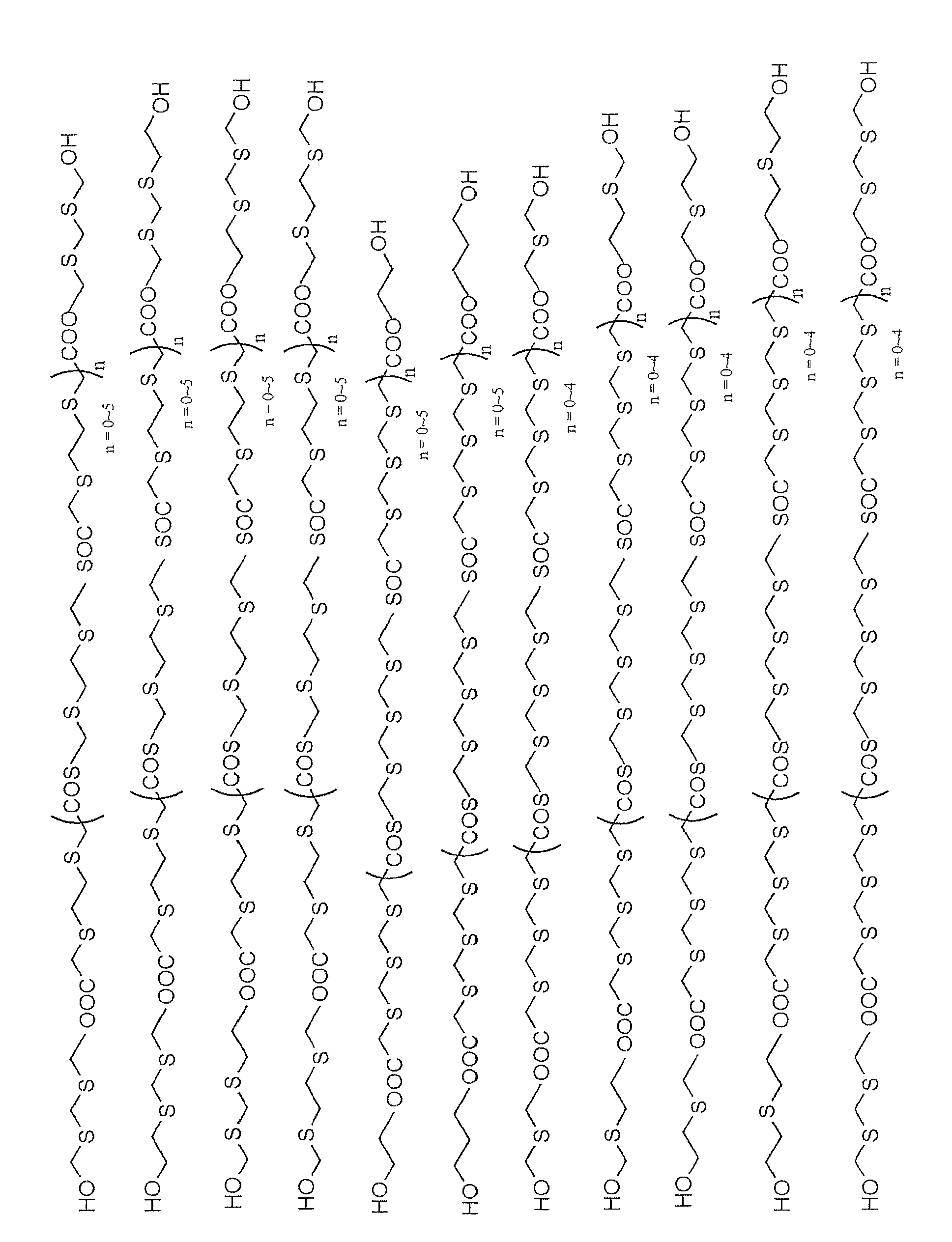

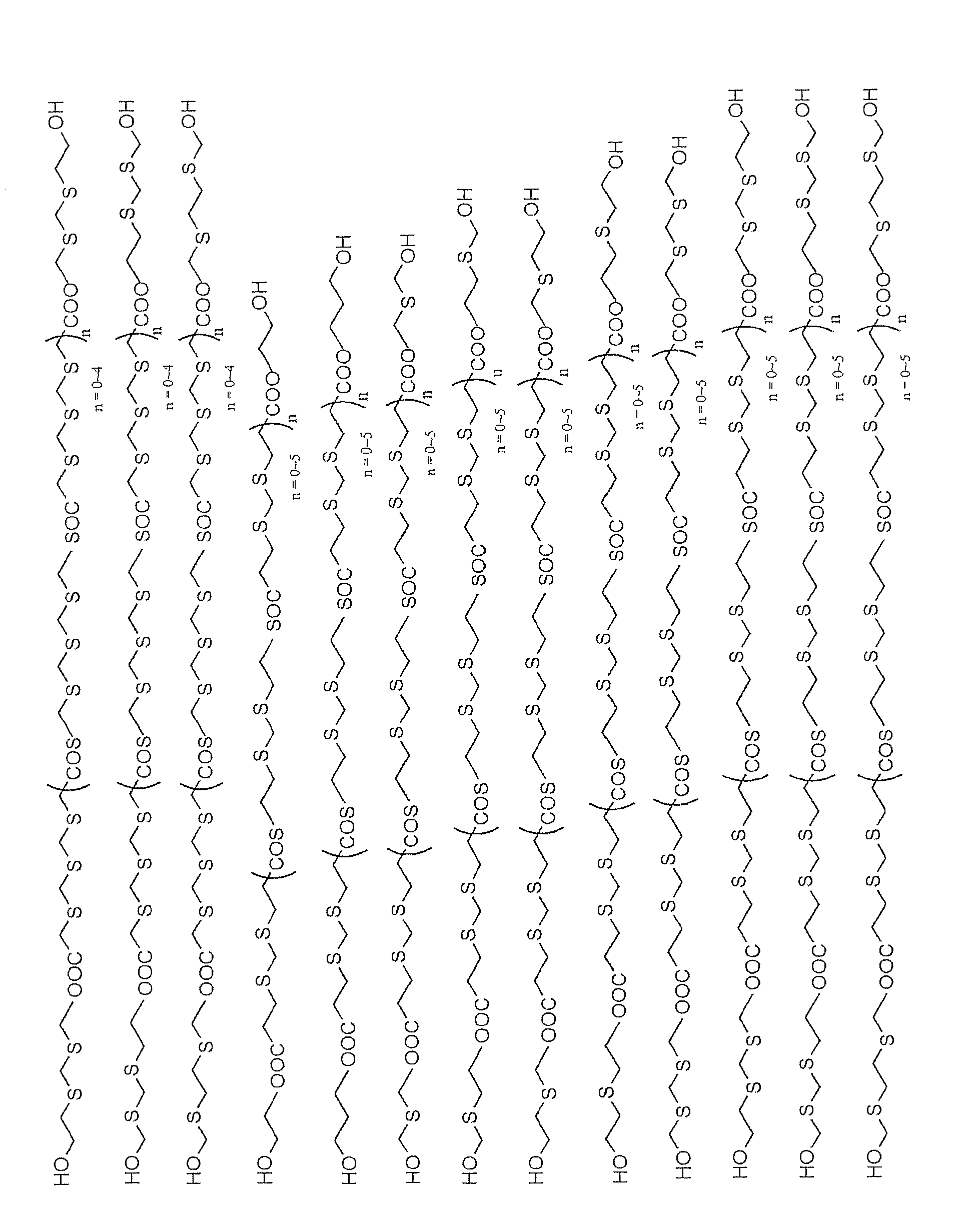

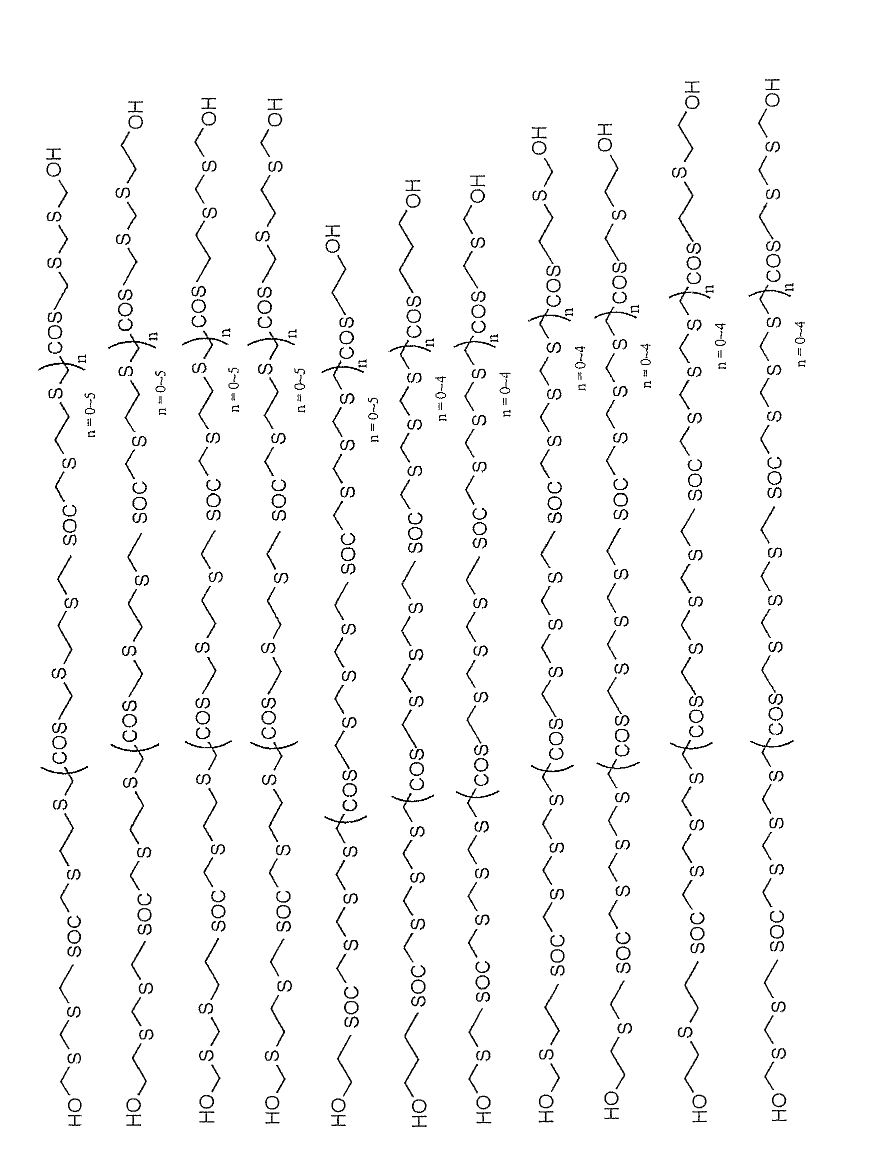

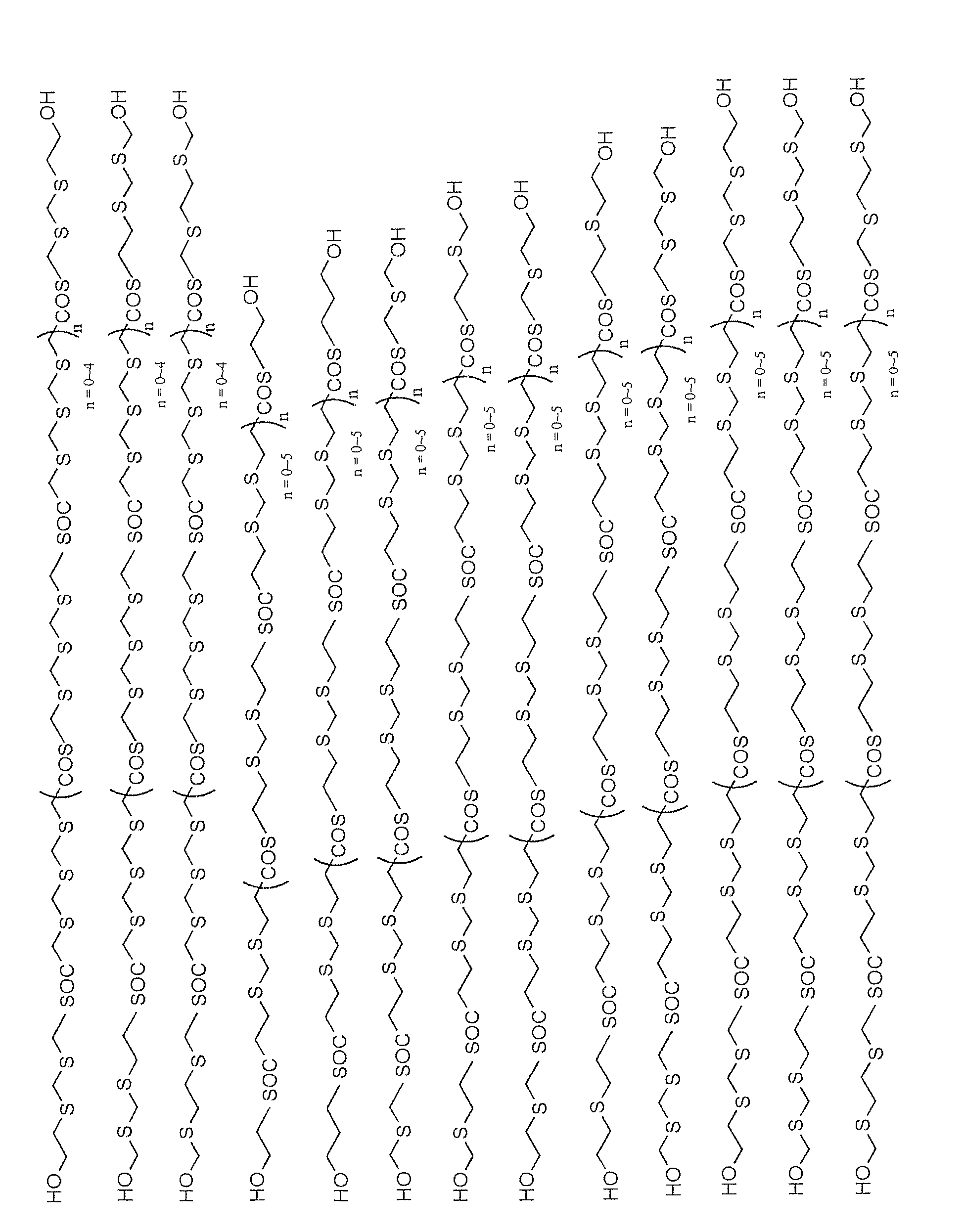

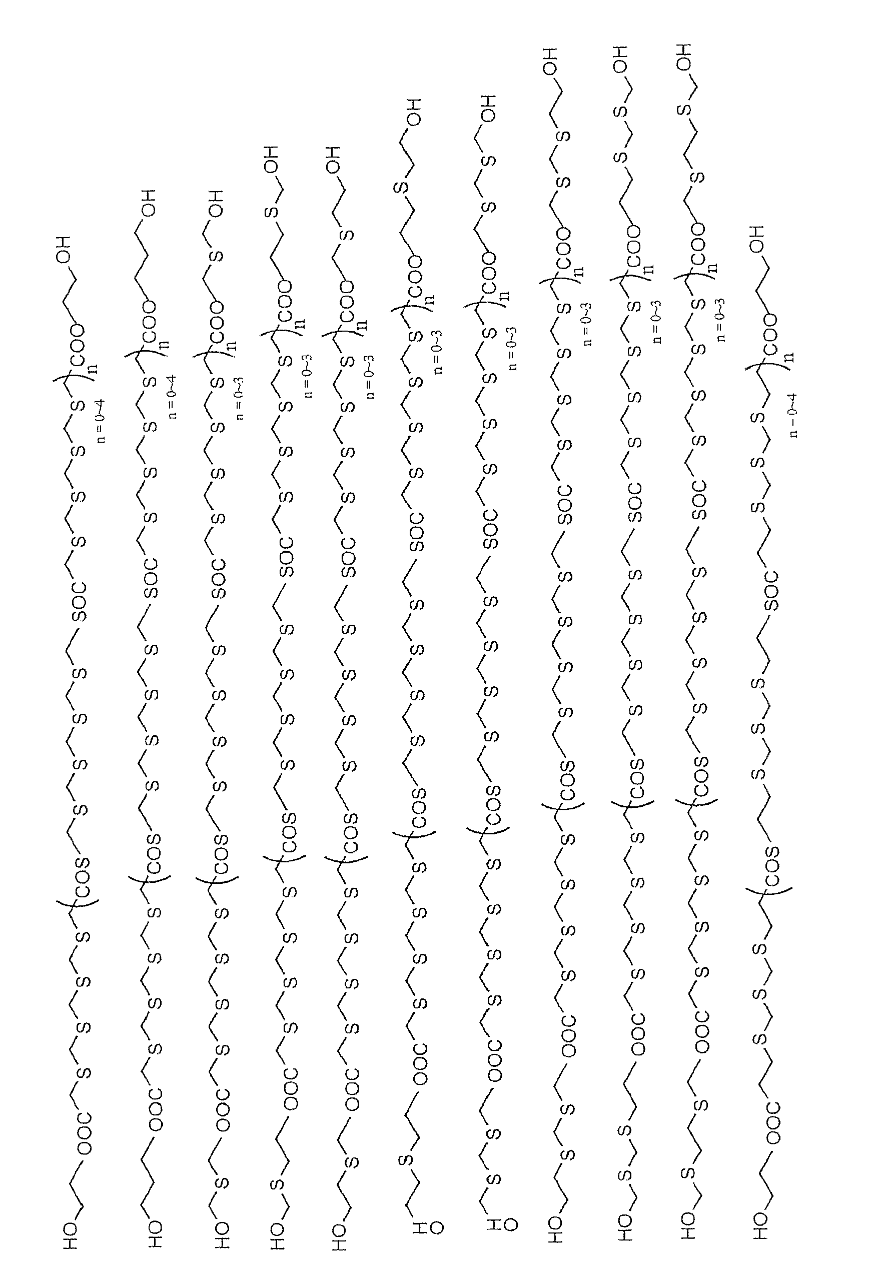

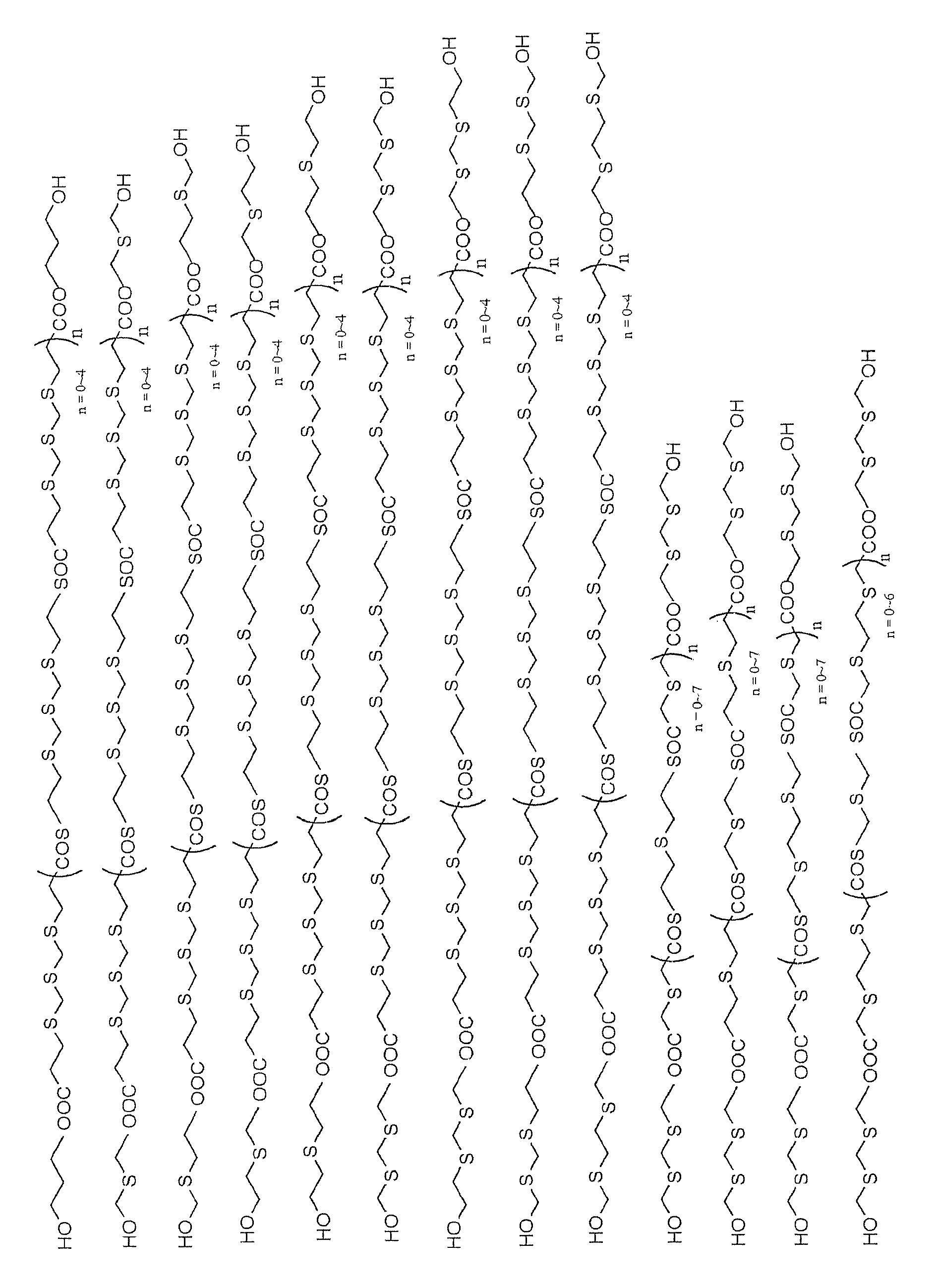

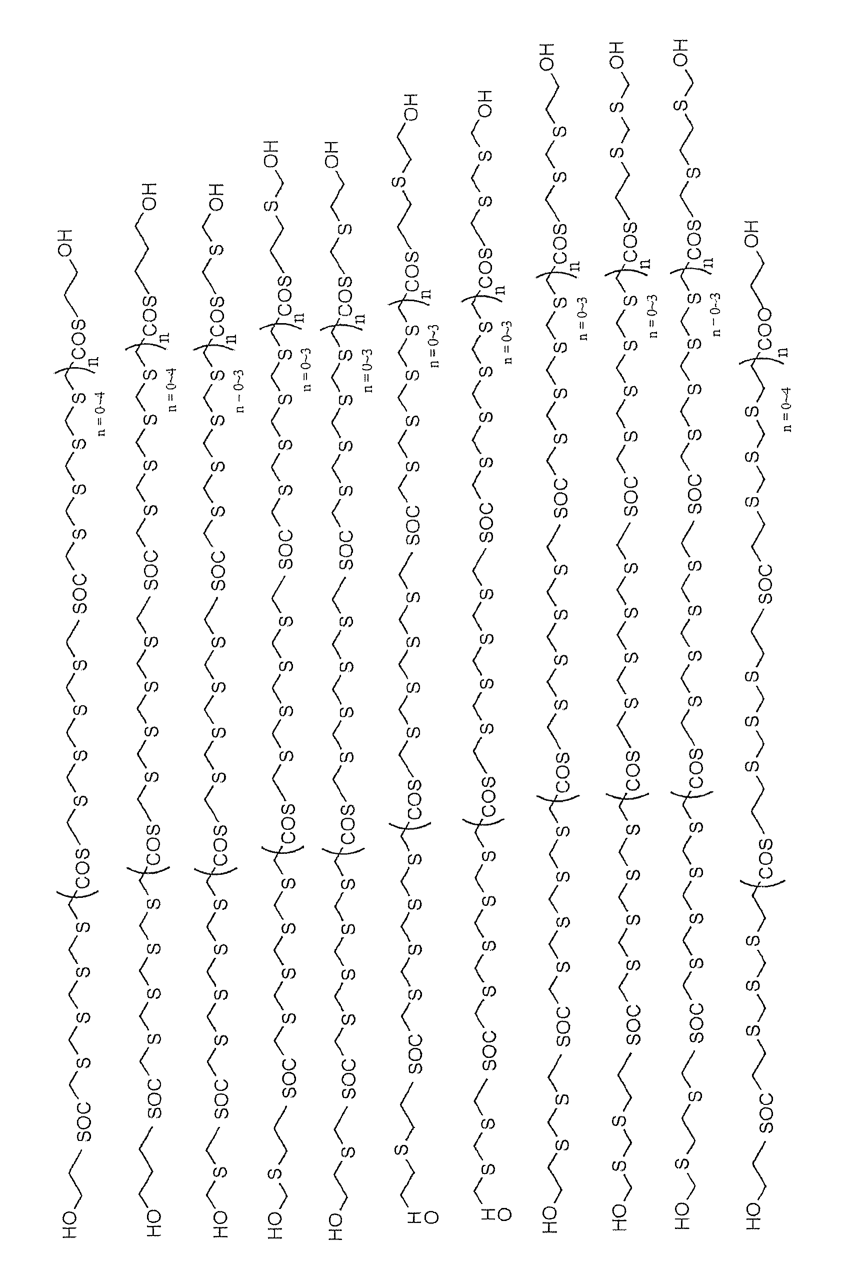

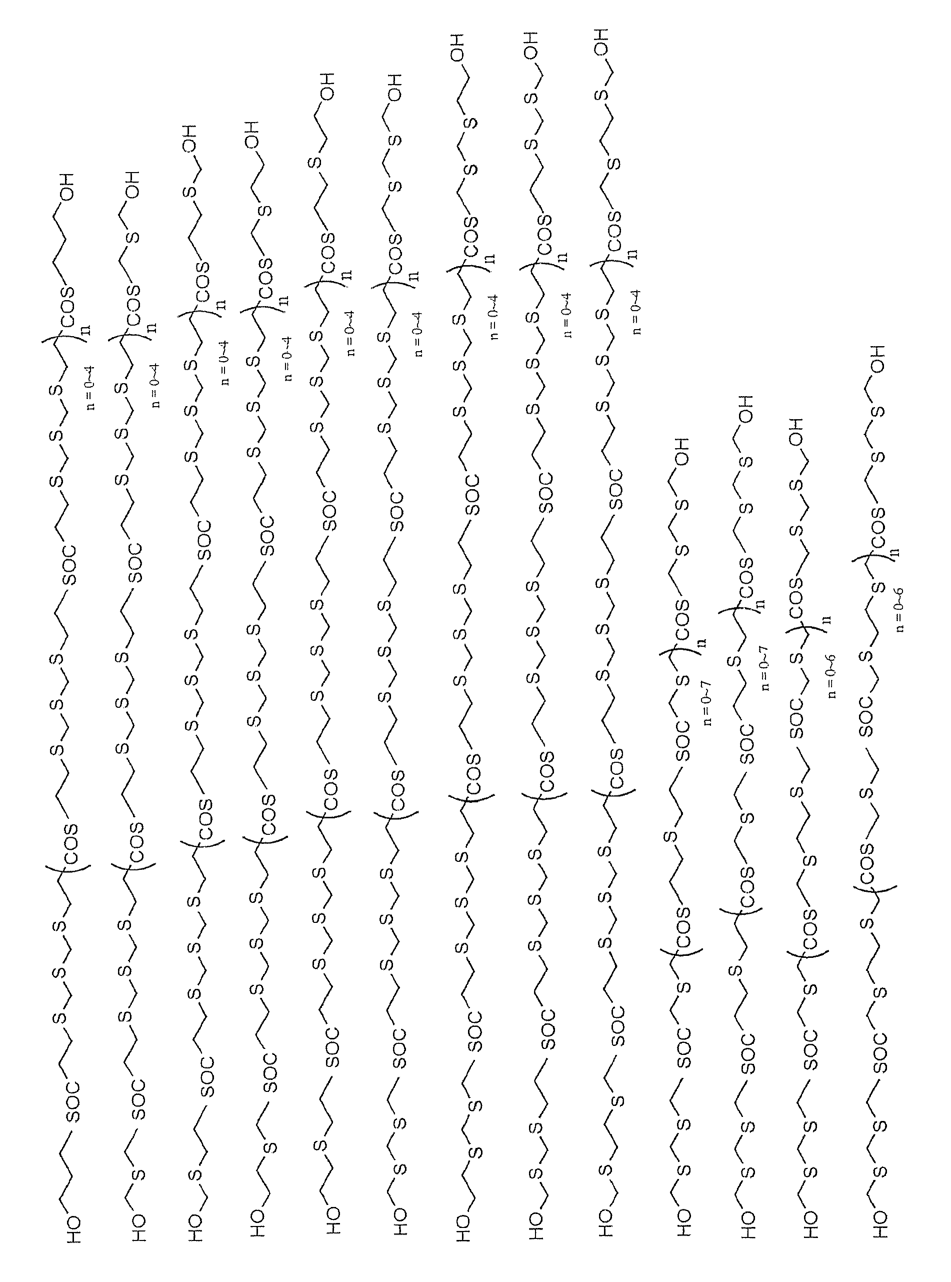

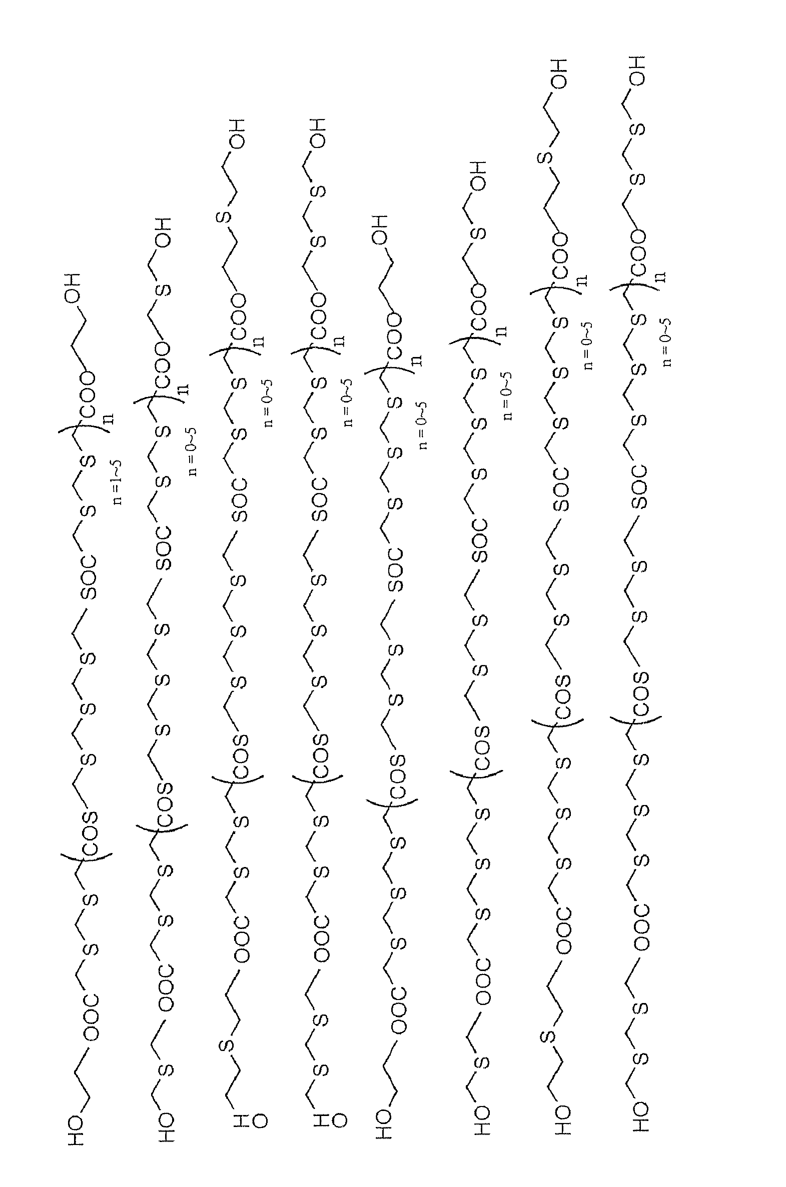

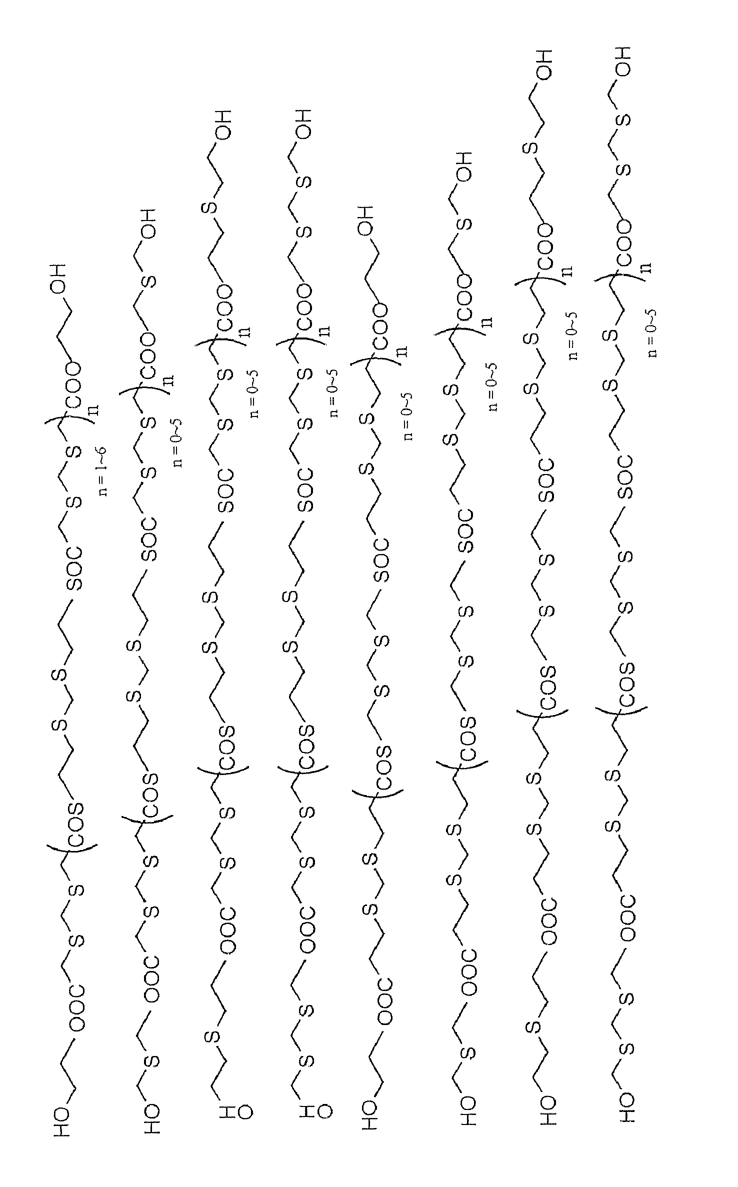

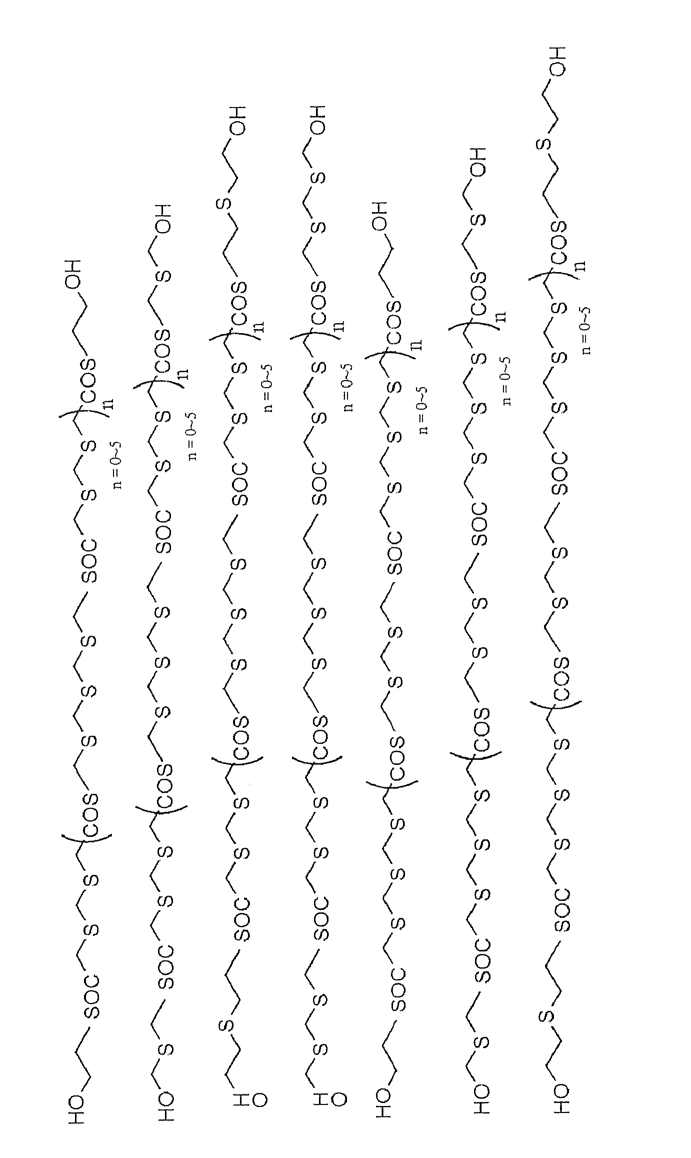

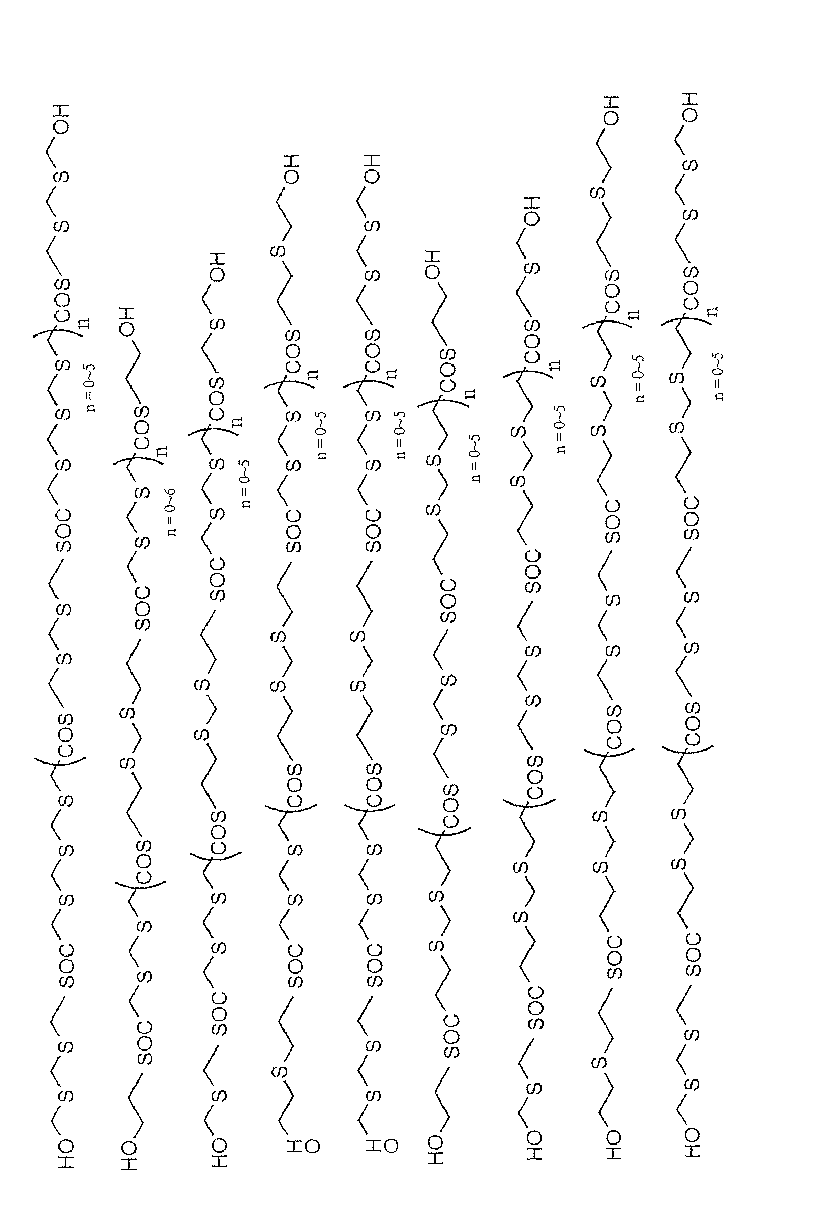

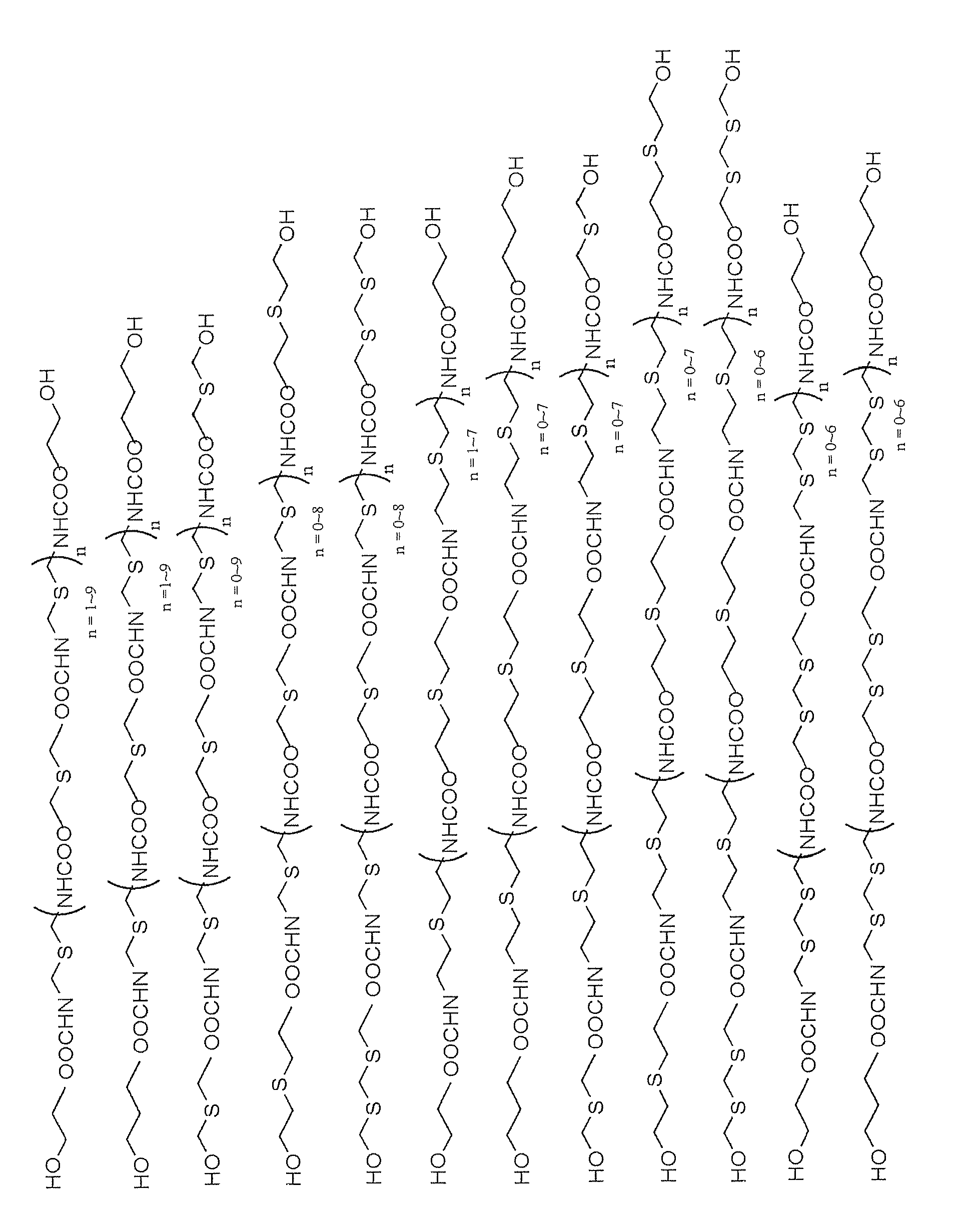

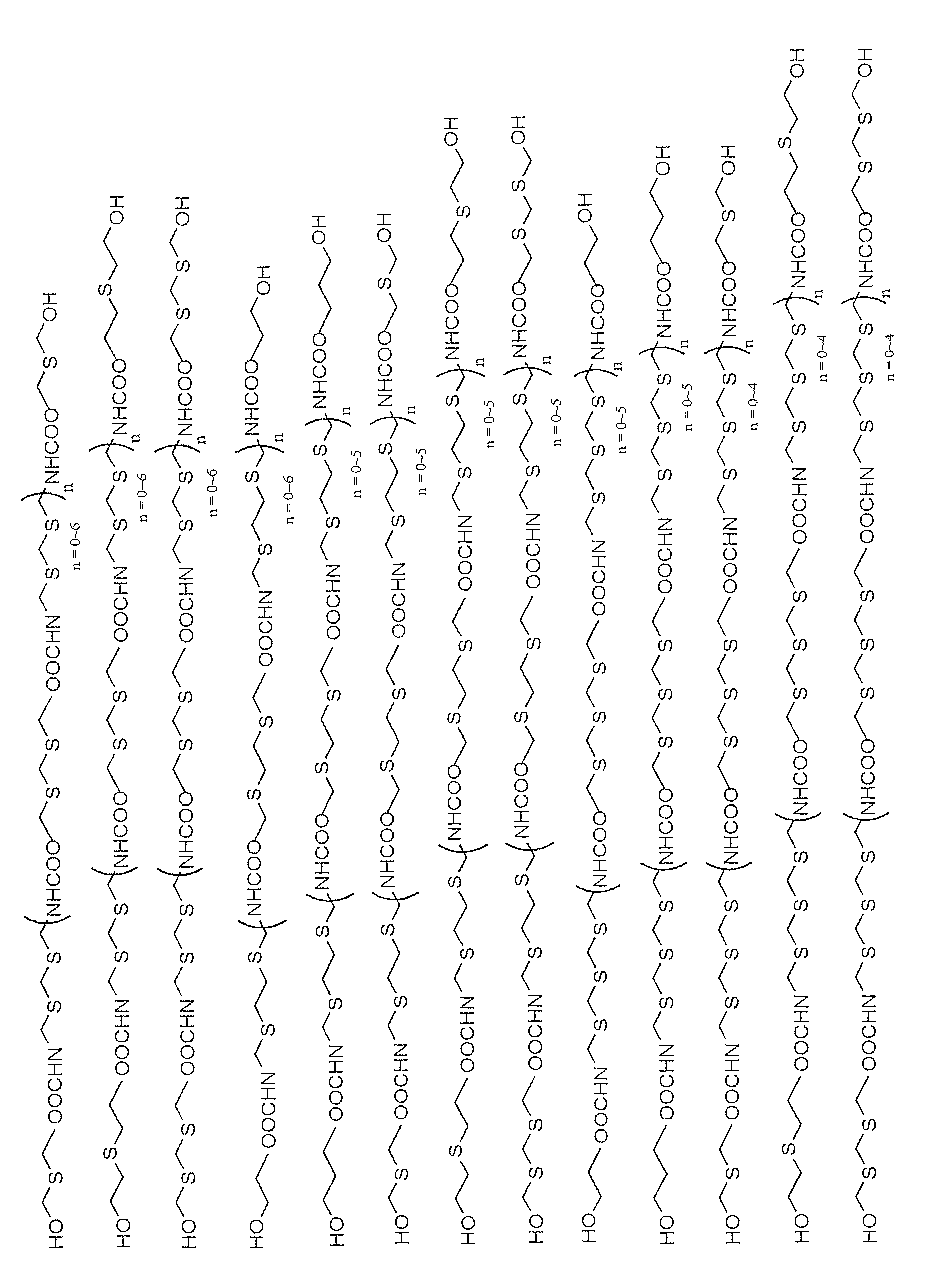

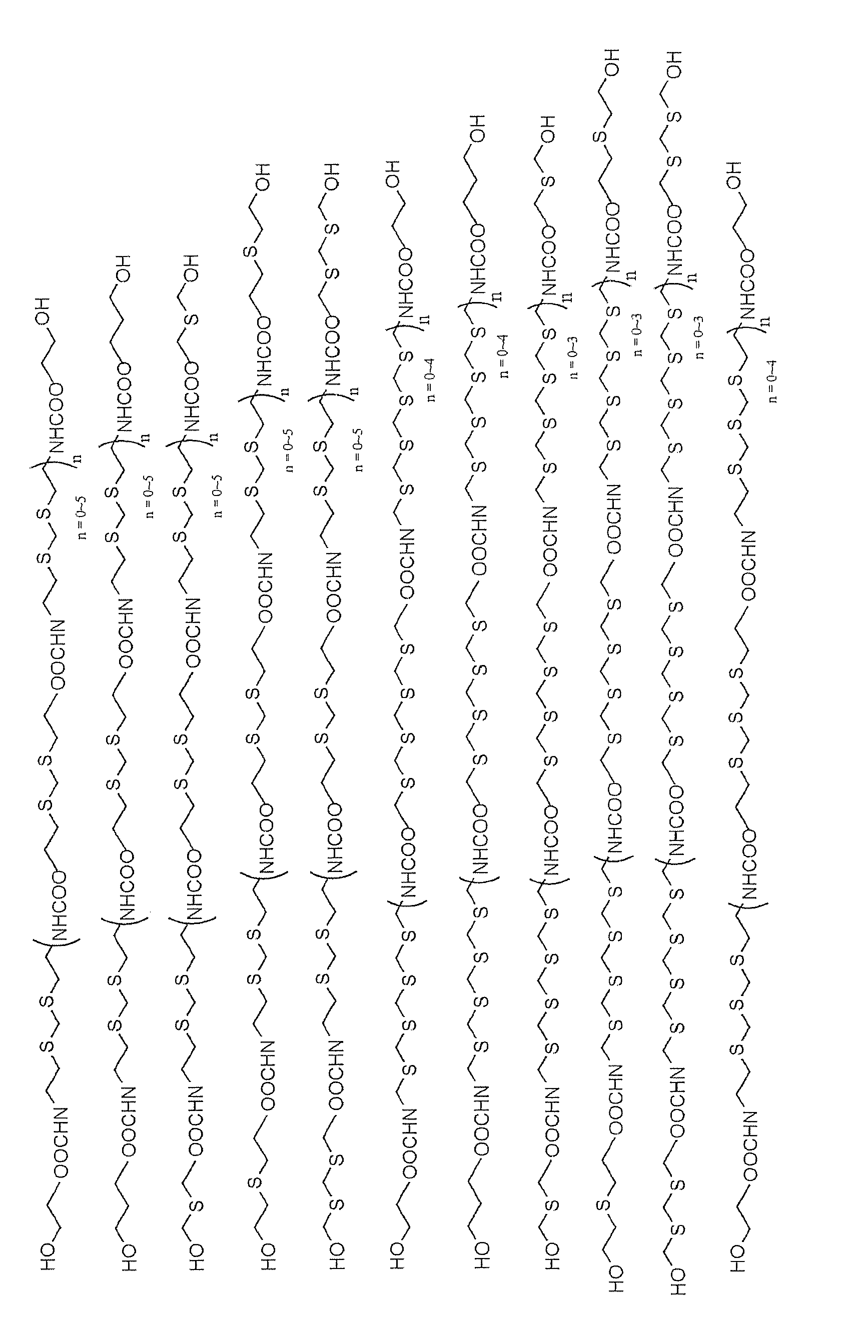

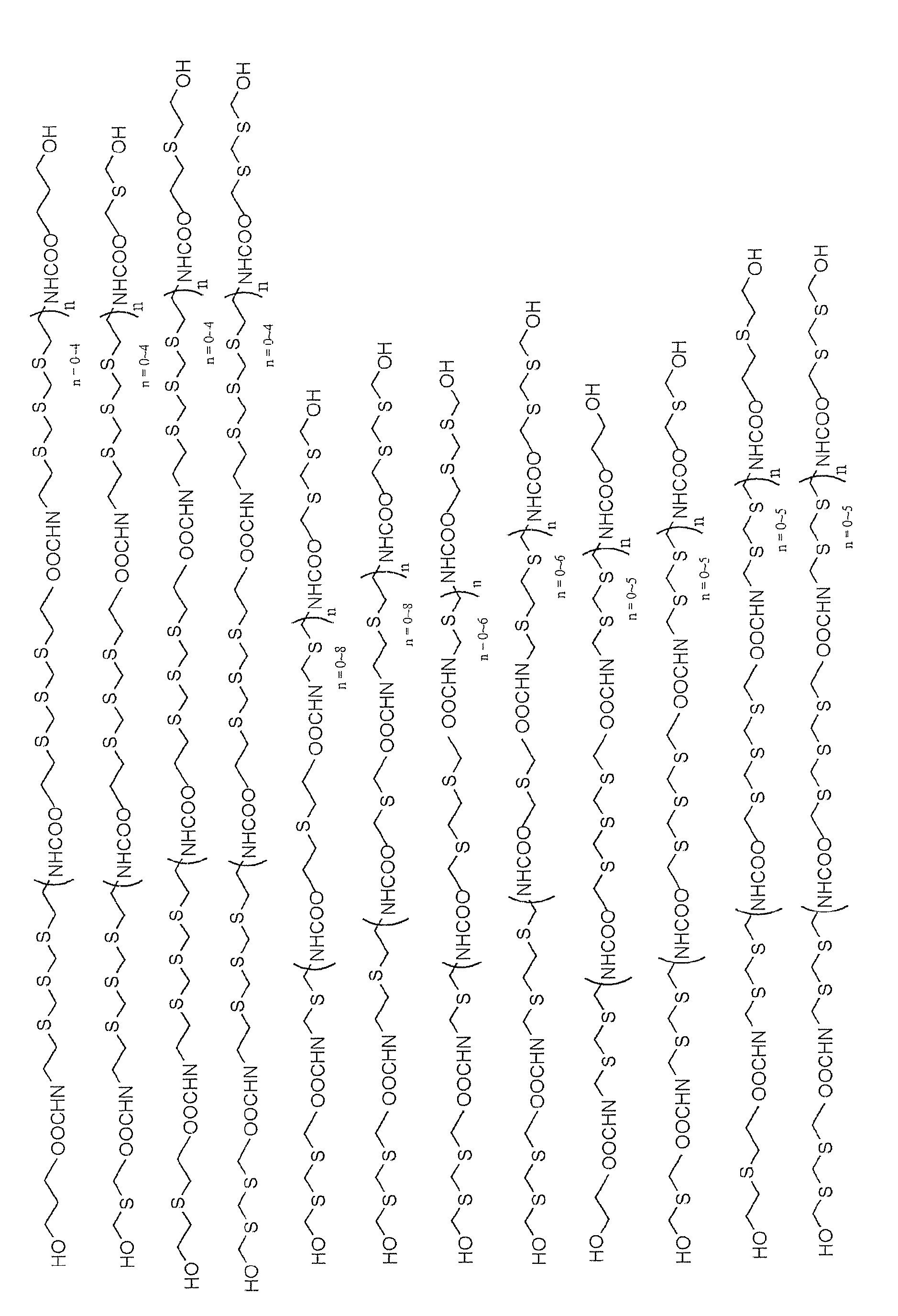

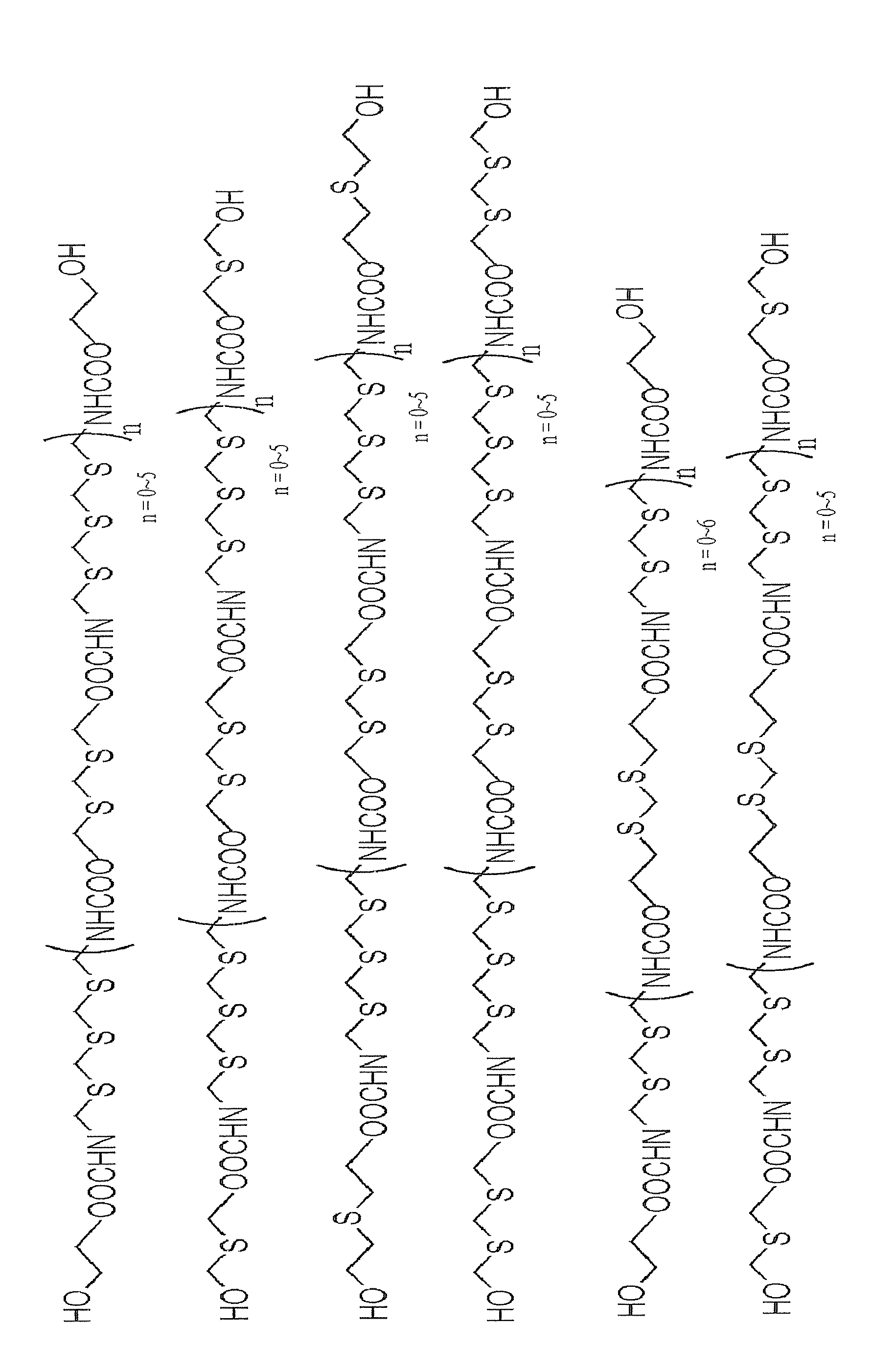

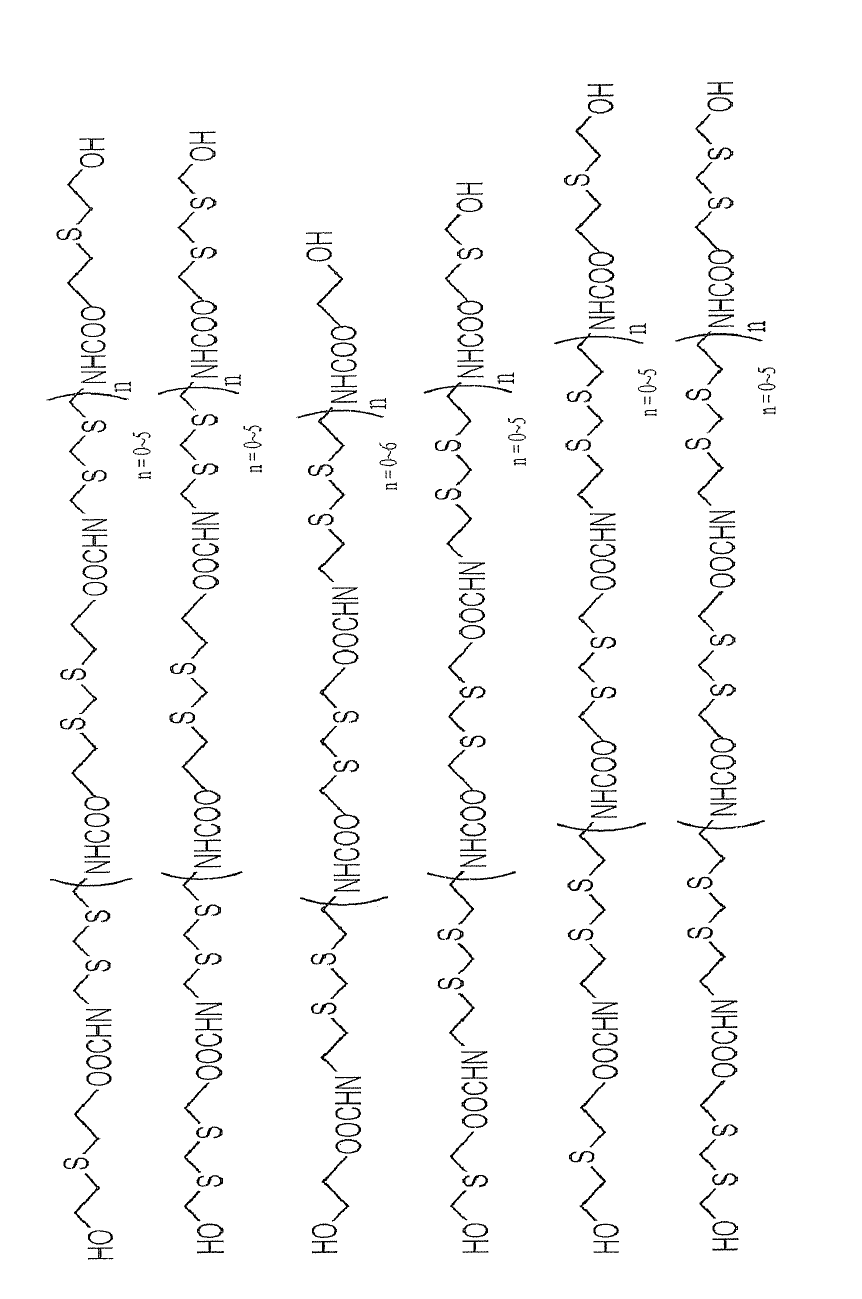

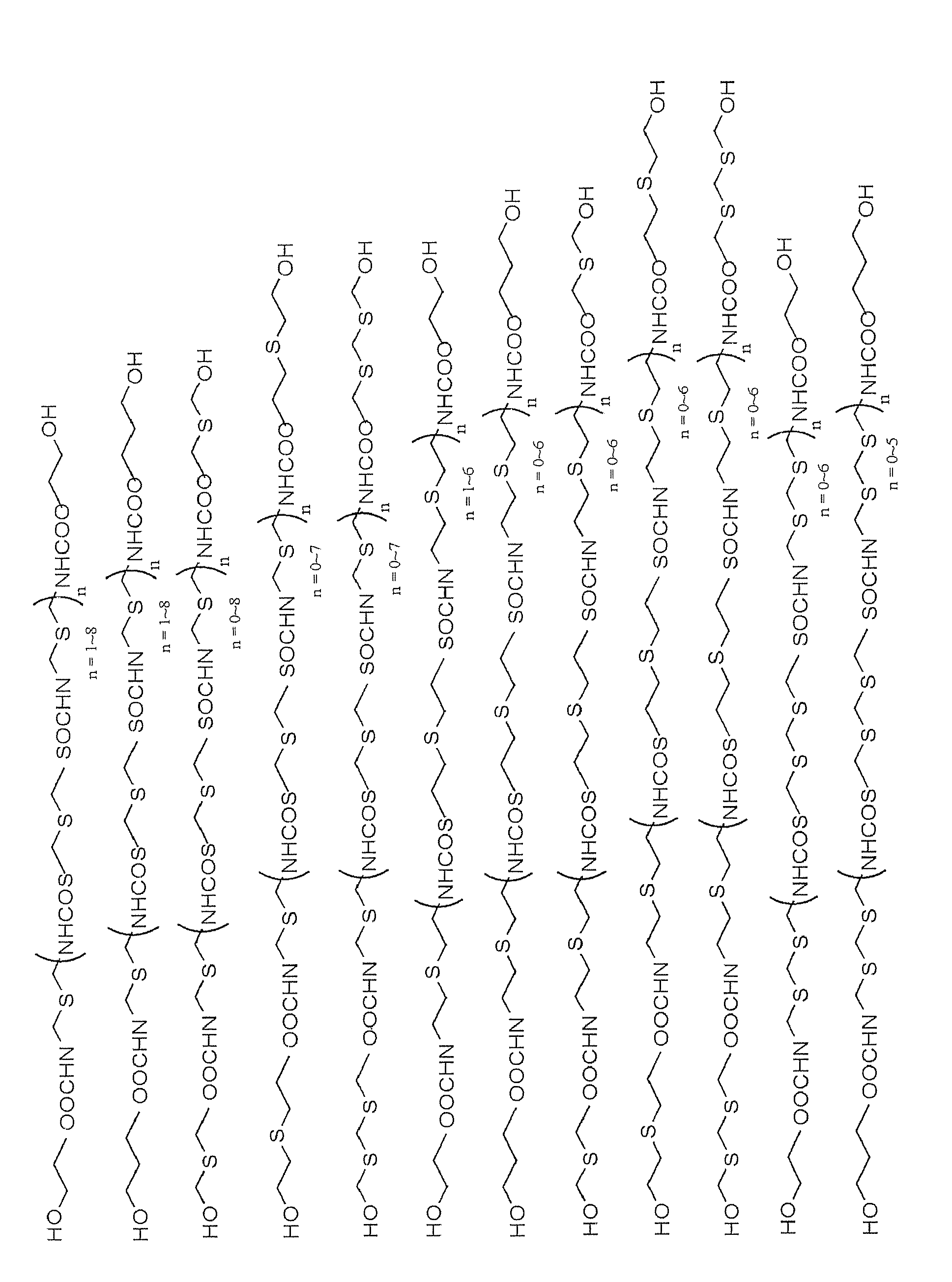

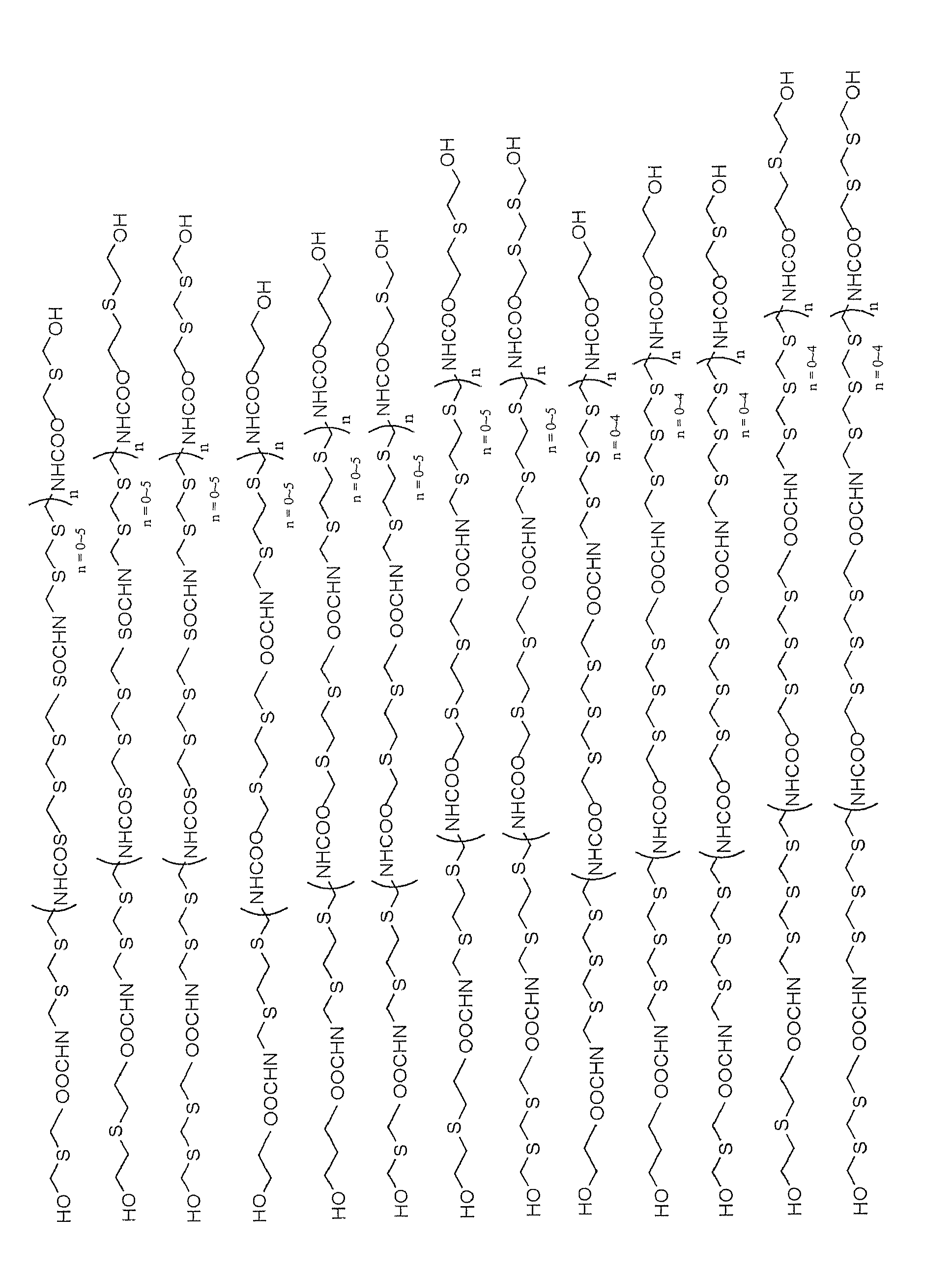

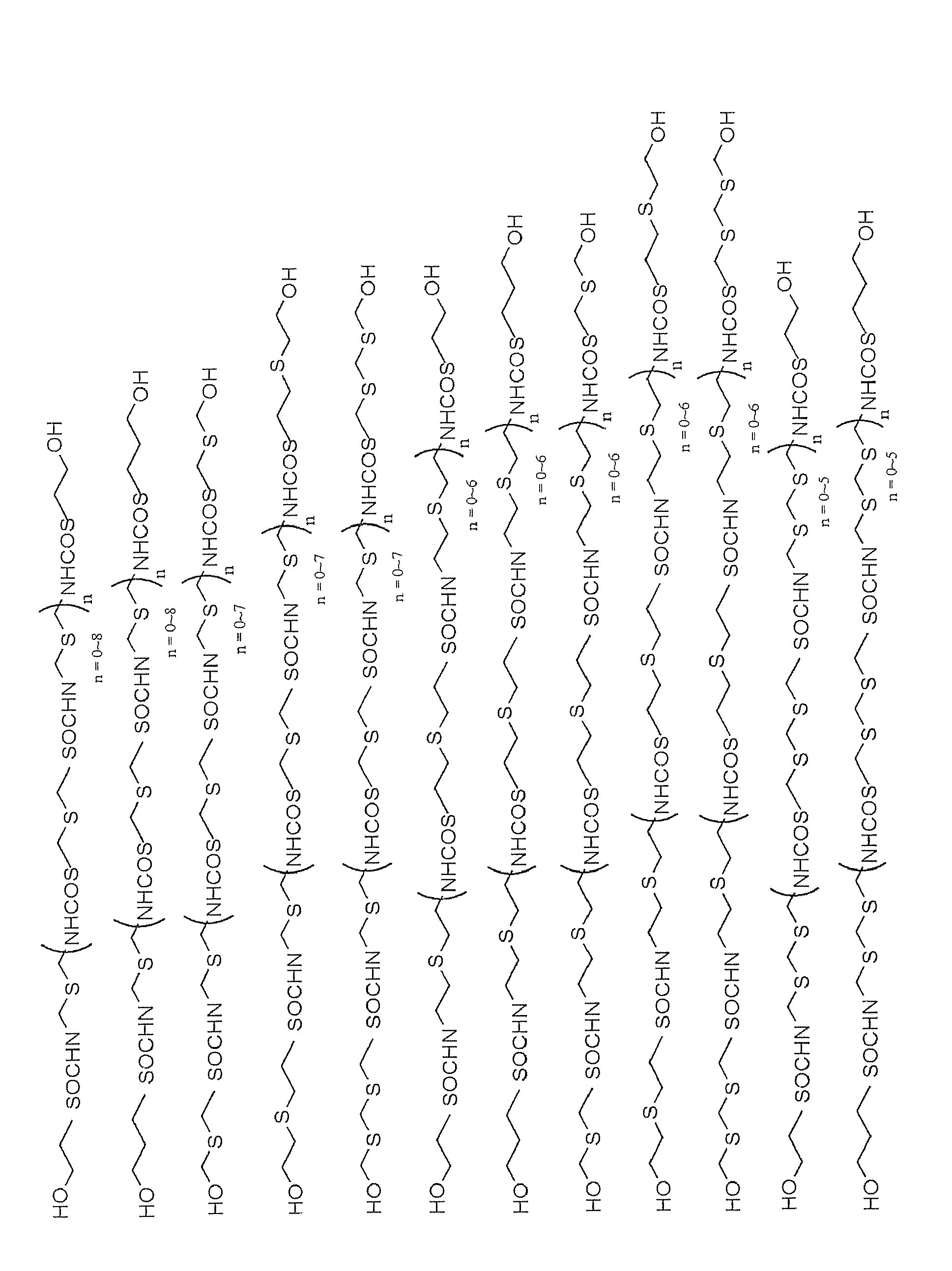

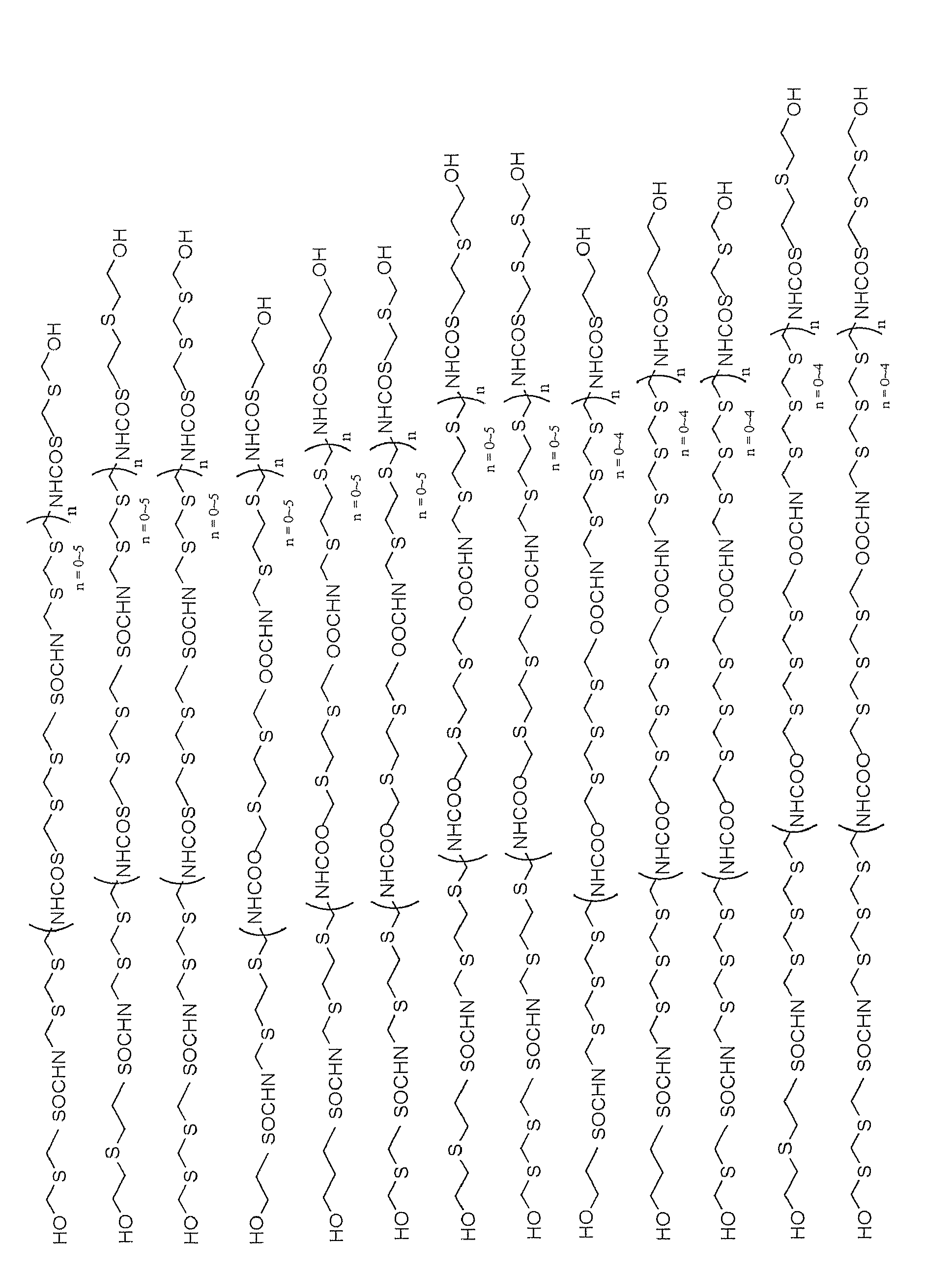

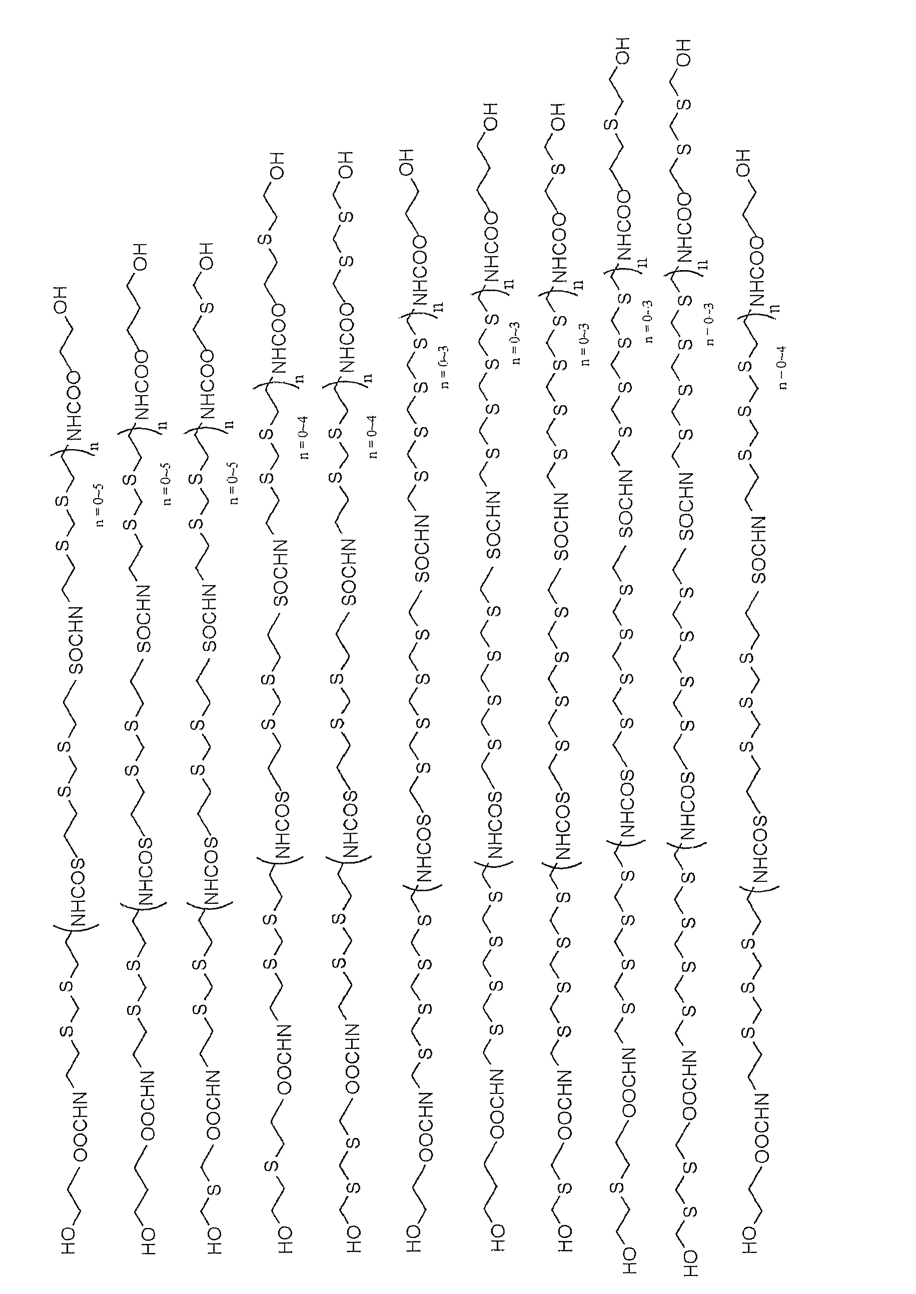

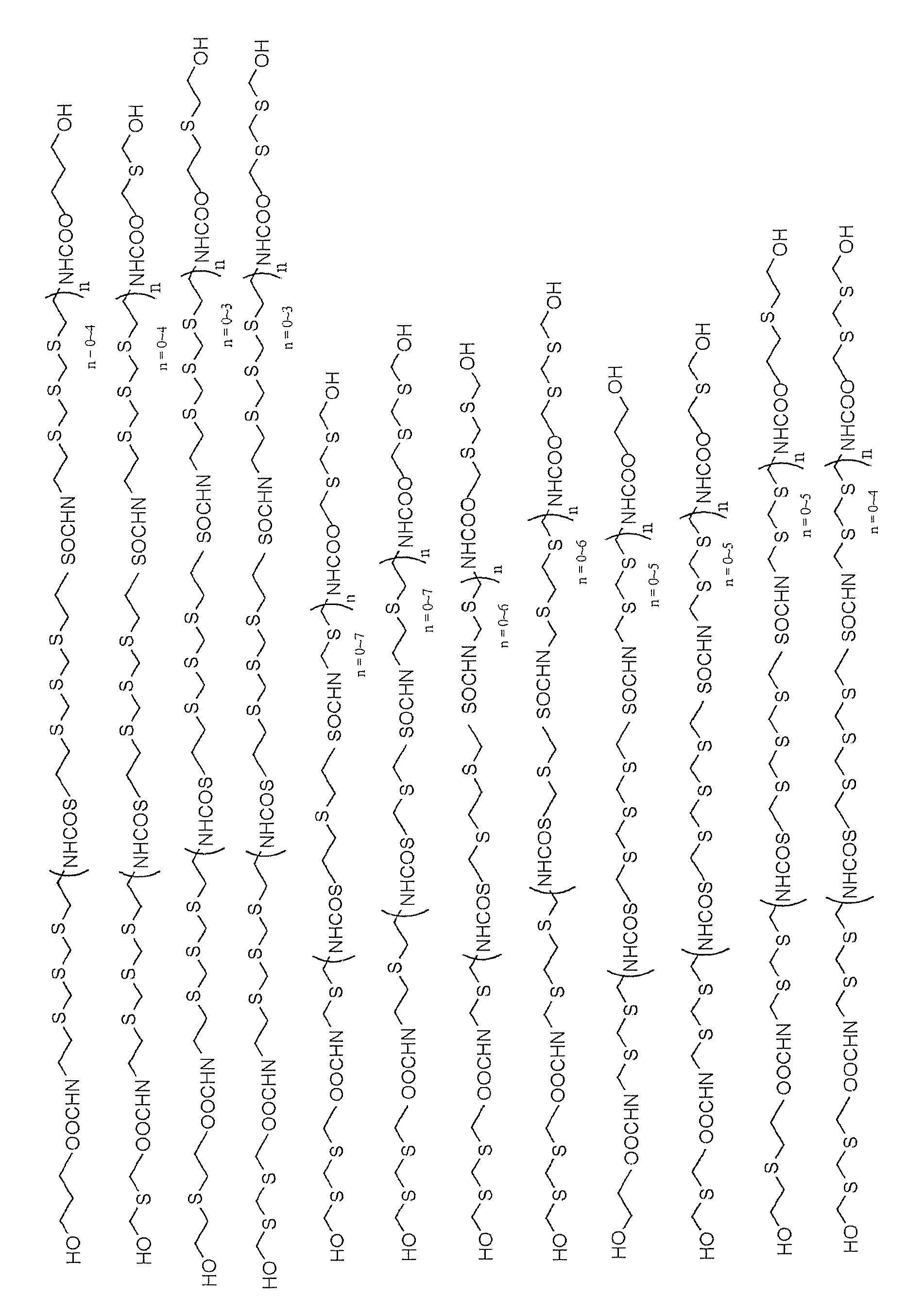

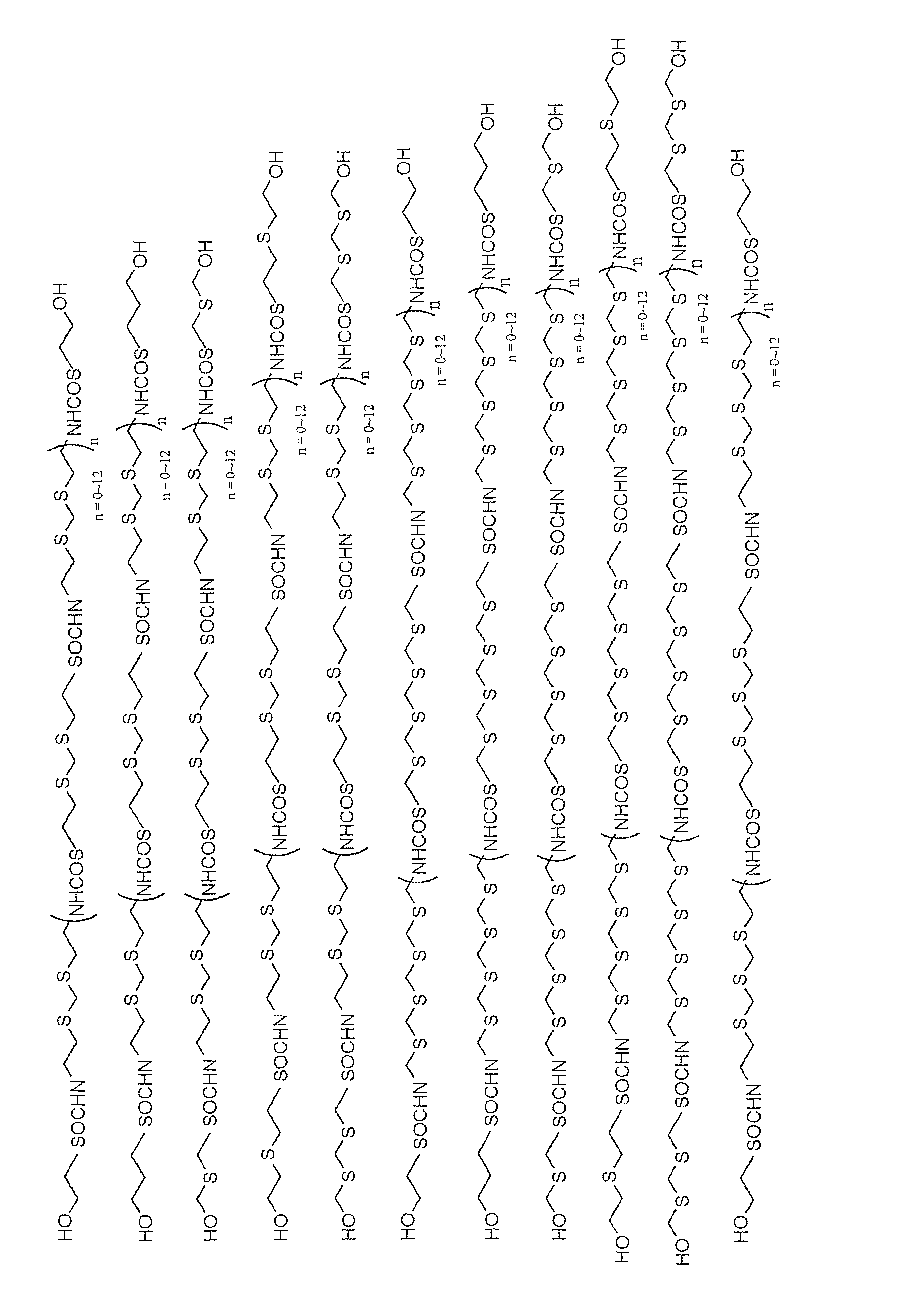

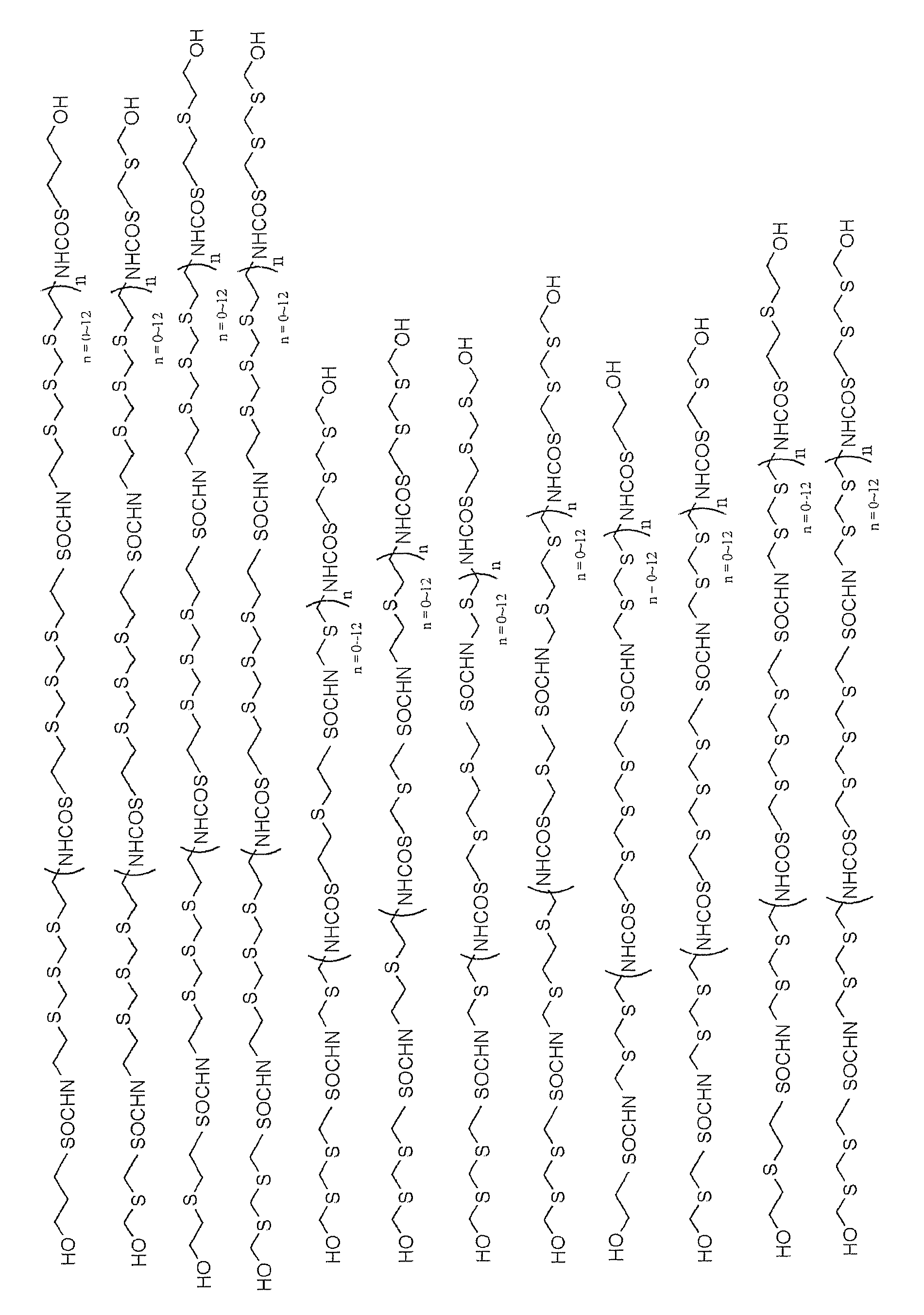

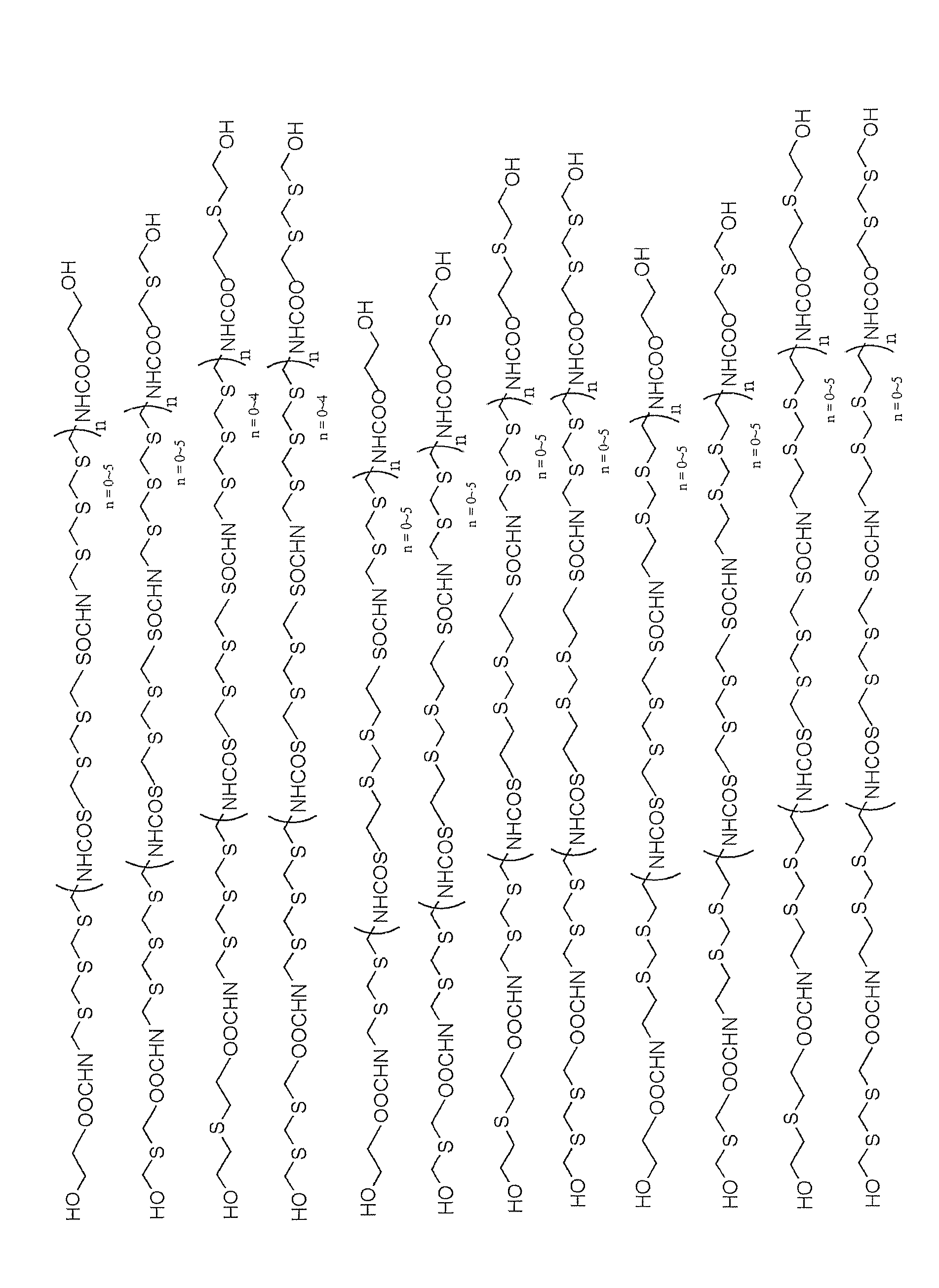

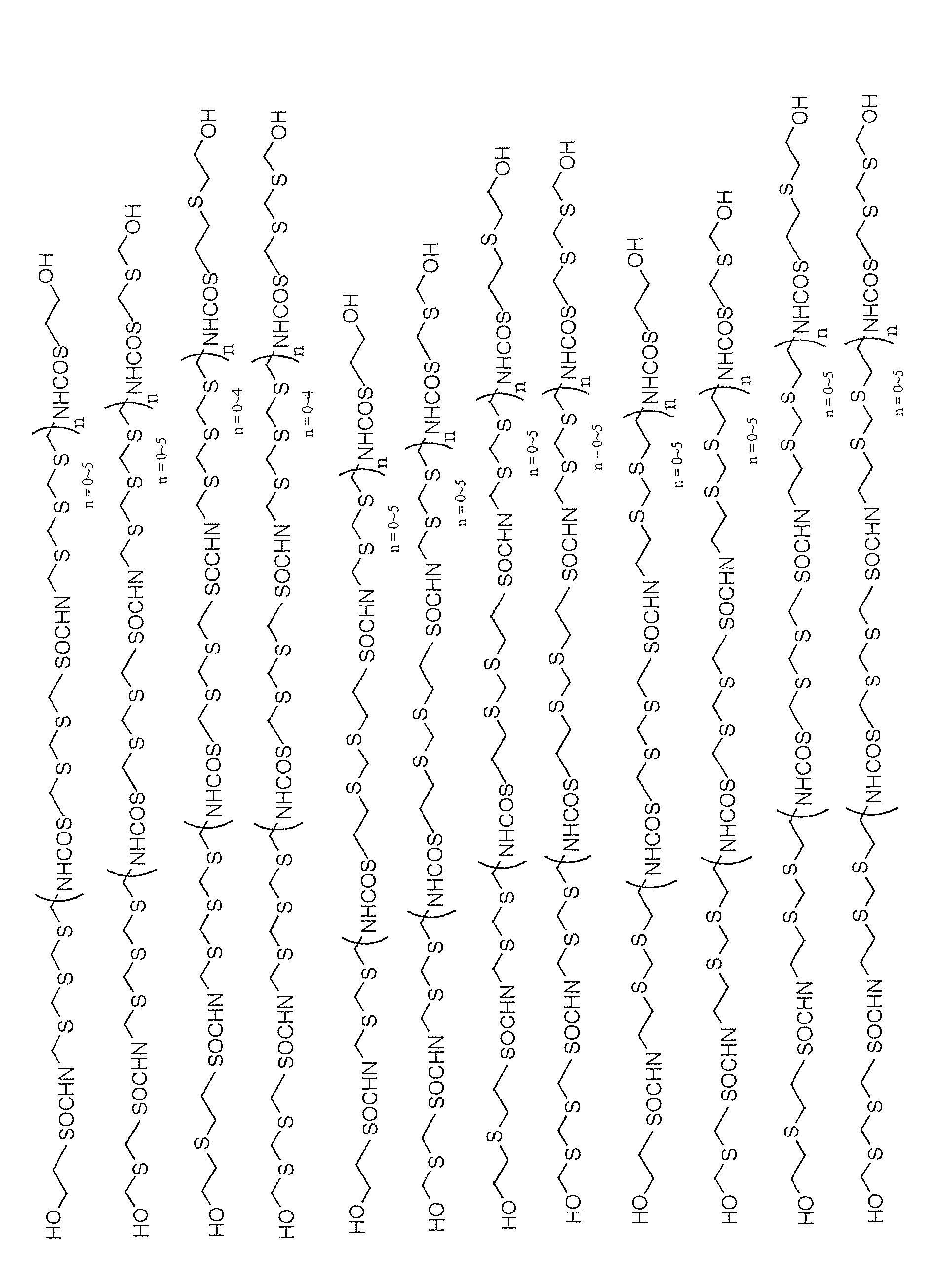

Among them, particularly preferred examples are as follows;

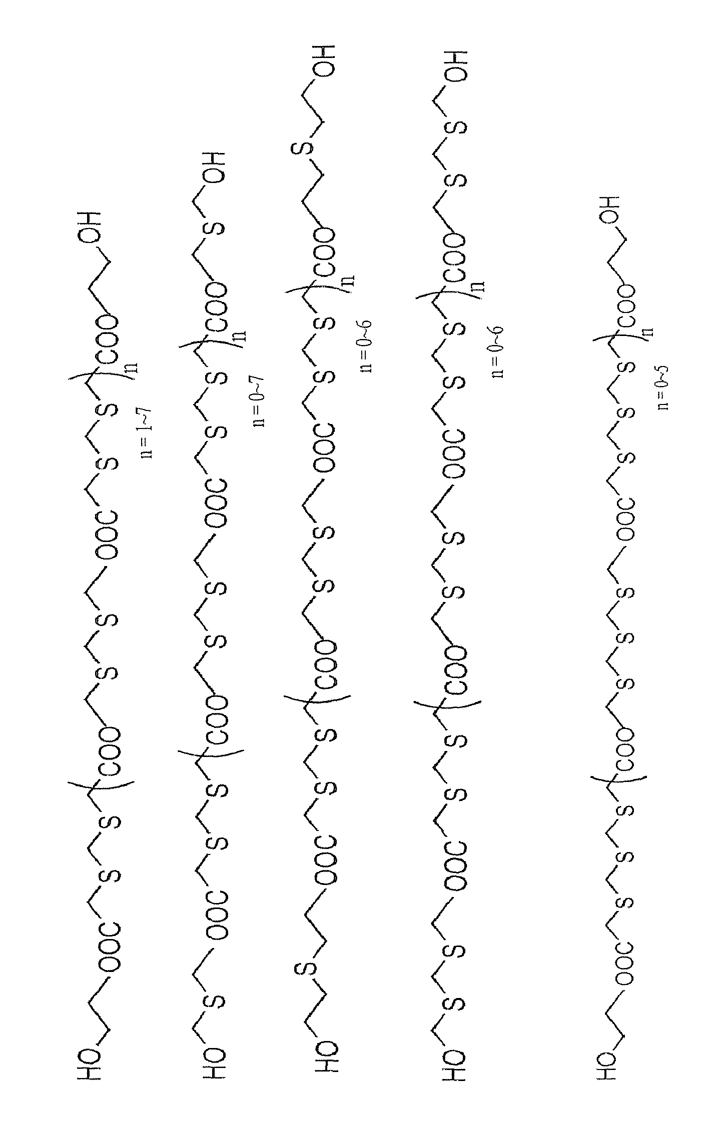

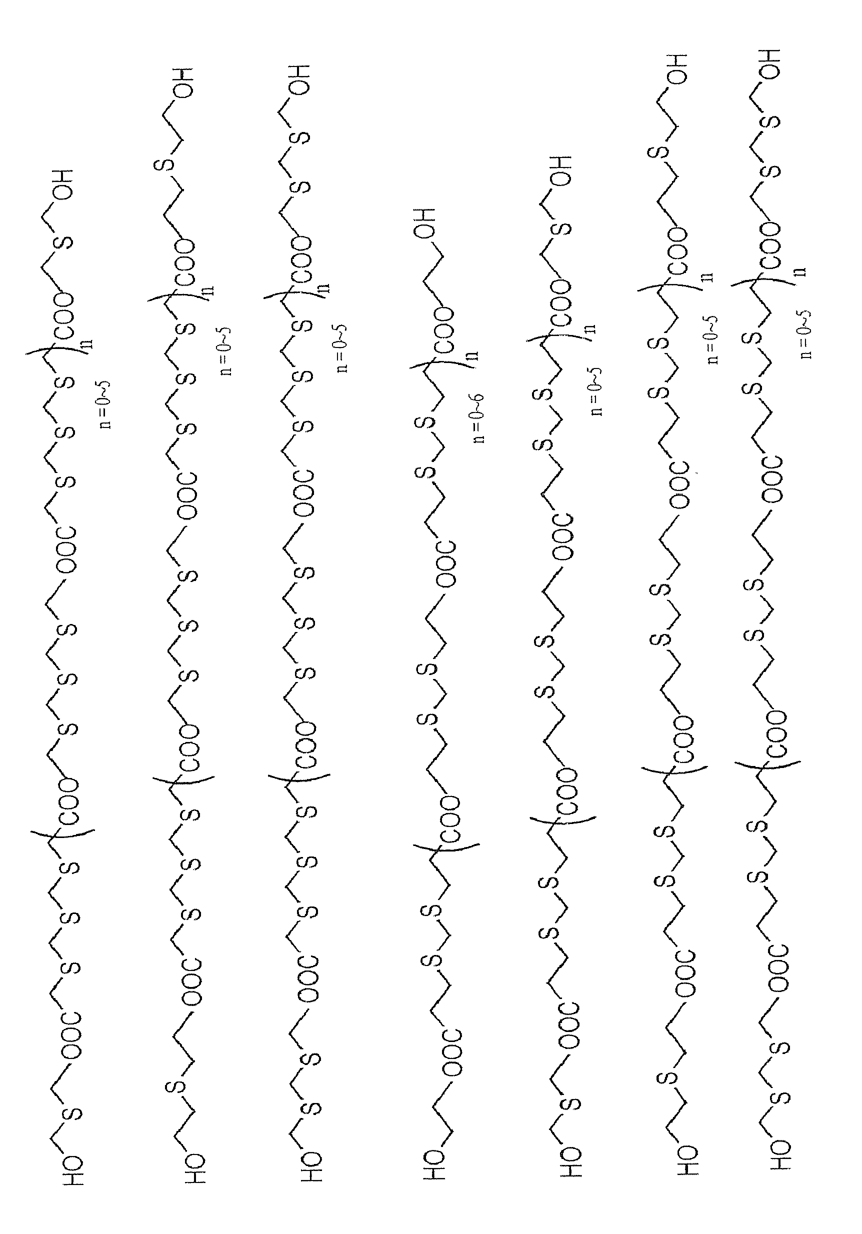

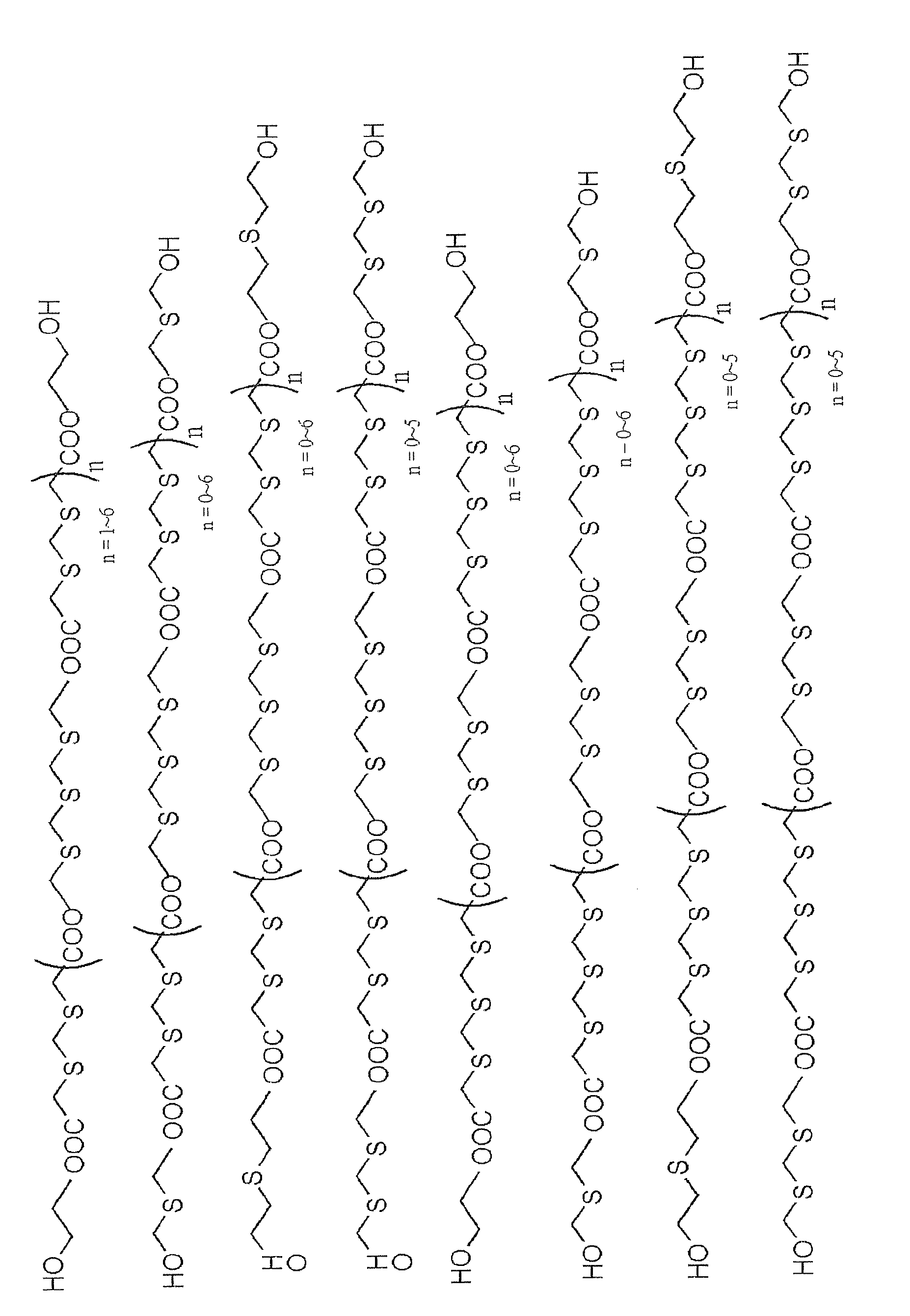

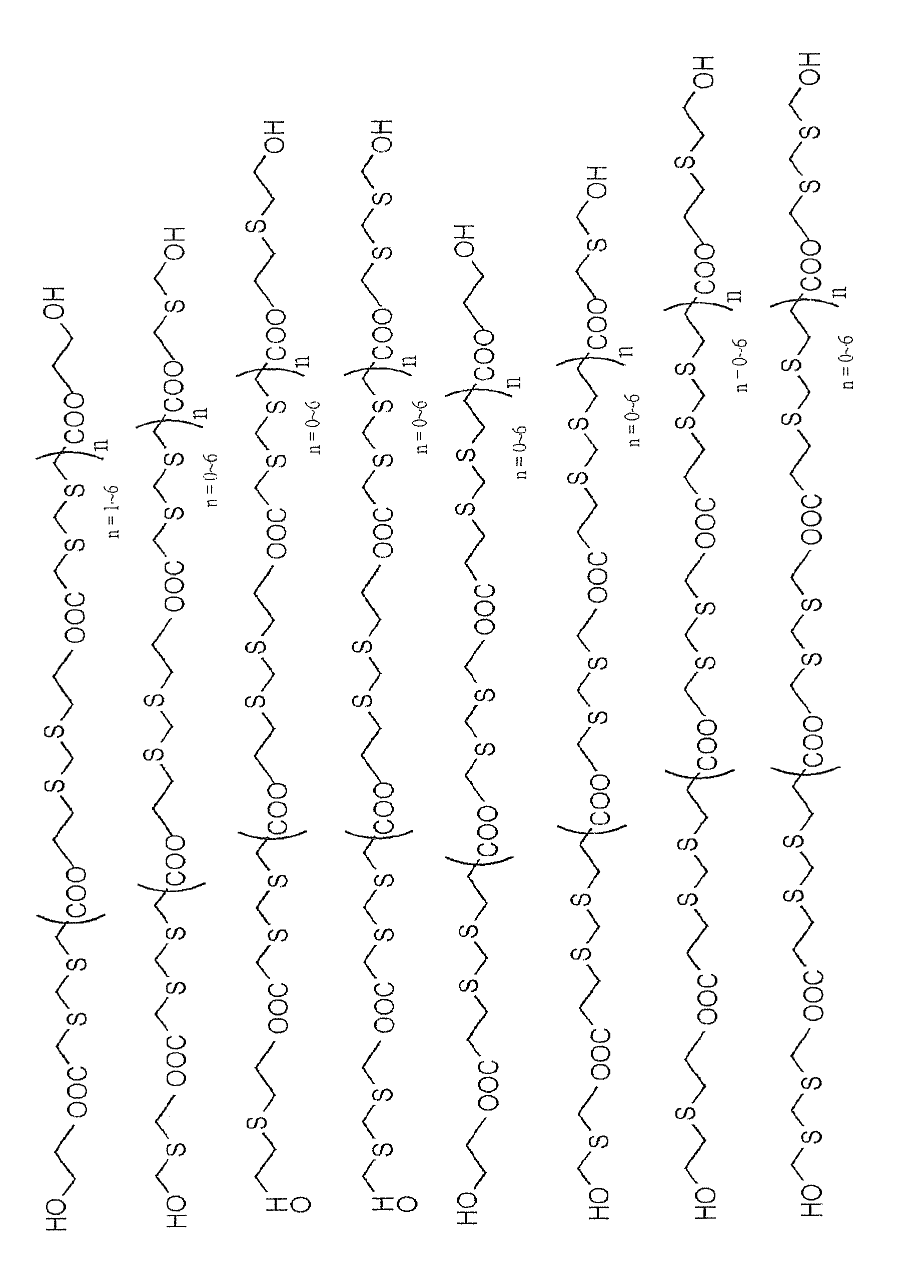

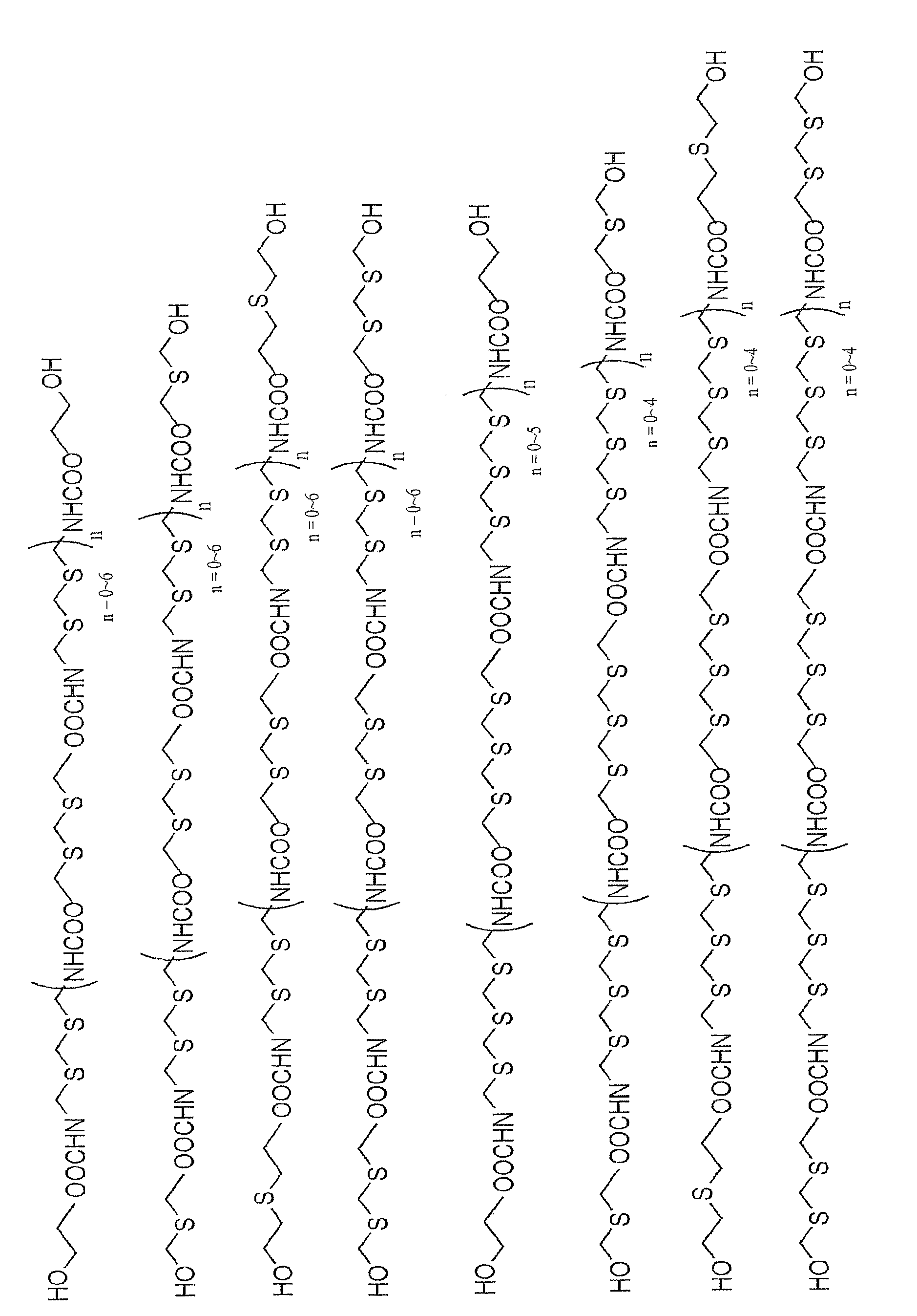

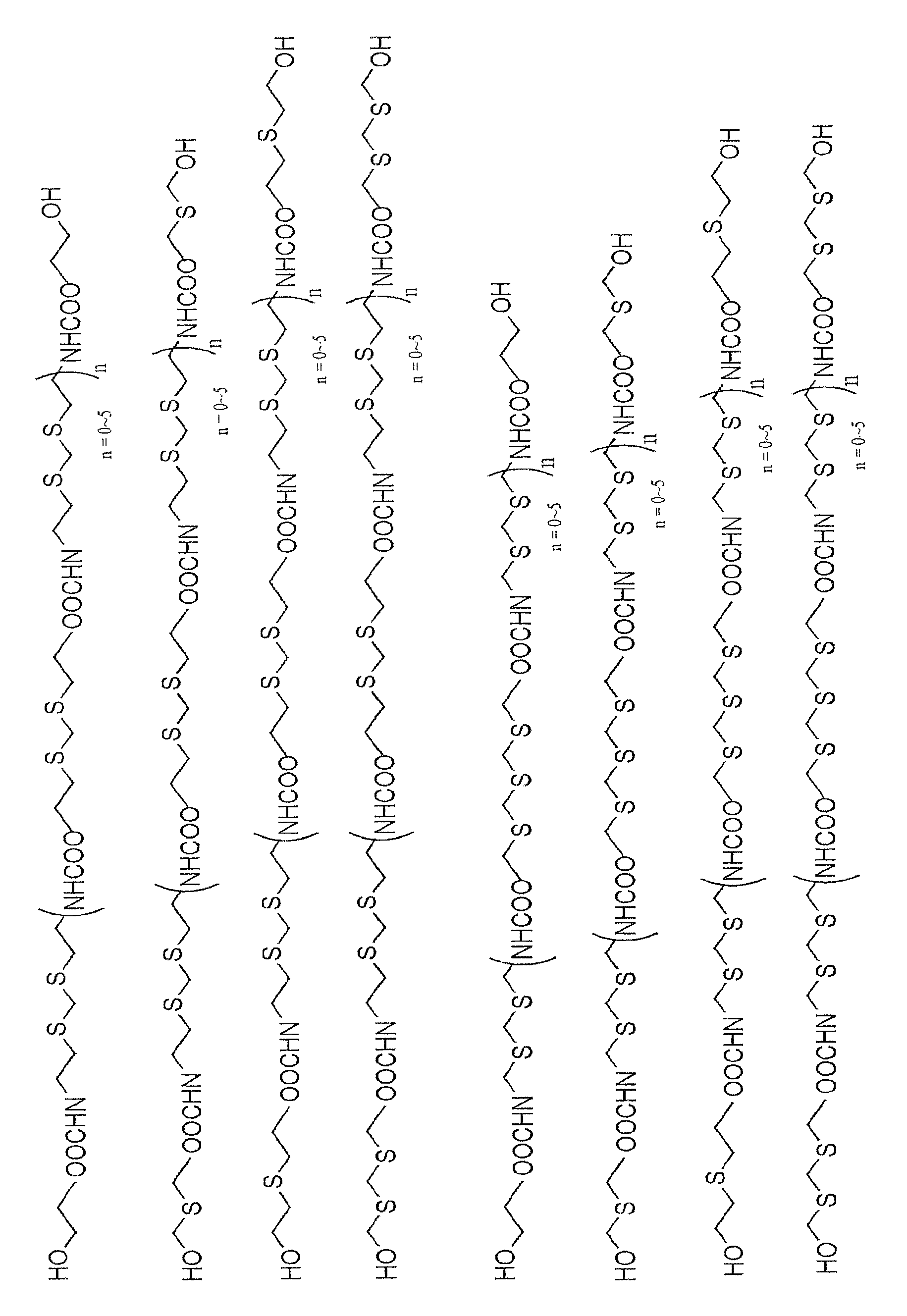

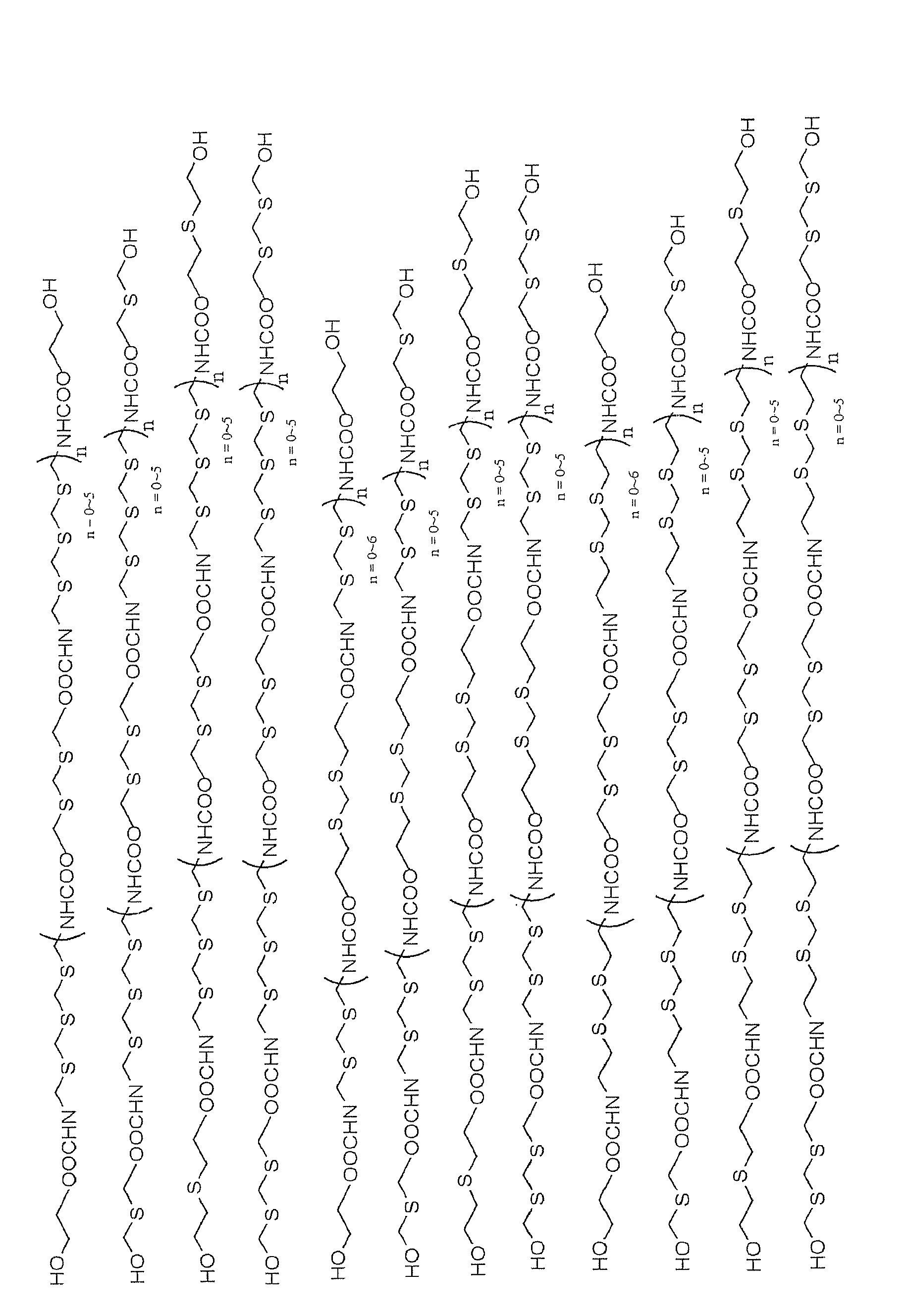

Known methods can be employed as a method of manufacturing the polyol compound of the present invention, denoted by general formula (I). With respect to the manufacturing method, there is no limitation so long as a desired polyol compound can be obtained. All starting material compounds employed for manufacturing the polyol compound of the present invention are commercially available or can be synthesized by known methods.

Examples of the method of manufacturing the polyol compound of the present invention are described below.

### The case where n denotes 0, B denotes an ester bond, and Z denotes an ethylene group in general formula (I)

First, ethylene glycol and a dicarboxylic acid compound containing a Y residue are heated without solvent or in a suitable solvent (such as toluene) to conduct an esterification reaction. During the reaction, a suitable quantity of catalyst is desirably employed to promote esterification, and water produced during the reaction is desirably regularly removed from the system. The ratio of ethylene glycol added relative to dicarboxylic acid compound is desirably a large excess (for example, tenfold or more (mole ratio)) when no solvent is employed because it is also employed as solvent, and desirably threefold or more (mole ratio) when separate solvent is employed, thereby preventing the production of oligomer by-products. The reaction product thus obtained is removed from the reaction system, and as needed, purified by distillation, column fractionation, or the like to obtain the targeted polyol compound. Even when dicarboxylic ester is used to replace the dicarboxylic acid containing a Y residue in the starting materials, the targeted polyol compound can be obtained by transesterification.

A schematic of the above reaction is given below.

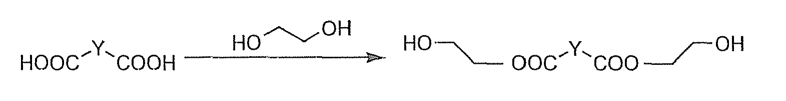

### The case where n denotes 0, B denotes a carbamate bond and Z denotes -CH₂₋CH₂-S-CH₂-CH₂ in general formula (I)

First, thiodiethanol and a diisocyanate compound containing a Y residue are heated without solvent or in a suitable solvent (such as toluene) to conduct a urethane reaction. During the reaction, a suitable quantity of catalyst may be employed to promote urethane reaction. The ratio of thiodiethanol added relative to the diisocyanate compound is desirably threefold or more (mole ratio), regardless of whether or not solvent is employed, to inhibit the formation of by-product oligomers. The reaction product thus obtained is removed from the reaction system, and as needed, purified by distillation, column fractionation, or the like to obtain the targeted polyol compound.

A schematic of the reaction is given below.

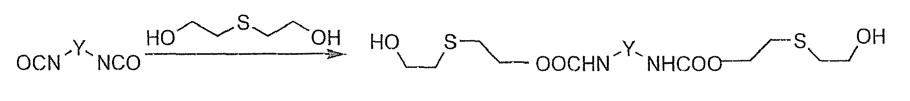

### The case where n denotes 1, A denotes a thiocarbamate bond, B denotes a carbamate bond, and Z denotes -CH₂ -CH₂-S-CH₂-CH₂- in general formula (I)

First, a dithiol compound having an X residue and a diisocyanate compound having a Y residue are heated in a suitable solvent (such as toluene) to conduct a urethane reaction. During the reaction, a suitable quantity of catalyst may be employed to promote urethane reaction. The ratio of diisocyanate added relative to the dithiol compound is desirable twofold or more (mole ratio). After the reaction has ended, unreacted diisocyanate compound is removed, thiodiethanol is added, the mixture is heated, and a urethane reaction is conducted again. In this process, a suitable quantity of solvent is desirably added when there is inadequate solvent. During the reaction, a suitable quantity of catalyst may be employed to promote urethane reaction. The ratio of thiodiethanol added relative to the dithiol compound is desirably threefold or more (mole ratio) to prevent the formation of by-product oligomers. The reaction product thus obtained is removed from the reaction system and, as needed, purified by distillation, column fractionation, or the like to obtain the targeted polyol compound.

A schematic of the above reaction is given below.

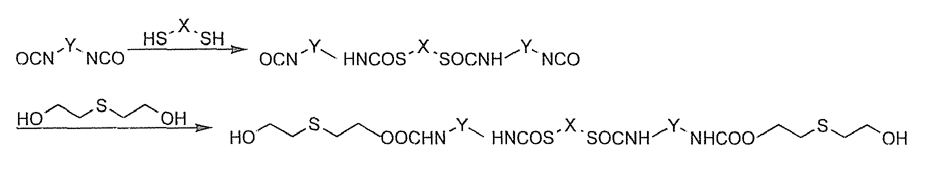

Further, in the above reaction, it is possible to vary the ratio of the dithiol compound having an X residue and the diisocyanate compound having a Y residue employed in the first urethane reaction and conduct a separation operation as needed to obtain a polyol compound of desired n. For example, when three moles of diisocyanate compound having a Y residue are employed per two moles of dithiol compound having an X residue, a polyol compound of n = 2 can be obtained. Further, the type and quantity of solvent and the reaction conditions are suitably selected to efficiently produce a polyol compound of desired n.

### The case where n denotes 2, A denotes a thioester bond, B denotes an ester bond, and Z denotes an ethylene group in general formula (I)

First, a dithiol compound having an X residue and a dicarboxylic acid compound having a Y residue are heated in a suitable solvent (such as toluene) to conduct an esterification reaction. During the reaction, a suitable quantity of catalyst is desirably employed to promote esterification and water formed by the reaction is desirably regularly removed from the system. The ratio of dicarboxylic acid compound added relative to the dithiol compound is desirably 1.5-fold (mole ratio). When the reaction has ended, the unreacted dicarboxylic acid compound is removed, ethylene glycol is newly added, and the mixture is heated to conduct another esterification reaction. In this process, a suitable quantity of solvent is added when the quantity of solvent becomes inadequate. During the reaction, a suitable quantity of catalyst is desirably added to promote esterification and water produced by the reaction is desirably regularly removed from the system. The ratio of ethylene glycol that is added is desirably threefold or more (mole ratio) relative to the dithiol compound to prevent the formation of by-product oligomers. The reaction product thus obtained is removed from the reaction system and, as needed, purified by distillation, column fractionation, or the like to obtain the targeted polyol compound. Even when dicarboxylic acid having a Y residue is use to replace dicarboxylic ester in the starting materials, the targeted polyol compound can be obtained by transesterification.

A schematic of the above reaction is given below.

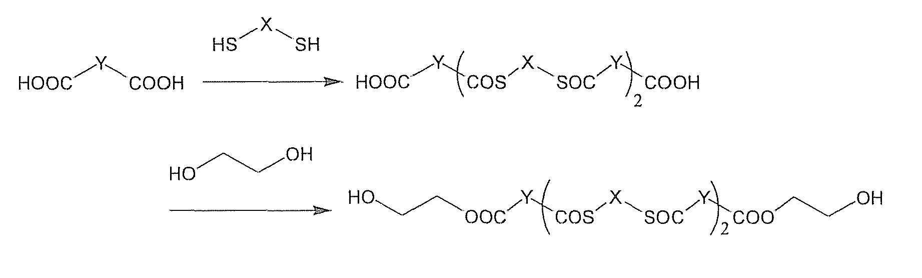

In the above reaction, the ratio of dithiol compound having an X residue and dicarboxylic acid compound having a Y residue employed in the first esterification reaction can be varied and a separation operation employed as needed to obtain a polyol compound of desired n. For example, four moles of dicarboxylic acid compound can be employed per three moles of dithiol compound to obtain a polyol compound of n = 3. Further, the type and quantity of solvent and the reaction conditions are desirably suitably selected to efficiently produce a polyol compound of desired n.

### (Transparent molded article)

The transparent molded article of the first aspect of the present invention is a transparent molded article obtained by polymerizing monomer components comprising the novel polyol compound of the present invention. The transparent molded article of the first aspect simultaneously possesses both a high refractive index and a high Abbé number because it is obtained by polymerizing monomer components comprising the polyol compound of the present invention. Since the ester bonds, thioester bonds, carbamate bonds, or thiocarbamate bonds present in the molecule of the polyol compound of the present invention impart intramolecular interactions and intermolecular interactions to the transparent molded article of the present invention, the transparent molded article of the first aspect has good mechanical characteristics such as high impact resistance.

The transparent molded article of the first aspect is obtained by polymerizing monomer components containing the polyol compound of the present invention. Examples are transparent molded articles comprised of polyurethane, polyester, polycarbonate, epoxy resin, and the like. These transparent molded articles can each be manufactured by known methods.

The transparent molded article of the first aspect can be comprised of polyurethane obtained by polymerizing monomer components comprising component (A) comprising the polyol compound of the present invention and component (B) comprising at least one multifunctional isocyanate compound.

Component (A) can impart a high refractive index while preventing a reduction in the Abbé number of the resulting transparent molded article by incorporating the polyol compound of the present invention. Further, the polyol compound of the present invention imparts intramolecular and intermolecular interactions to the transparent molded article of the first aspect through intramolecular ester bonds, thioester bonds, carbamate bonds, or thiocarbamate bonds. As a result, good mechanical characteristics including high impact resistance are imparted to the transparent molded article of the present invention. Further, since component (A) has good compatibility with component (B), it also has an effect of imparting better transparency to the transparent molded article obtained.

Compounds having bifunctional or greater hydroxyl groups or mercapto groups other than the polyol compound of the present invention can be incorporated to a degree that does not impede the physical properties of the transparent molded article of the present invention to suitably improve the physical properties of the transparent molded article obtained. Examples of such compounds are: 2,5-bis(mercaptomethyl)-1,4-dithane, oligomers thereof, 1,2,3-trimercaptopropane, tetrakis(7-mercapto-2,5-dithaheptyl)methane, 1,2-ethanedithiol, 1,3-propanedithiol, tetrakismercapto-methylmethane, 2-mercaptoethyl sulfide, pentaerythritol tetrakismercaptopropionate, pentaerythritol tetrakismercaptoacetate, 1,2-benzenedithiol, 1,3-benzenedithiol, 1,4-benzenedithiol, 1,3,5-benzenetrithiol, 1,2-dimercaptomethylbenzene, 1,3-dimercaptomethylbenzene, 1,4-dimercaptomethylbenzene, 1,3,5-trimercaptomethylbenzene, toluene-3,4-dithiol, tris(3-mercaptopropyl)isocyanurate, 1,3-bis(mercaptomethyl)cyclohexane, 1,4-bis(mercaptomethyl)cyclohexane, 2,2-bis(mercaptomethyl)-1,3-propanedithiol, 1,2-bis(2-mercaptoethylthio)-3-mercaptopropane, 4,8-bis(mercaptomethyl)-3,6,9-trithia- 1, 11-undecanedithiol, and other compounds having bifunctional or greater mercapto groups; ethylene glycol, trimethylol propane, glycerin, polyethylene glycol, polypropylene glycol, polytetramethylene glycol, 2-hydroxyethylsulfide, bisphenol A-propylene oxide five-mole adduct, glycerin-propylene oxide three-mole adduct, and other groups having bifunctional or greater hydroxyl groups. These compounds can be manufactured by known methods and are available as commercial products.

Component (B) comprises at least one multifunctional isocyanate compound. In the present invention, the term "multifunctional isocyanate compound" refers to an isocyanate compound that is bifunctional or greater. When a bifunctional isocyanate compound is employed in the present invention, straight-chained polyurethane can be obtained. When a trifunctional or greater isocyanate compound is employed, polyurethane with a three-dimensional network can be obtained. In the present invention, two or more isocyanate compounds with different numbers of functional groups can be incorporated into component (B) to impart various physical properties to the transparent molded article.

Examples of multifunctional isocyanate compounds incorporated into component (B) are: hexamethylene diisocyanate, butane diisocyanate, lysine diisocyanate, isocyanato methyl sulfide, 2-isocyanato ethyl sulfide, bis(isocyanatomethylthio)methane), 1,2-bis(isocyanatomethylthio)ethane), bis(2-isocyanatoethylthio)methane, 1,2-bis(2-isocyanatoethylthio)ethane, and other aliphatic chain diisocyanate compounds; o-xylylene diisocyanate, m-xylylene diisocyanate, p-xylylene diisocyanate, *α, α, α', α'-*tetramethyl-p-xylylene diisocyanate, α, α, α', α'-tetramethyl-m-xylylene diisocyanate, and other aromatic ring-comprising diisocyanate compounds; isophorone diisocyanate, norbornene diisocyanate, 4,4'-methylenebis(cyclohexylisocyanate), 1,2-bis(isocyanatomethyl)cyclohexane, 1,3-bis(isocyanatomethyl)cyclohexane, 1,4-bis(isocyanatomethyl)cyclohexane, 1,2-diisocyanatocyclohexane, 1,3-diisocyanatocyclohexane, 1,4-diisocyanatocyclohexane, 2,5-bis(isocyanatomethyl)-1,4-dithane, 2,3-bis(isocyanatomethyl)-1,4-dithiane, 2,6-bis(isocyanatomethyl)-1,4-dithiane, 2,4-bis(isocyanatomethyl)-1,3-dithiane, 2,5-bis(isocyanatomethyl)-1,3-dithiane, 4,6-bis(isocyanatomethyl)-1,3-dithiane, 4,5-bis(isocyanatomethyl)-1,3-dithiane, 2,5-diisocyanato-1,4-dithiane, 2,3-diisocyanato-1,4-dithiane, 2,6-diisocyanato-1,4-dithiane, 2,4-diisocyanato-1,3-dithiane, 2,5-diisocyanato-1,3-dithiane, 4,6-diisocyante-1,3-dithiane, 4,5-diisocyanato-1,3-dithiane, 4,5-bis(isocyanatomethyl)-1,3-dithiolane, 4,5-diisocyanato-1,3-dithiolane, 2,2-bis(isocyanatoethyl)-1,3-dithane, and other alicyclic diisocyanate compounds; and tris(6-isocyanatohexyl)isocyanurate, 1,3,5-tris(isocyanato methyl)cyclohexane, 1,3,5-triisocyanato cyclohexane, lysine triisocyanate, 1,5-diisocyanato-2-isocyanato methyl-3-thiapentane, 1,4-diisocyanato-2-isocyanato methyl-3-thiabutane, and other triisocyanate compounds. To achieve a transparent molded article with good weatherability and heat resistance, multifunctional isocyanate compounds in the form of an alicyclic diisocyanate compound and a triisocyanate compound are desirably incorporated into component (B). These compounds may be manufactured by known methods and are available as commercial products.

In the present invention, when polymerizing components (A) and (B), the mole ratio of the isocyanate groups contained in component (B) to the total of the hydroxyl groups and thiol groups contained in component (A) preferably ranges from 1.00 to 1.15 from the perspective of achieving a molded article with adequate toughness (strength). This mole ratio more preferably ranges from 1.02 to 1.12.

The transparent molded article of the first aspect can be manufactured for example by a method comprising casting a mixture of components (A) and (B) into a casting mold and then heating components (A) and (B) to polymerize them into a molded article. In this process, the heating temperature generally ranges from -20 to 160°C. The heating temperature does not have to be constant during polymerization; it can be changed in stepwise fashion. The heating period depends on conditions such as the heating temperature, and is generally from about 0.5 to 120 hours. In this manufacturing process, it is possible to employ a polymerization catalyst to enhance polymerization properties. Specifically, organic metal compounds including organic tin compounds and tertiary amines may be employed.

To the extent that functioning of the transparent molded article is not impeded, suitable quantities of various additives may be added to the transparent molded article of the first aspect. Ultraviolet radiation absorbing agents, coloring matter, pigments, and the like may be added to enhance absorbance. Oxidation inhibitors, coloration preventing agents, and the like may be added to enhance weatherability. And plasticizers and mold release agents may be added to enhance mold processing. These components can be mixed prior to or during the polymerization, or can be impregnated into the molded article obtained after the polymerization.

As stated above, in the first aspect, the polyol compound of the present invention may be employed as is as a monomer component to obtain a transparent molded article. Further, it may be first reacted with a compound having functional groups capable of reacting with the hydroxyl groups of the polyol compound of the present invention to prepare a prepolymer, and the prepolymer employed in polymerization to obtain a transparent molded article. Examples of compounds having functional groups capable of reacting with the hydroxyl groups of the polyol compound of the present invention are compounds having at least one isocyanate group and at least one (meth)acryloyl group in a molecule, and compounds having at least one epoxy group and at least one (meth)acryloyl group in a molecule. In the present invention, the term "(meth)acryloyl group" refers to both acryloyl groups and methacryloyl groups.

The transparent molded article of the first aspect can be the transparent molded article comprised of a polymerization-cured material obtained by polymerizing a polymerizable composition comprising a radical polymerizable compound having a carbamate bond, which has been obtained by prereacting the polyol compound of the present invention and a compound having at least one isocyanate group and at least one (meth)acryloyl group in a molecule.

Since the above radical polymerizable compound having a carbamate bond contains at least two radical polymerizable groups, it has a self-crosslinking property. Thus, when manufacturing a transparent molded article from this radical polymerizable compound, a high degree of crosslinking is achieved without adding a crosslinking agent as a secondary component, thereby yielding a transparent molded article with good solvent resistance and good weatherability. Since the radical polymerizable compound is obtained from the polyol compound of the present invention, a transparent molded article obtained from the radical polymerizable compound will simultaneously possess both a high refractive index and a high Abbé number. Further, in the transparent molded article of the first aspect, the intramolecular ester bonds, thioester bonds, carbamate bonds, or thiocarbamate bonds of the polyol compound of the present invention impart intramolecular and intermolecular interactions. Thus, the aforementioned transparent molded article has good mechanical characteristics, including good impact resistance.

Examples of compounds having at least one isocyanate group and at least one (meth)acryloyl group in a molecule are: acryloyl isocyanate, methacryloyl isocyanate, 2-isocyanato ethyl acrylate, 2-isocyanato ethyl methacrylate, 3-isocyanato propyl acrylate, and 3-isocyanato propyl methacrylate. These compounds can be synthesized by known methods and are available as commercial products. Among them, 2-isocyanato ethyl methacrylate is preferred from the perspective of the performance of the transparent molded article obtained and availability.

The above radical polymerizable compound having a carbamate bond can be obtained by urethane reaction reacting the polyol compound of the present invention with a compound having at least one isocyanate group and at least one (meth)acryloyl group in a molecule, normally in a ratio of 1.0/0.8 to 1.0/1.2 hydroxyl groups/isocyanate groups. The reaction method is not specifically limited; any common urethane reaction method may be employed.

In this reaction, a suitable catalyst may be employed as needed. Examples of this catalyst are tertiary amines and organic metal compounds such as organic tin compounds.

The reaction temperature and period varies with the type of starting material employed and whether or not a catalyst is employed. Normally, a temperature of -10 to 60°C and a period of 0.1 to 40 hours may be employed.

In the above polymerizable composition, other than the above-described radical polymerizable compound having a carbamate group, one or more radical polymerizable compounds capable of copolymerizing with the above radical polymerizable compound and having a radical polymerization group may be incorporated to suitably improve the physical properties of the transparent molded article. Specific examples of such compounds are methyl (meth)acrylate, ethyl (meth)acrylate, propyl (meth)acrylate, butyl (meth)acrylate, 2-ethylhexyl (meth)acrylate, benzyl (meth)acrylate, butoxyethyl (meth)acrylate, cyclohexyl (meth)acrylate, 2-hydroxyethyl (meth)acrylate, glycidyl (meth)acrylate, phenoxyethyl (meth)acrylate, ethylene glycol di(meth)acrylate, diethylene glycol di(meth)acrylate, triethylene glycol di(meth)acrylate, tetraethylene glycol di(meth)acrylate, polyethylene glycol di(meth)acrylate, neopentyl glycol di(meth)acrylate, ethylene glycol bisglycidyl (meth)acrylate, bisphenol A di(meth)acrylate, 2,2-bis(4-(meth)acryloxydiethoxyphenyl)propane, trimethylol propane tri(meth)acrylate, glycerol di(meth)acrylate, diallyl phthalate, diallyl terephthalate, diallyl isophthalate, diallyl carbonate, diethylene glycol bisallyl carbonate, styrene, chlorostyrene, methyl styrene, bromostyrene, dibromostyrene, 2,5-bis(2-thia-3-butenyl)-1,4-dithane, and 2,5-bis((meth)acryloylthiomethyl)-1,4-dithane. Preferred examples are 2,5-bis(2-thia-3-butenyl)-1,4-dithane. These compounds can be synthesized by known methods and are available as commercial products. The above "(meth)acrylate" refers to both acrylate and methacrylate; "(meth)acryloxy group" refers to both acryloxy groups and methacryloxy groups; and "(meth)acryloyl group" refers to both acryloyl groups and methacryloyl groups.

The content of the radical polymerizable compound having a carbamate bond in the above polymerizable composition is preferably 30 weight percent or more, more preferably 40 weight percent or more, and still more preferably, 50 weight percent or more.

Known radical polymerization methods employing heat, ultraviolet radiation, and the like may be employed to polymerize the above polymerizable composition to obtain the transparent molded article of the first aspect. Among such methods, the use of an ultraviolet irradiation method is desirable from the perspectives of achieving optical uniformity in the transparent molded article obtained and facilitating manufacturing.

In this process, a catalyst may be suitably employed to enhance polymerization reactivity; the use of a known sensitizer is effective. Examples of such catalysts are: benzophenone, 4,4-diethylaminobenzophenone, 1-hydroxycyclohexyl phenyl ketone, p-dimethylaminoisoamyl benzoate, 4-dimethylaminomethyl benzoate, benzoin, benzoin ethyl ether, benzoin isobutyl ether, benzoin isopropyl ether, 2,2-diethoxyacetophenone, o-benzoyl methyl benzoate, 2-hydroxy-2-methyl-1-phenylpropane-1-one, and acyl phosphine oxide. These catalysts may be employed singly or in combinations of two or more. These catalysts are available as commercial products.

The ultraviolet irradiation conditions may be suitably set based on whether or not a catalyst is employed, the type of catalyst, the type of radical polymerizable compound employed, and the like. In general, an irradiation intensity may be 0.1 to 100 mW/cm² and an irradiation period may be 5 seconds to 30 minutes.

One example of a method of manufacturing the transparent molded article of the first aspect is set forth below. A uniform mixture of the aforementioned radical polymerizable compound having a carbamate bond, a radical polymerizable compound capable of copolymerizing with this compound, and any additives and catalysts is cast by a known casting mold polymerization method into a mold comprised of resin or glass transparent to ultraviolet radiation and a gasket made of resin, and cured by irradiation with ultraviolet radiation. At that time, to facilitate removal of the resin following molding, the mold may be pretreated to release the resin or a mold release agent may be incorporated into the mixture comprising the radical polymerizable compound having a carbamate bond and the like. Following completion of irradiation with ultraviolet radiation, heating is desirably conducted to complete polymerization and alleviate stress generated within the material. The temperature and duration of heating vary with the energy level of the ultraviolet radiation employed and the like, and are generally from 30 to 150°C and 0.2 to 24 hours.

The transparent molded article of the first aspect can be surface treated following molding with a hard coating to improve scratch resistance or an antireflective coating to reduce reflectance.

The transparent molded article of the first aspect may be a lens such as an eyeglass lens or optical lens; a prism; an optical fiber; a substrate for a recording medium employed in an optical disk, magnetic disk, or the like; or an optical material such as a filter. The transparent molded article of the present invention is preferably a lens, more preferably an eyeglass lens.

### Second aspect

The transparent molded article of second aspect of the present invention is obtained by polymerizing monomer components comprising components (C) and (D).

### (Component C)

Component (C) is an isocyanate terminal prepolymer in the form of a reaction product of an aliphatic diisocyanate having an intramolecular cyclic structure and a diol or dithiol comprising an intramolecular sulfur atom and having an average molecular weight of 300-2,500.

Since the diisocyanate that is one of the starting materials of the isocyanate terminal prepolymer is an aliphatic diisocyanate having an intramolecular ring structure, control of the reaction during manufacturing of the prepolymer and during polymerization is facilitated. Further, the final molded article is readily imparted with suitable elasticity. Still further, the molded article obtained can be imparted with high heat resistance and good mechanical characteristics.

The term "aliphatic diisocyanate having an intramolecular cyclic structure" refers to an aliphatic diisocyanate having a cyclic structure in a main chain or in a side chain. The cyclic structure may be an alicycle, a sulfur-containing alicycle, aromatic ring, or heterocycle. However, the aliphatic diisocyanate having an intramolecular cyclic structure is desirably an alicyclic diisocyanate or sulfur-containing alicyclic diisocyanate from the perspectives of preventing yellowing of the molded article obtained and maintaining adequate elasticity and hardness. Compared to a molded article obtained from an alicyclic diisocyanate, a molded article obtained from isocyanate having an aromatic ring tends to undergo more pronounced yellowing. A molded article obtained from an aliphatic chain isocyanate is soft and tends to lose its shape. From these perspectives, the aliphatic diisocyanate in the present invention is desirably an alicyclic diisocyanate or sulfur-containing alicyclic diisocyanate.

Examples of alicyclic diisocyanates are: 4,4'-methylenebis(cyclohexylisocyanate), isophorone diisocyanate, 1,2-bis(isocyanatomethyl)cyclohexane, 1,3-bis(isocyanatomethyl)cyclohexane, 1,4-bis(isocyanatomethyl)cyclohexane, 1,2-diisocyanato cyclohexane, 1,3-diisocyanato cyclohexane, and 1,4-diisocyanato cyclohexane. Examples of sulfur-containing alicyclic diisocyanate are: 2,5-bis(isocyanatomethyl)-1,4-dithiane, 2,3-bis(isocyanatomethyl)-1,4-dithiane, 2,6-bis(isocyanatomethyl)-1,4-dithiane, 2,4-bis(isocyanatomethyl)-1,3-dithiane, 2,5-bis(isocyanatomethyl)-1,3-dithiane, 4,6-bis(isocyanatomethyl)-1,3-dithiane, 4,5-bis(isocyanatomethyl)-1,3-dithiane, 2,5-diisocyanato-1,4-dithiane, 2,3-diisocyanato-1,4-dithiane, 2,6-diisocyanato-1,4-dithiane, 2,4-diisocyanato-1 ,3-dithiane, 2,5-diisocyanato-1,3-dithiane, 4,6-diisocyanato-1,3-dithiane, 4,5-diisocyanato-1,3-dithiane, 4,5-bis(isocyanatomethyl)-1,3-dithiolane, 4,5-diisocyatiato-1,3-dithiolane, and 2,2-bis(isocyanatoethyl)-1,3-dithiolane. Further, examples of diisocyanates having an aromatic ring are: m-xylylene diisocyanate, o-xylylene diisocyanate, p-xylylene diisocyanate, and m-tetramethylxylylene diisocyanate.

The above aliphatic diisocyanate having an intramolecular cyclic structure desirably comprises at least one member selected from the group consisting of 4,4'-methylenebis(cyclohexylisocyanate), isophorone diisocyanate, 1,3-bis(isocyanatomethyl)cyclohexane, and 2,5-bis(isocyanatomethyl)-1,4-dithiane.

The average molecular weight of the diol or dithiol that is another starting material of the isocyanate terminal prepolymer of component (C) is characterized by ranging from 300 to 2,500. When this average molecular weight is less than 300, it is impossible to impart toughness to the molded article obtained, and when it exceeds 2,500, the molded article obtained becomes soft and unable to retain its shape. The average molecular weight of the diol or dithiol is desirably from 400 to 1,000.

Another characteristic of the diol or dithiol that is one of the starting materials of the isocyanate terminal prepolymer of component (C) is that of comprising an intramolecular sulfur atom. Incorporating a sulfur atom into the molecule prevents the Abbé number from decreasing and permits a higher refractive index. The state in which the sulfur atoms are present within the molecule is not specifically limited, however the sulfur is desirably incorporated into the molecule in the form of at least one bond from among a sulfide bond, disulfide bond, thioester bond, dithioester bond, thiocarbonate bond, and dithiocarbonate bond. When sulfur atoms are incorporated into the molecule in the form of the above bonds, component (C) has good compatibility with the other components and a molded article with good transparency is obtained without coloration. Further, when the sulfur atoms are incorporated into the molecule in a form other than the above bonds, component (C) tends to have poor compatibility with other components, it becomes necessary to add further components such as compatibilizing agents to maintain the transparence of the molded article obtained, and there is a possibility of marked coloration. From these perspectives, the diol or dithiol serving as one of the starting materials of the isocyanate terminal prepolymer of component (C) in the present invention desirably contains intramolecular sulfur in the form of at least one bond from among a sulfide bond, disulfide bond, thioester bond, dithioester bond, thiocarbonate bond, or dithiocarbonate bond.

The above-described polyol compound of the present invention can be employed as the diol compound serving as a starting material of component (C).

The diol compound having an average molecular weight of 300 to 2,500 denoted by general formula (III), for example, can also be employed as the diol compound serving as a starting material of component (C). 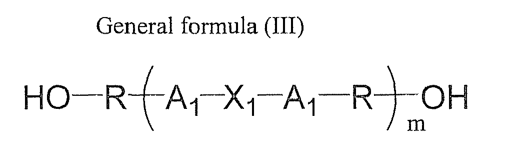 (In the formula, X₁ denotes an alkylene group having 3 to 12 carbon atoms, in which at least one methylene group contained is substituted with a sulfur atom (without direct bonding between sulfur atoms); R denotes an alkylene group having 2 to 6 carbon atoms, in which the methylene group contained is optionally substituted with a sulfur atom; A₁ denotes an ester group, thioester group, carbamate group, or thiocarbamate group; and m denotes any integer such that the diol compound denoted by general formula (III) falls within the stated molecular weight range.)

Examples of such diol compounds denoted by general formula (III) are compounds obtained by reacting a diol compound such as thiodiethanol, thiodipropanol, 1,5-dihydroxy-2,4-dithiapentane, 1,7-dihydroxy-3,5-dithiaheptane, 1,6-dihydroxy-2,5-dithiahexane, 1,8-dihydroxy-3,6-dithiaoctane, 1,9-dihydroxy-3,5,7-trithianonane, or 1,7-dihydroxy-2,4,6-trithiaheptane with a dibasic acid such as thiodiglycolic acid, thiodipropionic acid, methylenebis(thioglycolic acid), ethane-1,2-bis(thiopropionic acid), or thiodi(2-thiabutanoic acid). These can also be obtained by replacing the above diol compounds with dithiol compounds and the above dibasic acids with diisocyanate compounds. In the reaction for obtaining the diol compound denoted by general formula (III) above, the mole ratio of the diol compound and the dibasic acid can be suitably selected based on the value of m in general formula (III). For example, when in denotes 1, the diol compound to dibasic acid ratio can be from 2:1 to 10:1.

In addition to the polyol compound of the present invention and the diol compound denoted by general formula (III), further examples of diol compounds serving as starting materials for component (C) are two-terminal hydroxy-modified polyethylene sulfide and two-terminal hydroxy-modified polypropylene sulfide.

Examples of the dithiol compound serving as starting material for component (C) are dithiol compounds comprised of polyethylene sulfide, polypropylene sulfide, or dimercaptoethyl sulfide and a dibasic acid such as thiodiglycolic acid, thiodipropionic acid, methylenebis(thioglycolic acid), ethane-1,2-bis(thiopropionic acid), or thiodi(2-thiabutanoic acid); dithiol compounds comprised of ethanedithiol and one of the above dibasic acids; and Thiocol LP (name of product made by Toray Fine Chemicals Co., Ltd).

The content of the isocyanate groups in the isocyanate terminal prepolymer of component (C) is desirably 10 to 20 weight percent. When the content of the isocyanate groups is 10 percent or greater, the molded article obtained has high hardness; when 20 weight percent or less, a molded article having adequate strength (toughness) can be obtained.

### (Component (D))

Component (D) is one or more of the aromatic diamines denoted by general formula (II).

In general formula (II), R₁, R₂, and R₃ each independently denote methyl groups, ethyl groups, or thiomethyl groups. When R₁, R₂, and R₃ denote the above substituents, the crystallinity of component (D) is inhibited and compatibility with other components is enhanced. When R₁, R₂, and R₃ do not denote these substituents, the compatibility of component (D) with other components deteriorates and the transparency of the material obtained may decrease.

More specific examples of the above aromatic diamines are one or more of the following compounds: 1,3,5-trimethyl-2,4-diaminobenzene, 1,3,5-trimethyl-2,6-diaminobenzene, 1,3,5-triethyl-2,4-diaminobenzene, 1,3,5-triethyl-2,6-diaminobenzene, 1,3,5-trithiomethyl-2,4-diaminobenzene, 1,3,5-trithiomethyl-2,6-diaminobenzene, 3,5-diethyl-2,4-diaminotoluene, 3,S-diethyl-2,6-diaminotoluene, 3,5-dithiomethyl-2,4-diaminotoluene, 3,5-dithiomethyl-2,6-diaminotoluene, 1-ethyl-3,5-dimethyl-2,4-diaminobenzene, 1-ethyl-3,5-dimethyl-2,6-diaminobenzene, 1-ethyl-3,5-dithiomethyl-2,4-diaminobenzene, 1-ethyl-3,5-dithiomethyl-2,6-diaminobenzene, 1-thiomethyl-3,5-dimethyl-2,4-diaminobenzene, 1-thiomethyl-3,5-dimethyl-2,6-diaminobenzene, 1-thiomethyl-3,5-diethyl-2,4-diaminobenzene, 1-thiomethyl-3,5-diethyl-2,6-diaminobenzene, 3-ethyl-5-thiomethyl-2,4-diaminotoluene, 3-ethyl-5-thiomethyl-2,6-diaminotoluene, and 3-thiomethyl-5-ethyl-2,4-diaminotoluene.

In the above aromatic diamines, R₁ being a methyl group, R₂ and R₃ respectively being ethyl groups or thiomethyl groups is desirable in that a molded article tending not to fog and having adequate toughness is obtained.

More specific examples of these aromatic diamines are: 3,5-diethyl-2,4-diaminotoluene, 3,5-diethyl-2,6-diaminotoluene, 3,5-dithiomethyl-2,4-diaminotoluene, and 3,5-dithiomethyl-2,6-diaminotoluene.

In the polymerization of the transparent molded article of second aspect, a ratio of components (C) and (D) as a mole ratio of the isocyanate groups in component (C) to the amino groups in component (D) falling within the range of 1.00 to 1.15 is desirable from the perspective of obtaining a molded article having adequate toughness (strength). This mole ratio preferably ranges from 1.02 to 1.12.

To the extent that the transparency and mechanical characteristics of the transparent molded article of the present invention are not lost, additives such as ultraviolet radiation absorbing agents, coloring matter, pigments, and the like may be added to enhance absorbance; oxidation inhibitors, coloration preventing agents, peroxide decomposing agents and the like may be added to enhance weatherability; and mold release agents may be added to enhance the molding properties of the transparent molded article of second aspect in addition to components (C) and (D). These components may be admixed to the various components before polymerization, admixed during polymerization, or impregnated into the molded article obtained after polymerization.

In this invention, examples of ultraviolet radiation absorbing agents are benzotriazoles, benzophenones, and salicylic compounds. Examples of coloring matter and pigments are anthraquinones and azo compounds.

Examples of oxidation inhibitors and coloration preventing agents are monophenols, bisphenols, and high-molecular phenol compounds. Examples of peroxide-decomposing agents are phosphorus compounds. Examples of mold release agents are fluorine surfactants, silicone surfactants, acid phosphoric esters, and higher fatty acids.

The transparent molded article of second aspect can be manufactured by a method comprising casting a mixture of components (C) and (D) into a casting mold and then polymerizing components (C) and (D) to obtain a molded article. Due to the high polymerizability (reactivity) of components (C) and (D), the reaction proceeds even at room temperature. Thus, in the case of incorporating the above additional components, it is desirable to mix components (C) and (D) and rapidly cast into a casting mold after adding and uniformly dissolving them into either component (C) or component (D).

A detailed example of the polymerization reaction conditions is given below in Examples. The temperature generally ranges from -20 to 160°C and the duration depends on conditions such temperatures, and generally ranges from 0.5 to 120 hours.

Following molding, the transparent molded article of the second aspect may be surface treated with a hard coating to enhance scratch resistance, an antireflective coating to reduce reflectance, or the like.

The transparent molded article of the second aspect may be a lens such as an eyeglass lens or optical lens; a prism; an optical fiber; a substrate for a recording medium employed in an optical disk, magnetic disk, or the like; or an optical material such as a filter. The transparent molded article of the second aspect is preferably a lens, more preferably an eyeglass lens.

### EXAMPLES

The present invention will be described below based on Examples. However, the present invention is not limited to Examples.

The physical properties of the polyol compound and transparent molded article obtained were evaluated by the following methods.

### (1) ¹H-NMR spectrum (proton nuclear magnetic resonance spectrum)

This was measured with an FT-NMR unit, model EX270, made by JEOL Limited.

### (2) Refractive index (nD) and Abbé number (vD)

These were measured at 20°C with an Abbé Refractometer, Model 3T, made by Atago Co., Ltd.

### (3) Coloration

The obtained lens, which was a kind of optical products using the transparent molded article, was evaluated by visual observation. Those without coloration were rated as A, those with slight coloration (yellowing) as B, and those with marked coloration as C.

### (4) Transparency

The obtained lens, which was a kind of optical products using the transparent molded article, was evaluated by visual observation in a dark room under fluorescent lighting. Those in which no fogging or nontransparent matter precipitated out in the interior were rated as A. Those in which slight fogging and the like was observed were rated as B. And those in which severe fogging or the precipitation of nontransparent matter was clearly observed were rated as C. Lenses rated B or C were considered unsuitable for use as lenses.

### (5) Weatherability

The obtained lens, which was a kind of optical products using the transparent molded article, was placed in a weathermeter equipped with a sunshine carbon arc lamp. Once 200 hours had elapsed, the lens was removed. Its hue then was compared to that prior to the test. Those exhibiting no change were rated A, those exhibiting slight yellowing as B, and those exhibiting marked yellowing as C.

### (6) Optical distortion

The obtained lens, which was a kind of optical products using the transparent molded article, was evaluated by visual observation with the Schlieren method. Those without distortion were rated as A, those with only slight distortion along the edges as B, and those that were totally distorted as C.

### (7) Impact resistance

A steel ball weighing 16 g was dropped under its own weight from a height of 1.27 m, an FDA standard, onto the center of an S-4.00 lens with a center thickness of 1.3 mm. Test samples in which no lenses broke were rated A, test samples in which fewer than 30 percent (but at least one) of the lenses either cracked or were damaged or pierced were rated as B, and test samples in which 30 percent or more of the lenses were damaged were rated as C. Testing and evaluation were also similarly conducted with a steel ball weighting 1 kg.

### (Example 1)

### Manufacturing of methylenebis((thioglycolic acid)-5-hydroxy-3-thiapentylester)

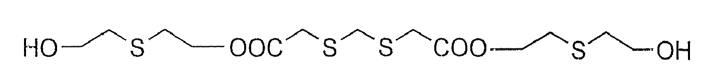

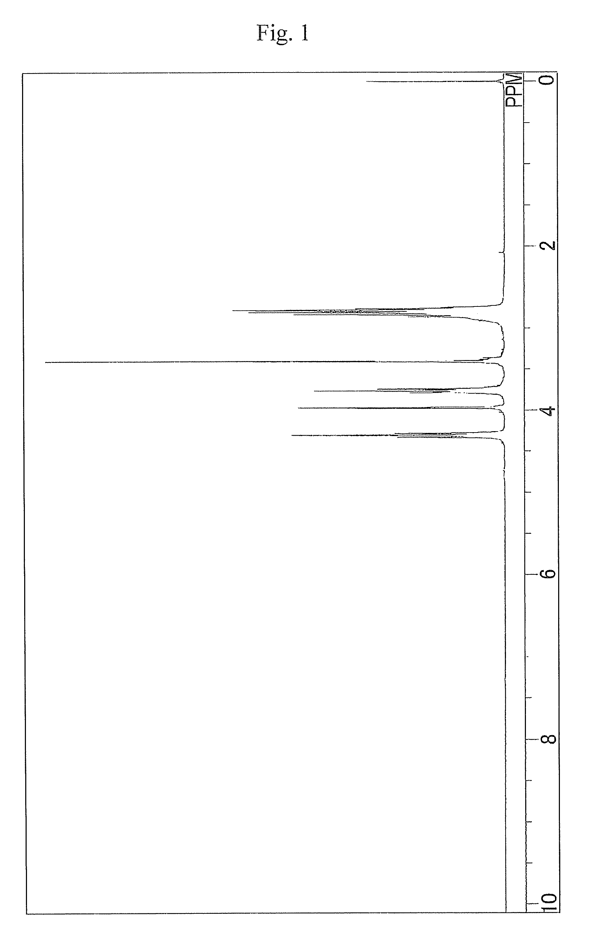

A 70.6 g (0.36 mole) quantity of methylenebis(thioglycolic acid), 131.8 g (1.08 moles) of thiodiethanol, and 2.2 g of paratoluene sulfonic acid were reacted for 20 hours at 90°C while being vigorously stirred in 300 mL of benzene. A quantitative moisture receiver was mounted on the reaction vessel to remove from the system the water that was generated. Subsequently, extraction was conducted with ethyl acetate, the extract was washed with water, and drying was conducted with magnesium sulfate. The ethyl acetate was then adequately distilled off, yielding 132.9 g (0.34 mole) of reaction product. This reaction product was confirmed to be the polyol compound denoted by general formula (I) based on ¹H-NMR spectral analysis. Fig. 1 gives the ¹H-NMR spectrum of this new polyol compound.

### (Example 2)

### (i) Manufacturing of 3,5,7-trithianonanedioic acid - di(2-hydroxyethylester)

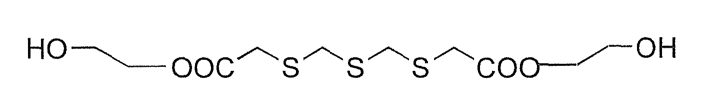

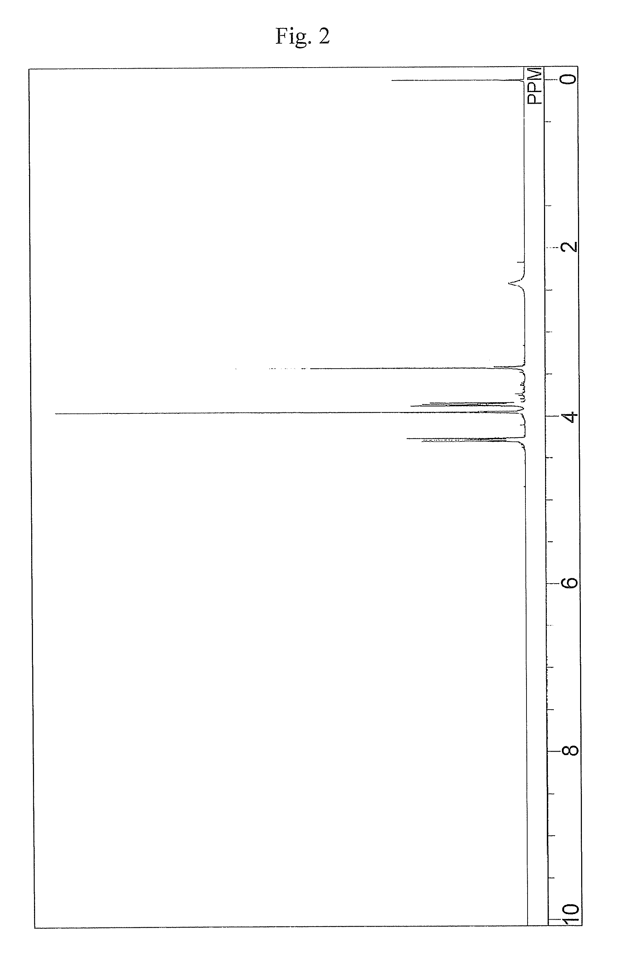

A 121.3 g (0.45 mole) quantity of 3,5,7-trithianonanedioic acid dimethyl ester and 1.8 g of paratoluene sulfonic acid were reacted for 20 hours at 90°C while being vigorously stirred in 500 mL (8.98 moles) of dehydrated ethylene glycol. At that time, the reaction was conducted while gradually reducing the pressure with an aspirator to remove from the system the methanol that was generated. Extraction with chloroform was conducted and the extract was washed with saturated brine and dried with magnesium sulfate. The chloroform was then adequately distilled off, yielding 107.5 g (0.33 mole) of reaction product. This reaction product was confirmed to be the polyol compound denoted by general formula (I) based on ¹H-NMR spectral analysis. Fig. 2 gives the ¹H-NMR spectrum of this new polyol compound.

### (ii) Manufacturing of 4,6-dithianonanedioic acid - di(2-hydroxyethylester)

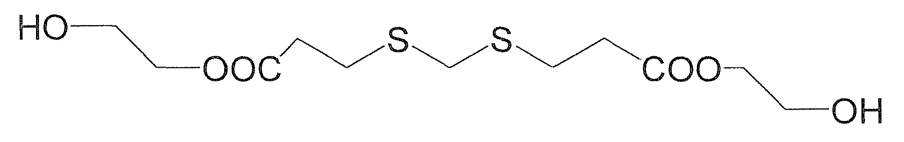

With the exception that the 121.3 g of 3,5,7-trithianonanedioic acid dimethyl ester was replaced with 113.4 g of 4,6-dithianonanedioic acid dimethyl ester, the same method as in (i) above was employed to obtain 4,6-dithianonanedioic acid - di(2-hydroxyethylester).

### (Example 3)

A mixture of 0.09 mole of the methylenebis((thioglycolic acid)-5-hydroxy-3-thiapentylester) (denoted as SO1 in Table 1) obtained in Example 1, 0.25 mole of 2,5-bis(mercaptomethyl)-1,4-dithiane (denoted as DMMD in Table 1), 0.20 mole of 1,3-bis(isocyanatomethyl)cyclohexane (denoted as HXDI in Table 1), 0.08 mole of tris(6-isocyanatohexyl)isocyanurate (denoted as CX in Table 1), and 3.4 x 10⁻⁴ mole of dibutyl tin dilaurate (denoted as DBTDL in Table 1) was uniformly stirred, cast into a lens-forming glass mold, and polymerized by heating for 10 hours at 50°C followed by 5 hours at 60°C and 3 hours at 120°C to obtain plastic lenses. Table 1 gives the various physical properties of the plastic lens obtained. As shown in Table 1, the lens of Example 3 had a high refractive index of 1.60, a high Abbé number of 41, no coloration, good transparency and weatherability, and no optical distortion.

### (Examples 4-6)

With the exception that the monomer compositions shown in Table 1 were employed, plastic lenses were obtained by the same operations as in Example 3. The various physical properties of these plastic lenses are given in Table 1 along with the various physical properties of the lens of Example 3. As shown in Table 1, the lenses of Examples 4 to 6 had high refractive indexes of 1.60 to 1.63 and high Abbé numbers of 39 to 44. They had no coloration, good transparency and weatherability, and no optical distortion.

### (Comparative Example 1)

A mixture of 0.06 mole of pentaerythritol tetrakismercaptopropionate (denoted as PETMP in Table 1), 0.12 mole of m-xylylene diisocyanate (denoted as XDI in Table 1), and 1.2 x 10⁻⁴ mole of dibutyl tin dilaurate (denoted as DBTDL in Table 1) was uniformly stirred, cast into a lens-forming glass mold, and hot polymerized for 10 hours at 50°C followed by 5 hours at 60°C and 3 hours at 120°C to obtain a plastic lens. Table 1 gives the various physical properties of the plastic lens obtained. As shown in Table 1, the lens of Comparative Example 1 had a high refractive index of 1.59 and good transparency. No coloration or optical distortion was observed. However, the Abbé number was a low 35 and the lens had poor weatherability.

### (Comparative Examples 2 to 3)

With the exception that the monomer composition of Table 1 was employed, plastic lenses were obtained by the same operations as in Comparative Example 1. The various physical properties of these plastic lenses are given in Table 1 along with the various physical properties of the lenses of Examples 3 to 6 and Comparative Example 1. As shown in Table 1, the lens of Comparative Example 2 had a high refractive index of 1.60 and a high Abbé number of 39. It exhibited good transparency and no optical distortion. However, it exhibited a yellow coloration and had poor weatherability. The lens of Comparative Example 3 had a high refractive index of 1.57 and a high Abbé number of 41, did not exhibit coloration, and had good weatherability. However, it exhibited internal fogging and optical distortion.

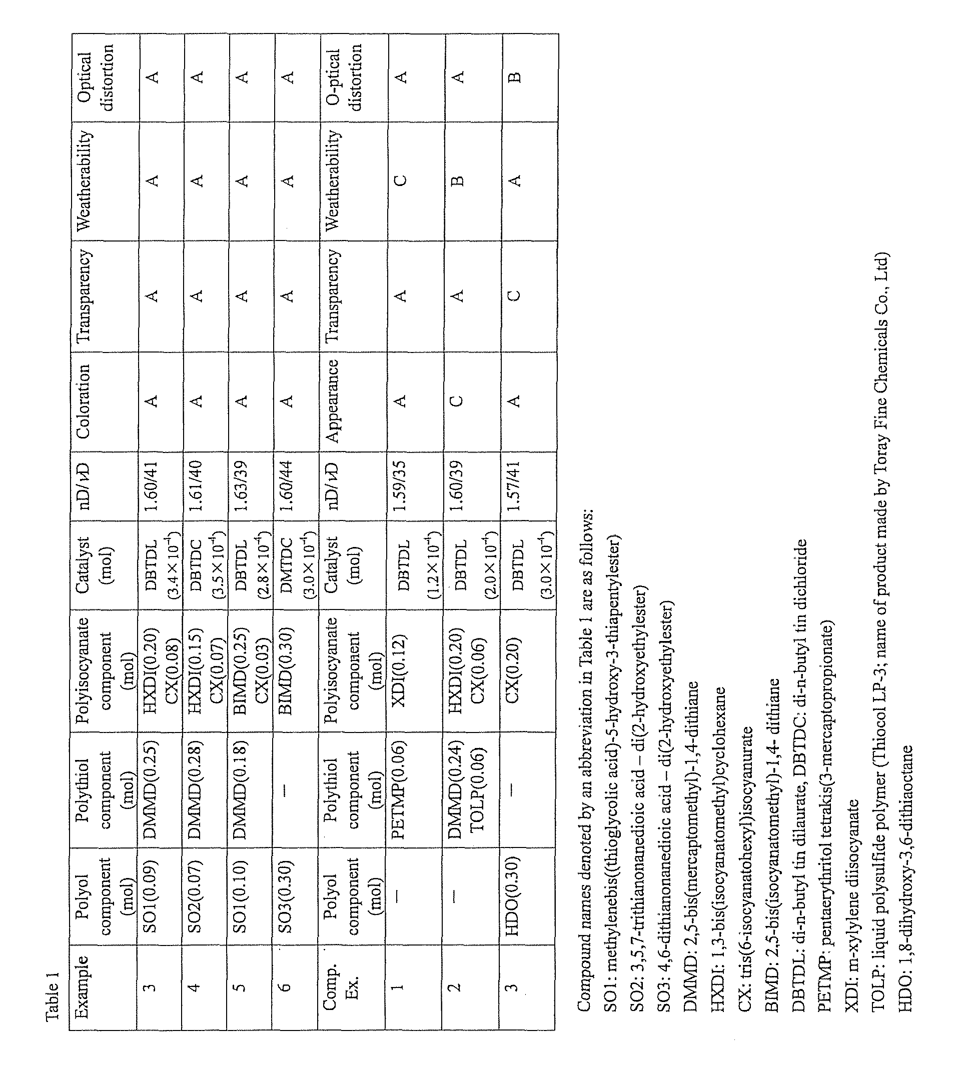

### (Example 7)

### (1) Manufacturing of a radical polymerizable compound having a carbamate bond

A 1.5 x 10⁻⁴ mole quantity of catalyst in the form of dibutyl tin dilaurate was added to a mixture of 0.15 mole of the methylenebis(thioglycolic acid)-5-hydroxy-3-thiapentylester) obtained in Example 1 and 0.30 mole of 2-isocyanato ethyl methacrylate. The mixture was then stirred for 2 hours at 40°C. Upon cooling, 0.15 mole of a colorless, transparent, slightly viscous radical polymerizable compound having a carbamate bond was obtained.

### (2) Manufacturing of optical products

A mixture of 100 weight parts of the radical polymerizable compound (denoted as HRM in Table 2) obtained in (1) above and 0.2 weight part of 2,2-diethoxyacetophenone (denoted as DEA in Table 2) was uniformly stirred, cast into a two-plate lens-forming glass mold, irradiated for 10 minutes with ultraviolet radiation with an intensity of 8 mW/cm², and maintained at 120°C for 3 hours by heating to obtain a plastic lens. Table 2 gives the various physical properties of the plastic lens obtained. As shown in Table 2, the lens of Example 7 had a high refractive index of 1.56, an extremely high Abbé number of 50, no coloration, good transparency and weatherability, and no optical distortion.

### (Examples 8 and 9)

With the exception that the polymerizable compositions shown in Table 2 were employed, plastic lenses were obtained by the same operations as in Example 7. The various physical properties of these plastic lenses are given in Table 2 along with the various physical properties of the plastic lens of Example 7. As shown in Table 2, the lenses of Examples 8 and 9 had high refractive indexes of 1.57 to 1.60, high Abbé numbers of 43 to 48, no coloration, good transparency and weatherability, and no optical distortion.

### (Comparative Example 4)

A mixture of 35 weight parts of epoxy methacrylate obtained by reacting acrylic acid with epoxy resin obtained from epichlorohydrin and tetrabromobisphenol A (denoted as TBBE in Table 2), 12 weight parts of bisphenol S bis(2-(2'-methacryloyloxyethoxy)ethyl)ether (denoted as BPS in Table 2), 35 weight parts of styrene (denoted as St in Table 1), 8 weight parts of hexamethylene diisocyanate (denoted as HDI in Table 2), and 10 weight parts of o-phenylphenylglycidylether (denoted as PPG in Table 2) to which catalysts in the form of 0.025 weight parts of dibutyl tin dilaurate (denoted as DBTDL in Table 2) and 0.1 weight part of azobisisobutyronitrile (denoted as AIBN in Table 2) had been added was uniformly stirred, cast into a two-plate lens-forming glass mold, heated from 35°C to 90°C over 17 hours, and then maintained for one hour at 90°C to obtain a plastic lens. Table 2 gives the various physical properties of the plastic lens obtained. As shown in Table 2, the lens of Comparative Example 4 had a high refractive index of 1.56 and good transparency, but a low Abbé number of 35, poor weatherability, slight coloration, and optical distortion.

### (Comparative Example 5)

A mixture of 60 weight parts of thiourethane methacrylate (denoted as BBM in Table 2) obtained by reacting equimolar quantities of 2,4,6-tribromobenzylthiol and 2-isocyanato ethyl methacrylate, 40 weight parts of methyl methacrylate (denoted as MMA in Table 2), and 2 weight parts of diisopropyl peroxycarbonate (denoted as IPPC in Table 2) was uniformly stirred, cast into a two-plate lens-forming glass mold, and heated for 6 hours at 35°C, 4 hours at 50°C, 3 hours at 70°C, 5 hours at 85°C, and 1 hour at 100°C to obtain a plastic lens. The various physical properties of the plastic lens obtained are given in Table 2. As shown in Table 2, the lens of Comparative Example 5 had a high refractive index of 1.57, but a low Abbé number of 33, poor weatherability, and marked coloration. Further, fogging and slight optical distortion were exhibited on the interior.

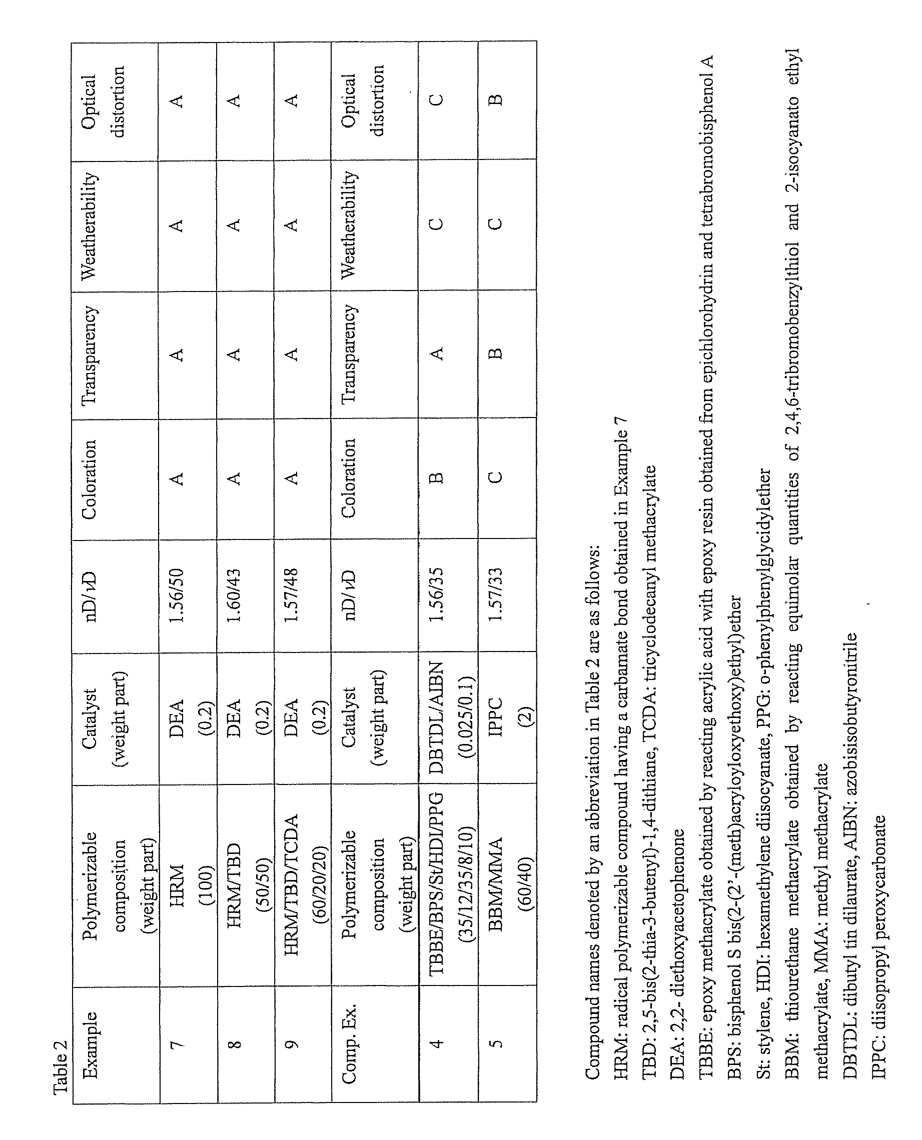

### (Example 10)

A 25 weight part quantity of a mixture of 3,5-diethyl-2,4-toluenediamine and 3,5-diethyl-2,6-toluenediamine (denoted as DETDA in Table 3) was uniformly mixed at 60 to 70°C with 100 parts of isocyanate terminal prepolymer (denoted as STP-1 in Table 3) with a 13 percent isocyanate group content comprised of methylenebis((thioglycolic acid)-5-hydroxy-3-thiapentylester) and 2,5-bis(isocyanatomethyl)-1,4-dithiane that had been degassed in advance, and stirred for a short period at high speed. Immediately following stirring, the mixture was cast into a lens-forming glass mold and polymerized by heating for 15 hours at 120°C, yielding a plastic lens (transparent molded article). The various physical properties of the plastic lens obtained are given in Table 3. As shown in Table 3, the plastic lens obtained had a high refractive index of 1.60, a high Abbé number of 39, and good transparency. It also had good impact resistance, remaining unbroken both in the FDA standard ball dropping test of 16 g as well as at 1 kg.

### (Example 11)

With the exception that the monomer composition shown in Table 3 was employed, plastic lenses were obtained by the same operations as in Example 10. The various physical properties of these plastic lenses are given in Table 3 along with the various physical properties of the lens of Example 10. As shown in Table 3, the plastic lenses of Example 11 had a high refractive index (nD) of 1.63, a high Abbé number *(vD)* of 38, and good transparency. They also had good impact resistance, remaining unbroken both in the FDA standard ball dropping test of 16 g as well as at 1 kg.

### (Comparative Example 6)

With the exception that isocyanate terminal prepolymer (denoted in Table 3 as PTO) with a 13 percent isocyanate group content comprised of polytetramethylene glycol and 4,4'-methylenebis(cyclohexylisocyanate) with an average molecular weight of 400 was used instead of isocyanate terminal prepolymer with a 13 percent isocyanate group content comprised of methylenebis((thioglycolic acid)-5-hydroxy-3-thiapentylester) and 2,5-bis(isocyanatomethyl)-1,4-dithiane, the same operations were conducted as in Example 10. The various physical properties of the plastic lens obtained are shown in Table 3. As shown in Table 3, the plastic lens obtained had a high Abbé number of 45, good transparency, and good impact resistance, remaining unbroken both in the FDA standard ball dropping test of 16 g as well as at 1 kg. However, the refractive index was a low 1.53.

### (Example 12)

The plastic lens, which is a transparent molded article, prepared in Example 10 was thoroughly cleaned by immersion for 5 minutes in a 10 percent sodium hydroxide aqueous solution at 55°C, coated by dipping (at a lifting rate of 20 cm/min) in a coating solution prepared by the method described below, and heated for 2 hours at 120°C to form a hard coating. Subsequently, the plastic lens was heated to 85°C and a seven-layer antireflective film was formed by vacuum vapor deposition (at a vacuum of 2 x 10⁻⁵ Torr) over the hard coating. The various physical properties of the plastic lens (transparent molded article) having both a hard coating and an antireflective layer that was obtained are given in Table 3. As shown in Table 3, the plastic lens obtained had a refractive index of 1.60 and an Abbé number of 39, just as it had prior to the formation of the hard coating and antireflective layer, as well as good transparency. It also had good impact resistance, remaining unbroken both in the FDA standard ball dropping test of 16 g as well as at 1 kg.

### (Comparative Example 7)

A mixture of 0.20 mole of 4-mercaptomethyl-1,8-dimercapto-3,6-dithiaoctane (denoted as MMMO in Table 3), 0.30 mole of norbornene diisocyanate (denoted as NDI in Table 3), and 3.0 x 10⁻⁴ mole of dibutyl tin dilaurate (denoted as DBTDL in Table 3) was uniformly stirred, cast into a lens-forming glass mold, and polymerized by heating for 10 hours at 50°C, 5 hours at 60°C, and 3 hours at 120°C to obtain a plastic lens. A hard coating and an antireflective layer were formed on the plastic lens by the same methods as in Example 12. The various physical properties of the plastic lens having a hard coating and an antireflective layer that was obtained are given in Table 3 along with the various physical properties of the plastic lens of Example 2. As shown in Table 3, the plastic lens of Comparative Example 7 had a high refractive index of 1.60, a high Abbé number of 41, and good transparency. However, it had poor impact resistance, with all of the lenses breaking in a 1 kg ball dropping test and 30 percent or more of the lenses breaking in the FDA standard 16 g ball dropping test.

### (Reference Example - Preparation of coating solution)

A 141 weight part quantity of colloidal silica suspended in water (40 percent solid component, average particle diameter 15 millimicrons) was charged to a glass container equipped with magnetic stirrer. A 30 weight part quantity of acetic acid was added with stirring, and the mixture was thoroughly mixed. Subsequently, 74 weight parts of γ glycidoxypropyltrimethoxysilane were added dropwise and the mixture was stirred for 24 hours at 5°C. Next, 100 weight parts of propylene glycol monomethyl ether, 150 weight parts of isopropyl alcohol, 0.2 weight part of silicone surfactant, and 7.5 weight parts of a curing agent in the form of aluminum acetyl acetate were added. The mixture was then thoroughly stirred and filtered to complete preparation of the coating solution.

**Table 3**

| Example | Component(C) | Component(D) | Hard coating and antireflective layer | nD/*v*D | Transparency | Impact resistance | |
|---|---|---|---|---|---|---|---|
| | (weight part) | (weight part) | | | | 16g | 1kg |
| 10 | STP-1 | DETDA | - | 1.60/39 | A | A | A |
| | (100) | (25) | | | | | |
| 11 | STP-2 | DETDA | - | 1.63/38 | A | A | A |
| | (100) | (22) | | | | | |
| 12 | STP-1 | DETDA | Provided | 1.60/39 | A | A | A |
| | (100) | (25) | | | | | |
| Comp.Ex. | Component(C) | Component(D) | Hard coating | nD/*v*D | Transparency | Impact resistance | |
| | (weight part) | (weight part) | | | | 16g | 1kg |
| 6 | PTO | DETDA | - | 1.53/45 | A | A | A |
| | (100) | (25) | | | | | |
| 7 | NDI | MMMO(52) | Provided | 1.60/41 | A | C | C |
| | (62) | DBTDL(0.2) | | | | | |

| | | | | | | | |
|---|---|---|---|---|---|---|---|
| Compound names denoted by an abbreviation in Table 3 are as follows: | | | | | | | |
| STP-1: isocyanate terminal prepolymer with a 13 percent isocyanate group content comprised of methylenebis((thioglycolic acid)-5-hydroxy-3-thiapentylester) and 2,5-bis(isocyanatomethyl)-1,4-dithiane | | | | | | | |
| STP-2: isocyanate terminal prepolymer with a 11 percent isocyanate group content comprised of methylenebis((2-thiopropionic acid)-5-mercapto-3-thiapentylthioester) and 2,5-bis(isocyanatomethyl)-1,4-dithiane | | | | | | | |
| DETDA: mixture of 3,5-diethyl-2,4-toluenediamine and 3,5-diethyl-2,6-toluenediamine | | | | | | | |
| PTO: isocyanate terminal prepolymer with a 13 percent isocyanate group content comprised of polytetramethylene glycol and 4,4'-methylenebis(cyclohexylisocyanate) with an average molecular weight of 400 | | | | | | | |
| NDI: norbornene diisocyanate | | | | | | | |
| MMMO: 4-mercaptomethyl-1,8-dimercapto-3,6-dithiaoctane | | | | | | | |
| DBTDL,: di-n-butyl tin dilaurate | | | | | | | |

### INDUSTRIAL APPLICABILITY

The polyol compound of the present invention is, for example, employed as a useful starting material of a transparent molded article employed for optical plastic lens materials and the like. By employing the novel polyol compound of the present invention, a transparent molded article having a high refractive index and high Abbé number as well as having excellent impact resistance and weatherability can be provided. The transparent molded article of the first aspect of the present invention, obtained by employing the polyol compound of the present invention, has high refractive index and high Abbé number as well as exhibits no optical distortion, no coloration and excellent weatherability. Furthermore, the transparent molded article of the first aspect of the present invention also has excellent impact resistance and weatherability. The transparent molded article of the first aspect of the present invention can be suitably employed for various lenses such as eyeglass lens, a prism, an optical fiber, a substrate for recording media and optical products such as a filter.

Since the transparent molded article of the second aspect of the present invention is comprised of polyurea having an intramolecular urethane bond or thiourethane bond, it has characteristics in the form of excellent refractive index, Abbé number, impact resistance and transparency as well as no optical distortion. Accordingly, the transparent molded article of the second aspect of the present invention can be suitably employed for eyeglass lenses, camera lenses and other optical lenses, and the like.

## Claims

1. A polyol compound **characterized by** being denoted by general formula (I): 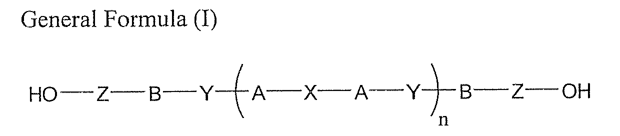 (wherein X and Y each independently denote alkylene groups comprising 3 to 12 carbon atoms, in which at least one of methylene groups (excluding terminals) contained is substituted with a sulfur atom (without direct bonding between sulfur atoms); Z denotes an alkylene group comprising 2 to 6 carbon atoms, in which a methylene group (excluding terminals) contained is optionally substituted with a sulfur atom (without direct bonding between sulfur atoms); A and B each independently denote an ester group, thioester group, carbamate group, or thiocarbamate group; and n denotes an integer ranging from 0 to 12).

2. The polyol compound according to claim 1, wherein said polyol compound has an average molecular weight ranging from 300 to 2500.

3. The polyol compound according to claim 1 or 2, wherein, in general formula (I), X and/or Y comprise the following structure:
―S―CH₂―S―.

4. The polyol compound according to any of claims 1 to 3, wherein, in general formula (I), B is an ester group or carbamate group.

5. A transparent molded article obtained by polymerizing monomer components comprising the polyol compound according to any of claims 1 to 4.

6. The transparent molded article according to claim 5, which is comprised of polyurethane obtained by polymerizing monomer components comprising component (A) comprising the polyol compound according to any of claims 1 to 4 and component (B) comprising at least one multifunctional isocyanate compound.

7. The transparent molded article according to claim 5, which is comprised of a polymerization-cured material obtained by polymerizing a polymerizable composition comprising a radical polymerizable compound having a carbamate bond, which has been obtained by prereacting the polyol compound according to any of claims 1 to 4 and a compound having at least one isocyanate group and at least one (meth)acryloyl group in a molecule.

8. The transparent molded article according to any of claims 5 to 7, wherein the transparent molded article is a lens.

9. The transparent molded article according to claim 8, wherein the lens is an eyeglass lens.

10. A transparent molded article obtained by polymerizing monomer components comprising the following components (C) and (D).
Component (C): isocyanate terminal prepolymer in the form of a reaction product of an aliphatic diisocyanate having an intramolecular cyclic structure and a diol or dithiol comprising an intramolecular sulfur atom and having an average molecular weight of 300-2,500.
Component (D): one or more aromatic diamines denoted by general formula (II). (In general formula (II), R₁, R₂ and R₃ are each independently any of a methyl, ethyl or thiomethyl group.)

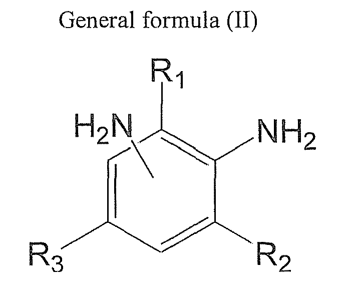

11. The transparent molded article according to claim 10, wherein the aliphatic diisocyanate having an intramolecular cyclic structure, that is a starting material of component (C), is an alicyclic diisocyanate.

12. The transparent molded article according to claim 11, wherein the alicyclic diisocyanate comprises a sulfur atom in its structure.

13. The transparent molded article according to any of claims 10 to 12, wherein the diol or dithiol comprising an intramolecular sulfur atom and having an average molecular weight of 300-2,500, that is a starting material of component (C), comprises an intramolecular sulfur atom in the form of at least one bond from among a sulfide bond, disulfide bond, thioester bond, dithioester bond, thiocarbonate bond and dithiocarbonate bond.

14. The transparent molded article according to any of claims 10 to 12, wherein the diol comprising an intramolecular sulfur atom and having an average molecular weight of 300-2,500, that is a starting material of component (C), is the polyol compound according to any of claims 2 to 4.

15. The transparent molded article according to any of claims 10 to 14, wherein the isocyanate group content of component (A) ranges from 10 to 20 weight percent.

16. The transparent molded article according to any of claims 10 to 15, wherein, in general formula (II), R₁ is a methyl group, and R₂ and R₃ are independently any of a ethyl group or thiomethyl group.

17. The transparent molded article according to any of claims 10 to 16, wherein, in said polymerization, the molar ratio of the isocyanate group of component (C) to an amino group of component (D) ranges from 1.00 to 1.15.

18. The transparent molded article according to any of claims 10 to 17, wherein the transparent molded article is a lens.

19. The transparent molded article according to claim 18, wherein the lens is an eyeglass lens.

20. A method of manufacturing a transparent molded article, comprising casting a mixture of component (C) and component (D) defined in any of claims 10 to 19 into a casting mold and then polymerizing component (C) and component (D) to obtain a molded article.
